# EUROPEAN PATENT APPLICATION

(11) **EP 2 386 563 A1**
(43) Date of publication of application: **16.11.2011**
(21) Application number: 10290254.1
(22) Date of filing: 12.05.2010
(51) Int. Cl.: C07H 3/06, C08B 37/00, G01N 33/50

(54) **Novel O-acetylated decasaccharides**

(71) Applicant: Institut Pasteur, 75015 Paris (FR)
(72) Inventor: Mulard, Laurence, 94270 Le Kremlin Bicetre (FR); Gauthier, Charles, Péribonka, Quebec G0W 2G0 (CA)
(74) Representative: Noel, Chantal Odile

(57) **Abstract**

The present invention relates to diversely acetylated decasaccharides of formula (I) representative of two repeating units of *Shigella flexneri* serotype 2a O-antigen, conjugates and method of preparation thereof. These compounds exhibit antigenic properties and are particularly useful for the diagnosis of *Shigella* infection. 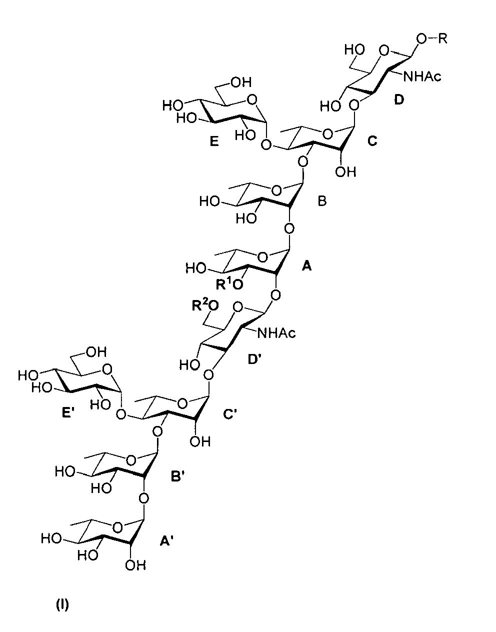 wherein R₁ and R₂ are as defined in claim 1.

## Description

The present invention relates to diversely acetylated decasaccharides of formula (I) representative of two repeating units of *Shigella flexneri* serotype 2a O-antigen, conjugates and method of preparation thereof. These compounds exhibit antigenic properties and are particularly useful for the diagnosis *of Shigella* infection.

The invention also relates to fully protected decasaccharide intermediates of formula (II) which are particularly useful for the preparation of the diversely acetylated decasaccharides of formula (I).

Shigellosis or bacillary dysentery is an invasive enteric infection, which is characterized by a high pathogenic potential, increased antibiotic-resistance,¹ and a negative impact on function and development.² In the absence of a broadly available vaccine,³ shigellosis remains a major health concern associated to high rates of morbidity and mortality, especially in the pediatric population.^{1,4} Disease is caused by Gram negative enteroinvasive bacteria named *Shigella,* whose only reservoir is humans. *Shigellae* are divided into four species, among which *Shigella flexneri,* which is the major responsible for endemic shigellosis especially in developing countries.⁴

Based on the chemical composition of the specific polysaccharide part of their lipopolysaccharide (LPS) component, the O-antigen (O-Ag), *S*. *flexneri* strains are classified into 14 serotypes. With the exception of serotype 6, the O-Ags of all serotypes possess a common backbone consisting of a *N*-acetyl-β-D-glucosamine (**D**) and three α-L-rhamnopyranose (**A, B, C**) residues (Figure la). Phage-mediated modifications resulting in α-D-glucopyranose and/or *O*-acetyl substitutions on the basic tetrasaccharide repeat confer the serotype specificity.⁵ Despite a highly variable geographical distribution of *S*. *flexneri* serotypes, available epidemiological data indicate that *S*. *flexneri* 2a (SF2a) remains the single most prevalent *S*. *flexneri* subserotype in most if not all countries.^{1,3,4}

Amongst the various vaccine strategies under study,³ conjugate vaccines derived from the bacterial LPS are of particular interest.^{6,7} Indeed, besides LPS serving as an essential virulence factor, its O-Ag component is thought to be a major target of the host protective humoral immune defences against natural infection. In recent years, short synthetic saccharidic haptens emerged as potent alternatives to detoxified LPSs in the field of carbohydrate-based vaccines against shigellosis.⁹⁻¹¹

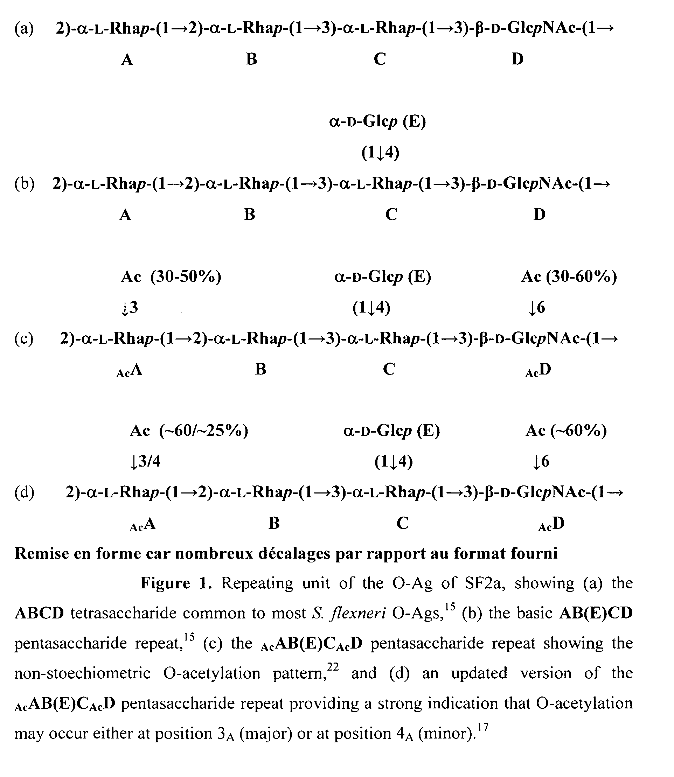

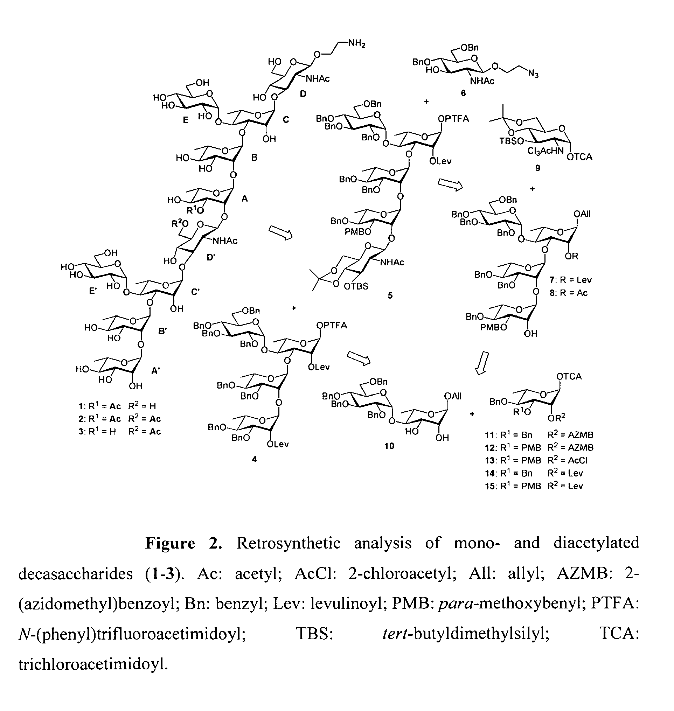

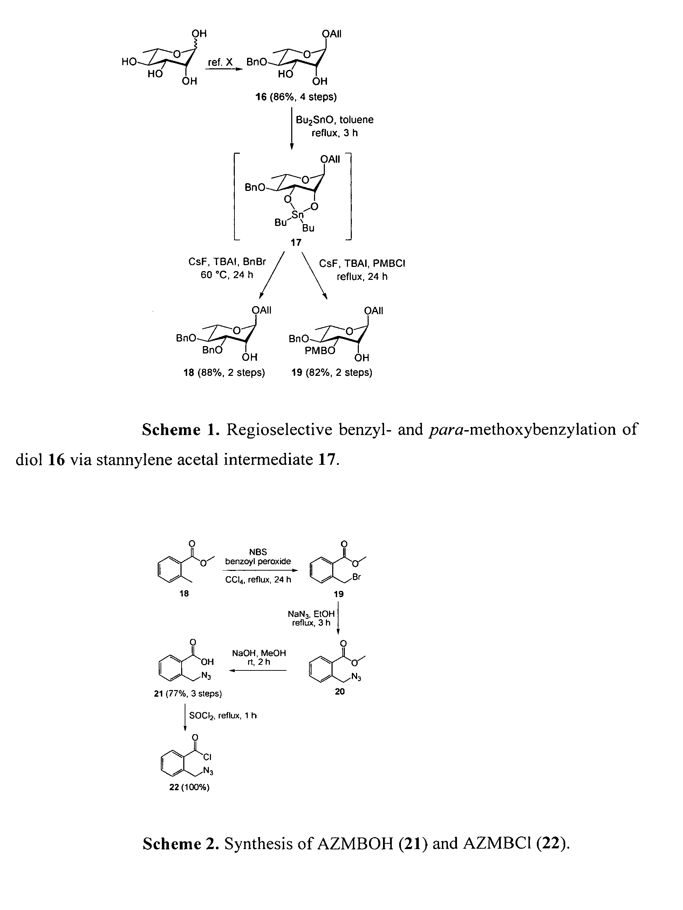

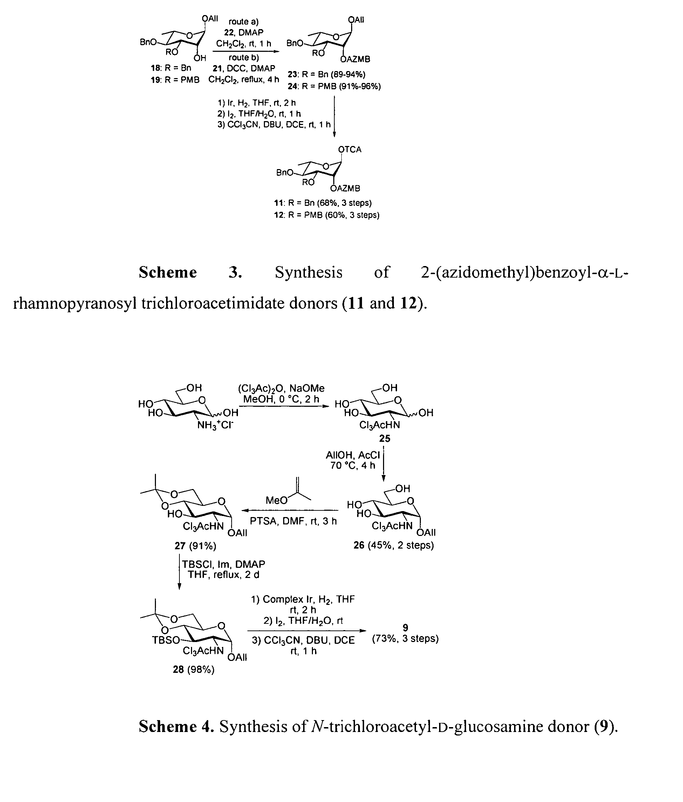

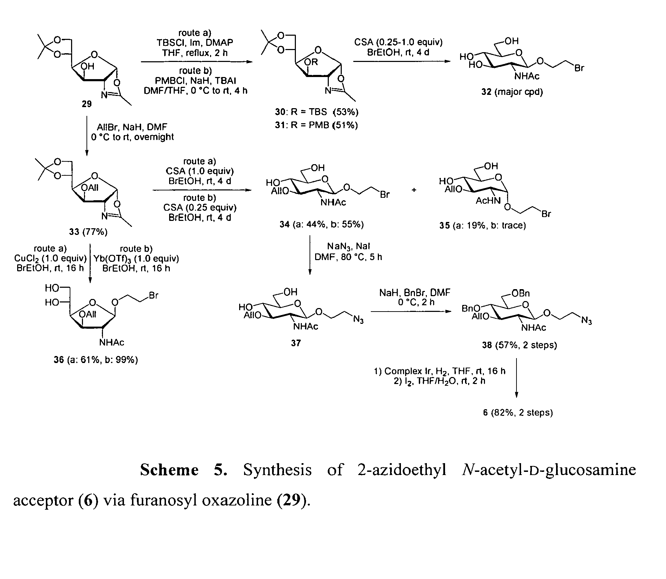

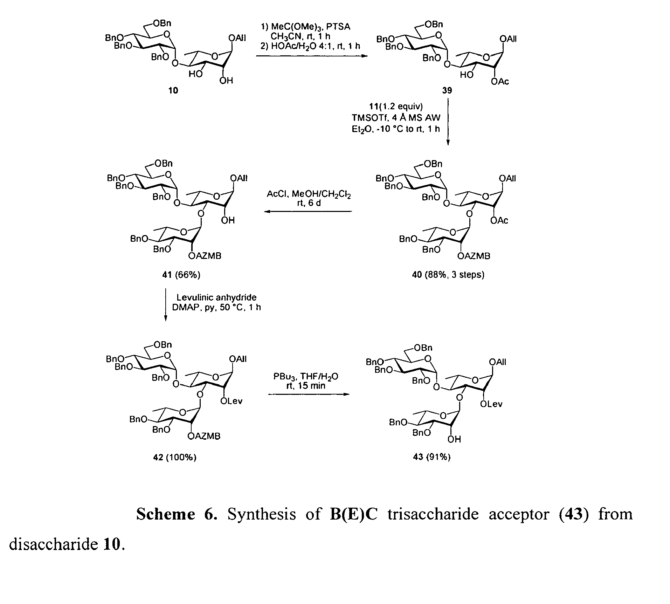

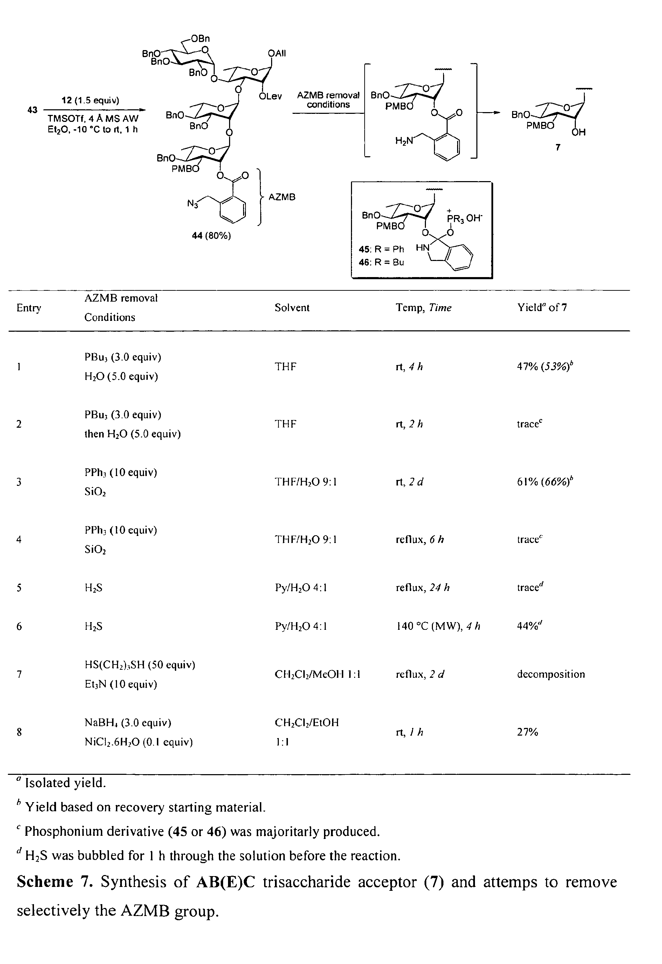

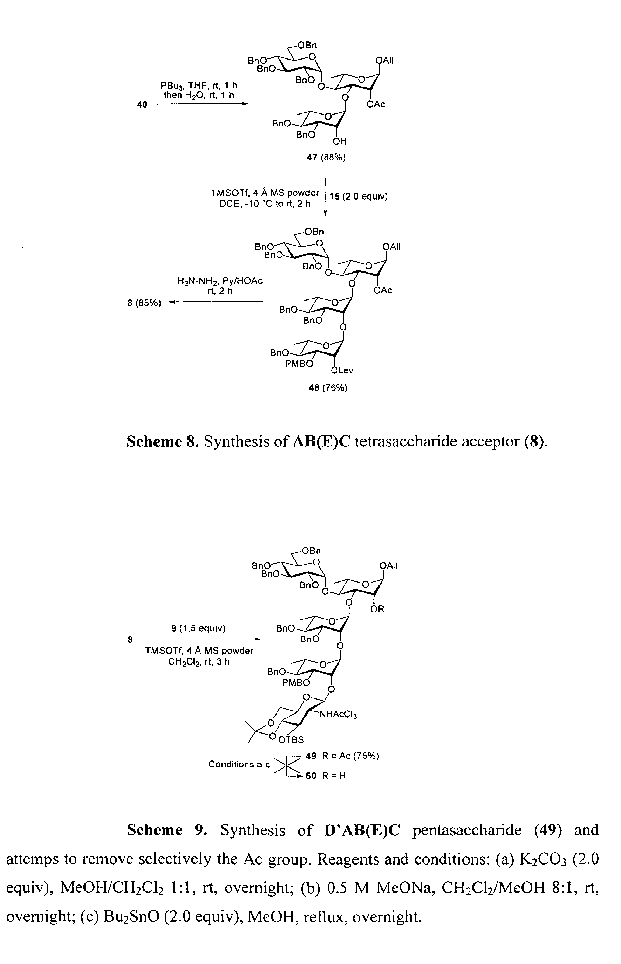

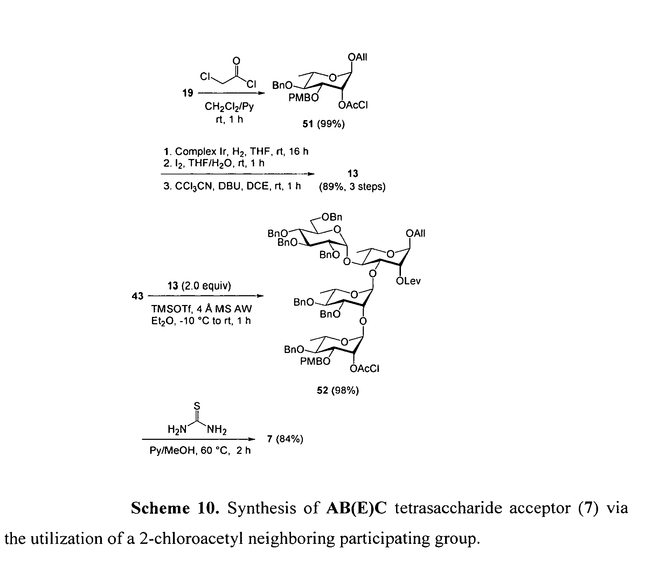

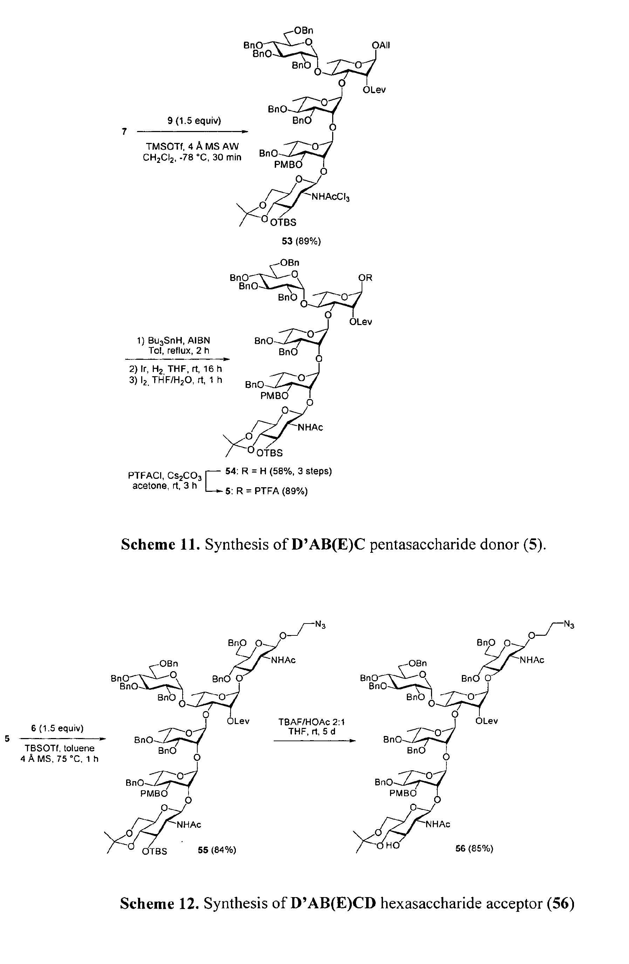

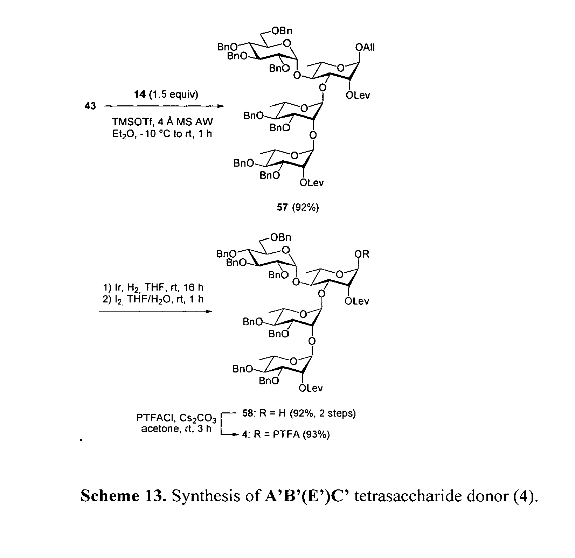

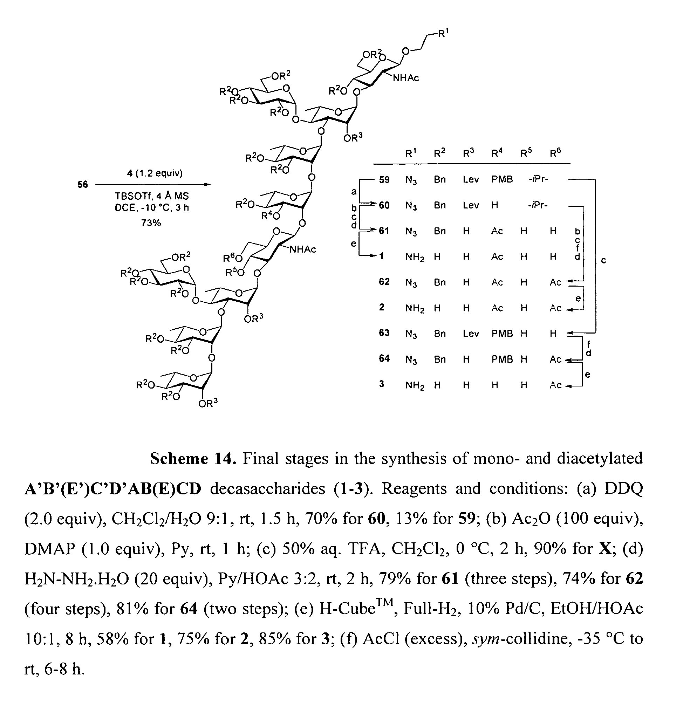

A first synthetic oligosaccharide-based conjugate has thus been proposed as a vaccine candidate against SF2a.¹² It is of note that the synthetic pentadecasaccharide hapten ([**AB**(**E**)**CD**]₃) selected in this vaccine construct, and in others,¹³ corresponds to a non O-acetylated segment of the SF2a O-Ag made of three consecutive basic O-Ag repeating units (Figure 1b). Interestingly, this pentadecasaccharide was also identified as a potent structural mimic of the SF2a O-Ag_{.}¹⁴

Besides, it is worth noting that although O-acetylation had been noticed when the repeating unit of the SF2a O-Ag was first determined,¹⁵ the exact pattern of O-acetylation, that is the number and setting of O-acetyl groups present in the repeat, was disclosed only recently (Figures 1c and 1d).^{16,17} Although not a general trend, both the occurrence and position of O-acetyl groups on a polysaccharide antigen may critically impact on the immune response induced against this antigen. Indeed, available data suggest that the input of polysaccharide O-acetylation on immunogenicity does not obey any rule. For example, the importance of O-acetylation on polysaccharide immunogenicity was reported for *Salmonella typhi*¹⁸ and *Neisseria meningitidis* A.¹⁹ Furthermore, polysaccharide O-acetylation is thought to play a key role in the case of Group B *Streptococcus* infection,²⁰ but it was not found essential to the ability of the polysaccharide antigen to induce a functional antibody response in the case of *Streptococcus pneumoniae* type 9V,²¹ while a misdirecting effect of the O-acetate side groups was hypothesized in the case of *N. meningitidis* C.²²

Besides, preliminary data obtained by use of the chemically O-deacetylated LPS suggested partial loss of antigenicity, and as outlined by the authors, emphasized the need for a more detailed study.¹⁶ Interestingly, the SF2a O-Ag acetylation pattern is shared, at least in part, by the *S*. *flexneri* 1a and *S*. *flexneri* 1b O-Ags.¹⁷

Accordingly, in the absence of any convincing general trend and in view of developing a potent synthetic oligosaccharide-based SF2a vaccine, the input of the newly disclosed SF2a O-Ag acetylation pattern on immunogenicity thus deserved to be carefully investigated.

Moreover, following a recent clinical trial, O-acetylation was provided as a likely explanation for the cross-protection induced against several non-vaccine *S. flexneri* serotypes, among which serotype 6, in recipients of a detoxified SF2a LPS-conjugate vaccine.⁷ In the context of the need for a broad serotype coverage vaccine, this observation does support further investigation on the relationship between O-Ag O-acetylation and immunogenicity.

The present invention in one aspect provides synthetic acetylated decasaccharides {A'B'(E')C'D'AB(E)CD}-O-R of formula (I) which mimic the possible intra-chain O-acetylation patterns of the SF2a O-Ag and which exhibit antigenicity. 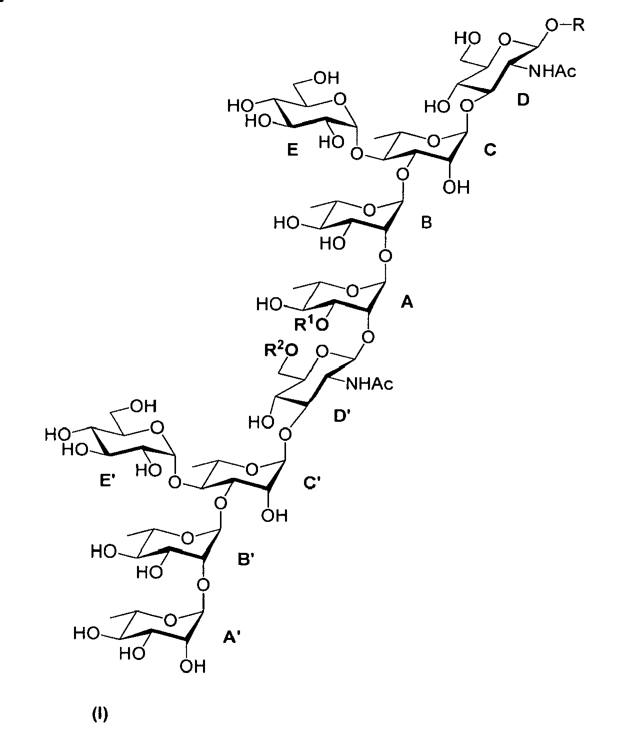 Wherein R, R¹ and R² are as defined infra.

Another object of the present invention is to provide a conjugate molecule comprising a decasaccharide residue {A'B'(E')C'D'AB(E)CD}-O- wherein A', B', E', C', D', A, B, E, C, and D are as defined in formula (I) supra, said decasaccharide residue being covalently bound to a carrier.

Another object of the present invention is to provide a kit for the diagnostic of *Shigella* infection, wherein said kit comprises a conjugate molecule according to the invention.

Another object of the present invention is to provide a method of preparation of these decasaccharides of formula (I) based on a commonly fully protected decasaccharide intermediate of formula (II). 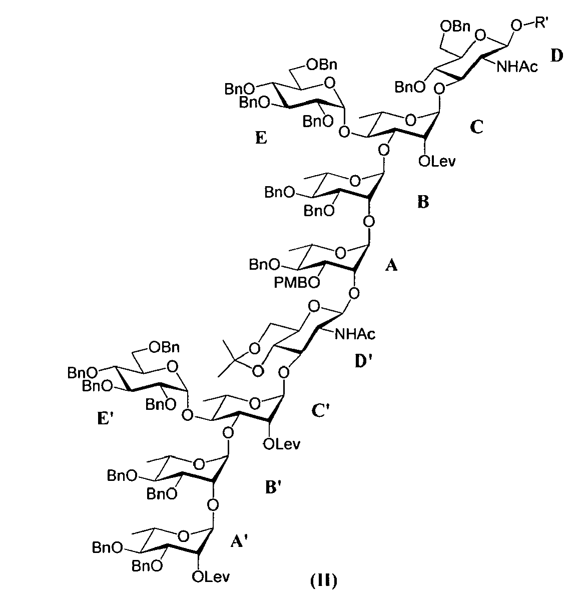 Wherein R' is as defined infra.

This intermediate is particularly advantageous as it allows, according to the used conditions of deprotection, to gain access to various specifically O-acetylated oligosaccharides mimicking SF2a O-antigen, in only a few steps along with satisfactory yields.

Another object of the present invention is to provide a method of preparation of the intermediate decasaccharides of formule (II).

Yet another object of the invention is to provide novel saccharides intermediates which are useful for the preparation of intermediate decasaccharides of formula (II), namely a hexasaccharide of formula (III), a pentasaccharide D'AB(E)C of formula (V) and a tetrasaccharide A'B'(E')C' of formula (IV) :

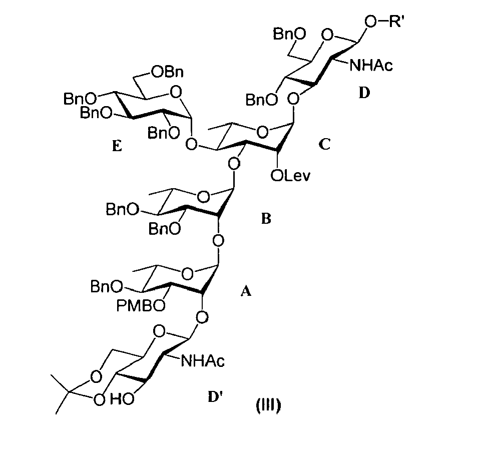

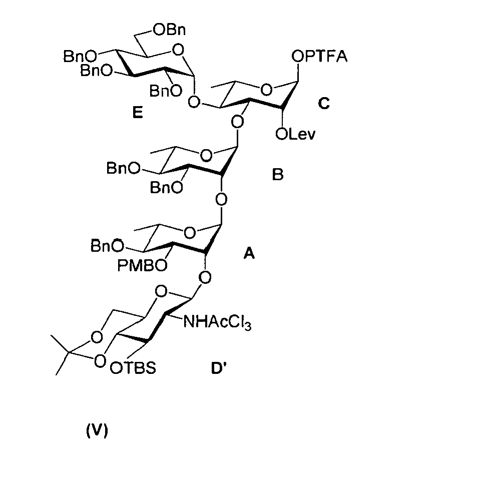

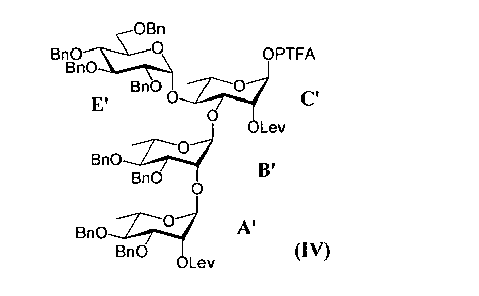

These and other objects, features and advantages of the invention will be disclosed in the following detailed description of the patent disclosure.

According to a first aspect, the invention provides decasaccharides {A'B'(E')C'D'AB(E)CD}-OR of formula (I):

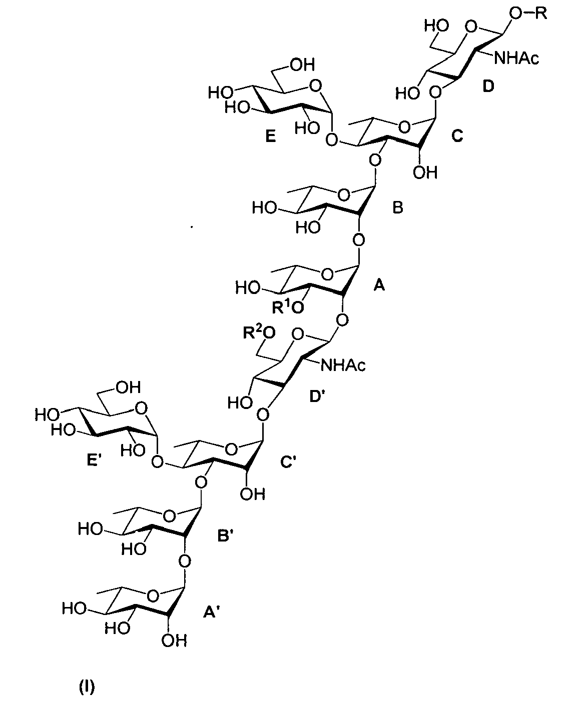

Wherein:
R is H, or -Alk-Z;
Z is a group selected from NH₂, NHR₃, -C(=NH)-OR₃, -C≡N, -C(=O)OH, -C(=O)-R₃, -CH(OR₃)(OR₄), -CH(SR₃)(OR₄), -CH(SR₃)(SR₄), -CH=CH-R₃, -C≡C-R₃, -N=C=O, -N=C=S, -SCO-R₃, -SH, -S-S-R₃, -N₃, -CONHNH₂, - NH-NH₂, an epoxyde group, or halogen,
Alk is a C₁-C₁₂ alkylene group ;
R₃, R₄ are each independently H, C₁-C₆ alkyl or C₂-C₈ alkenyl, or R₃ and R₄ together form with the atoms to which they are attached, a 5 to 7 membered heterocycloalkyl group;
R₁ and R₂ are each independently selected from H or CH₃C(=O)- (noted Ac), with the proviso that at least one of R₁ or R₂ is Ac ;

Thus, in the above formula (I), A, B, C, D, E, A', B', C', D'and E' have the following meanings:
A' is an alphaLRha*p*-(1,2) residue,
B and B' are an alphaLRha*p*-(1,3) residue,
E and E' are an alphaDGlc*p*-(1,4) residue,
C and C' are an alphaLRha*p*-(1,3) residue,
D' is a betaDGlc*p*NAc-(1,2) residue or a betaD[6-OAc]Glc*p*NAc-(1,2) residue,
A is an alphaLRha*p*-(1,2) residue or an alphaL[3-OAc]Rha*p*-(1,2) residue,
D is a betaDGlc*p*NAc-(1,2) residue,
E is branched to C,
E' is branched to C',
It being understood that at least one of D' or A is acetylated.

It is worth noting that the O-acetyl group at posision 3 on the Rha*p* A residue is likely to be in equilibrium with an O-acetyl group at position 4 on the Rha*p* A residue in solution¹⁷

Preferably, Alk is -(CH₂)ₙ- with n being an integer from 1 to 10, and more preferaby n is 2.

Preferably, Z is NH₂.

Preferably, R is -(CH₂)ₙ-NH₂, more preferably -(CH₂)₂-NH₂.

Decasaccharides of formula (I) are notably those of formula (Ia), (Ib) or (Ic):

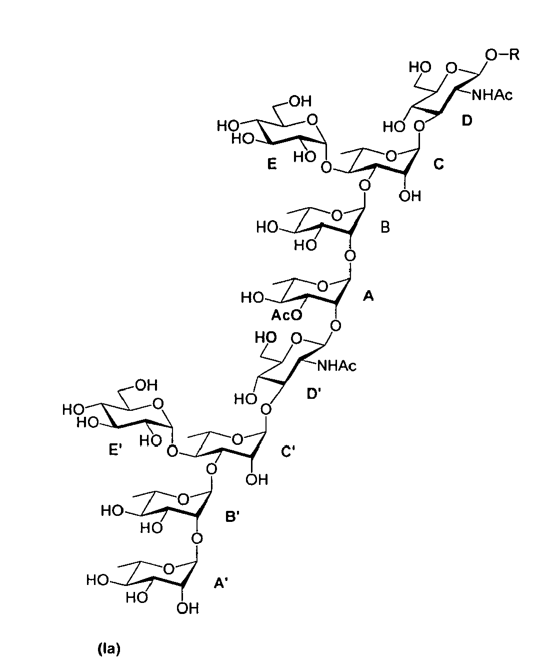

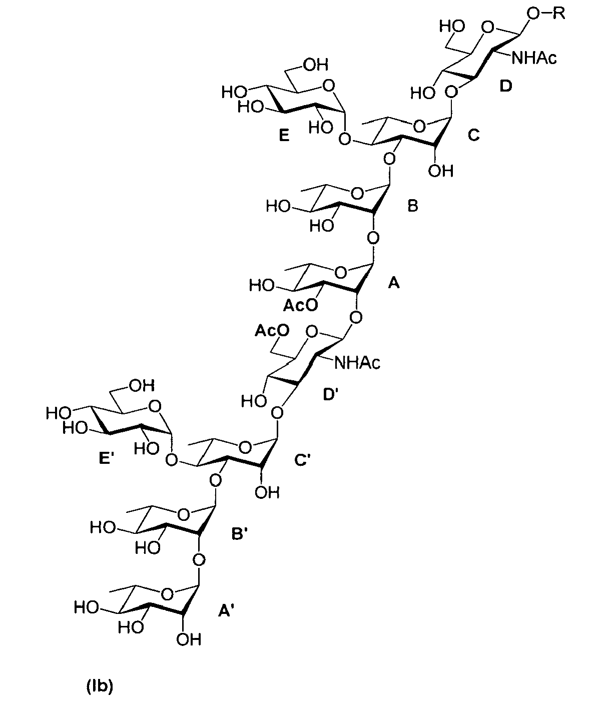

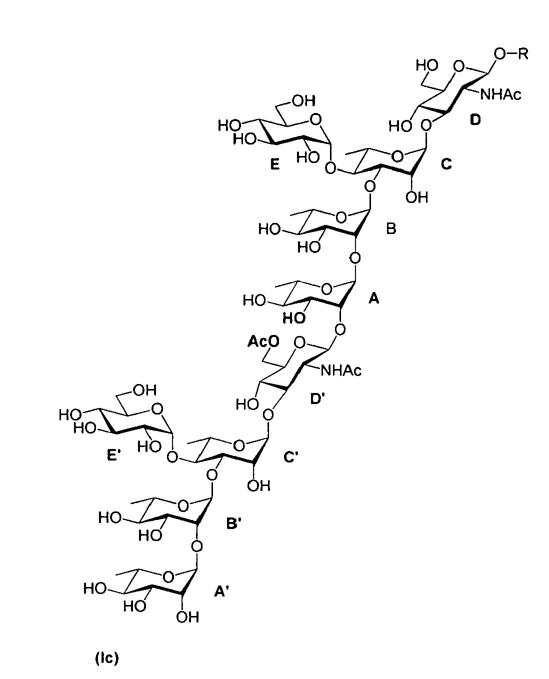

### Decasaccharide conjugates (glycoconjugates)

According to another object, the invention provides a conjugate molecule comprising a decasaccharide residue {A'B'(E')C'D'AB(E)CD}-O-, wherein A', B', E', C', D', A, B, E, C, and D are as defined in formula (I) hereabove, said decasaccharide residue being covalently bound to a carrier, notably to a peptide or protein/multimeric carrier, either directly or via a spacer group.

Methods for binding oligo- and/or polysaccharides to a protein are well known in the art. For example, in U.S. Patent 5,204,098 and U.S. Patent 5,738,855 it is taught that an oligo- or polysaccharide containing at least one carboxyl group, through carbodiimide condensation, may be thiolated with cystamine, or aminated with adipic dihydrazide, diaminoesters, ethylenediamine and the like. Groups which could be introduced by the method, or by other methods known in the art, include thiols, hydrazides, amines and carboxylic acids. Both the thiolated and the aminated intermediates are stable, may be freeze dried, and stored in cold. The thiolated intermediate may be reduced and covalently linked to a polymeric carrier containing a disulfide group, such as a 2-pyridyldithio group. The aminated intermediate may be covalently linked to a polymeric carrier containing a carboxyl group through carbodiimide condensation.

The decasaccharides residue of the invention can be covalently bound to a carrier with or without a linking molecule (also called spacer group). To conjugate without a linker, for example, a carboxyl-group-containing decasaccharide of formula (I) (that is wherein Z is C(=O)OH) and an amino-group-containing carrier are mixed in the presence of a carboxyl activating agent, such as a carbodiimide, in a choice of solvent appropriate for both the oligo- or polysaccharide and the carrier, as is known in the art (Szu, S.C., A.L. Stone, J.D. Robbins, R. Schneerson, and J.B. Robbins, 1987, Vi capsular polysaccharide-protein conjugates for prevention of typhoid fever. J Exp. Med., 166:1510-1524).

The decasaccharide of formula (I) is preferably conjugated to a carrier using a linking molecule. A linker or crosslinking agent, as used in the present invention, is preferably a small linear molecule having a molecular weight of approximately <500 daltons.

To conjugate with a linker or crosslinking agent, either or both of the decasaccharide of formula (I) and the carrier may be covalently bound to a linker first. The linkers or crosslinking agents are homobifunctional or heterobifunctional molecules, (see references provided in Bioconjugate Techniques, G. T. Hermanson, Ed, Academic Press, San Diego, 1995). *e.g.*, adipic dihydrazide, ethylenediamine, cystamine, N-succinimidyl 3-(2-pyridyldithio)propionate (SPDP), N-succinimidyl-[N-(2-iodoacetyl)-β-alanyl] propionate-propionate (SIAP), succinimidyl 4-(N-maleimidomethyl)cyclohexane-1-carboxylate (SMCC), 3,3'-dithiodipropionic acid, and the like.

According to the type of bonding between the decasaccharide residue and the carrier, there is the possibility of preparing a conjugate molecule wherein the ratio of the decasaccharide residue versus the carrier can vary between 1:1 and 30:1. Preferably, this ratio is comprised between 5:1 and 20:1.

A carrier can be a natural, modified-natural, synthetic, semisynthetic or recombinant material containing one or more functional groups, for example primary and/or secondary amino groups, azido groups, thiol groups, or carboxyl groups. The carrier can be water soluble or insoluble. Carriers that fulfil these criteria are well-known to those of ordinary skill in the art.

### Diagnosis

According to another object, the present invention provides a kit for the diagnostic of *Shigella* infection, notably *Shigella flexneri,* more particularly *Shigella flexneri* type 2a infection, wherein said kit comprises a conjugate molecule according to the invention.

The decasaccharide conjugates according to the present invention can be used to test the presence of *Shigella,* notably *Shigella flexneri,* more particularly *S*. *flexneri* type 2a-specific antibodies. Decasaccharide conjugates according to the invention may be used for epidemiological studies, for example for determining the geographic distribution and/or the evolution of *S*. *flexneri* type 2a infection worldwide, as well as for evaluating the *S*. *flexneri* type 2a-specific antibody response induced by an immunogen.

The decasaccharide conjugates according to the present invention may be advantageously labelled and/or immobilized onto a solid phase, according to standard protocols known to the man skilled in the art. Such labels include, but are not limited to, luminescent or fluorescent molecules. For example a decasaccharide conjugated to biotine, according to the present invention may be immobilized onto a solid phase, to detect the presence of *S*. *flexneri* type 2a-specific antibodies in biological samples.

Such immunoassays include, but are not limited to radioimmunoassay, enzyme-linked immunosorbent assays, fluorescence assays, western-blots and the like.

Such assays may be for example, of direct format (where the labelled antibody / decasaccharide is reactive with the antigen/antibody to be detected), an indirect format (where a labelled secondary antibody is reactive with said antibody/ decasaccharide), a competitive format (addition of a labelled antibody/ decasaccharide), or a sandwich format (where both labelled and unlabelled antibodies are used).

For all diagnostic uses, the decasaccharide conjugates of the invention, alone or linked to a carrier, as well as antibodies and other necessary reagents and appropriate devices and accessories may be provided in kit form so as to be readily available and easily used.

### Decasaccharide intermediate of formula (II):

In another object, the invention provides a decasaccharide {A'B'(E')C'D'AB(E)CD}-OR' of formula (II): 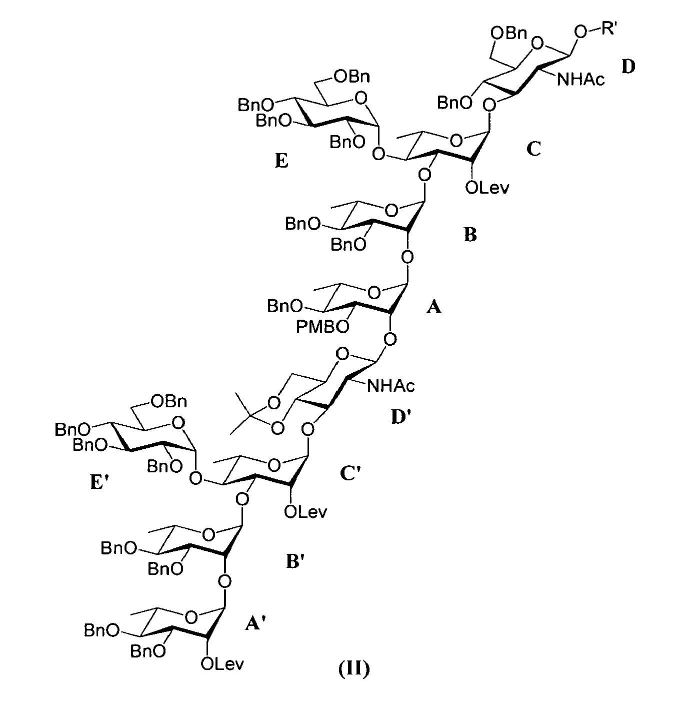 Wherein:
R' is R as defined above in formula (I) or a precursor thereof.

This decasaccharide is a synthetic intermediate which advantageously allows to obtain decasaccharides of formula (I) in only a few steps together with satisfactory yields.

More specifically, it has been advantageously demonstrated that the PMB and isopropylidene (-iPr-) protecting groups, could be each independently removed, thus allowing the regioselective O-acetylation of the 3_{A} and/or 6_{D'} hydroxyl moieties. This was particularly unexpectable as both PMB and (-iPr-) protecting groups are known to be sensitive to acid conditions and hence have been rarely used as orthogonal groups for the deprotection of isopropylidene selectively to PMB.

R' is preferably a group Alk-Z', wherein Z' is a precursor group of Z, wherein Alk and Z are as defined in formula (I) hereabove.

According to the present invention, the expression "precursor group" of a functional group refers to any group which can, by one or more chemical reactions, lead to the desired function, by means of one or more suitable reagents. Those chemical reactions include deprotection, as well as usual addition, substitution, reduction or functionalization reactions. Examples of precursor groups notably include -N₃, -CN, -NCbz (-NH-C(=O)O-CH₂Ph), -S-SR₃, -CH₂CH=CH₂, -C(=O)OR₃, - CH(OR₃)(OR₄), -CH(SR₃)(OR₄), -CH(SR₃)(SR₄), wherein R₃ and/or R₄ are as defined in formula (I) hereabove.

Preferably, R' is a precursor group of -Alk-NH₂, notably -Alk-N₃, in particular -(CH₂)ₙ-N₃, wherein n is as defined in formula (I) hereabove.

Preferably, R' is -(CH₂)₂-N₃.

Method of preparation of decasaccharides of formula (I) from decasaccharides of formula (II)

In another object, the invention provides a method for preparing a decasaccharide of formula (I) as defined above, said method comprising the steps consisting of:
(i) deprotection of the PMB and/or (-iPr-) groups on the 3_{A} and/or 6_{D'} hydroxyl moieties of a compound of formula (II) according to the invention;
(ii) acetylation of the resulting deprotected 3_{A} and/or 6_{D'} hydroxyl moieties;
(iii) cleavage of all remaining protecting groups in the obtained compound; and optionally
(iv) conversion of R' into R, thereby obtaining the decasaccharides of formula (I).

Preferably, the method of the invention further comprises the subsequent step (v) of recovering the obtained decasaccharides of formula (I).

As used herein the expression "3_{A} and/or 6_{D'} hydroxyl moieties" refers to the hydroxyl functions located on the C₃ and C₆ position in the glucosyl units A and D', respectively.

### Step i)

According to a particular embodiment, step i) is an orthogonal deprotection step. As used herein, the expression "orthogonal deprotection" referring to PMB and (-iPr-) groups, means that under specific reaction conditions, it is possible to chemoselectively remove one of the protecting groups, either PMB or (-iPr-) groups, and thus to differentiate the 3_{A} and 6_{D'} hydroxyl functions of the compound of formula (II).

In a preferred embodiment, the PMB orthogonal deprotection step on the 3_{A} hydroxyl moiety of the compound of formula (II) is performed in the presence of 2,3-dichloro-5,6-dicyanobenzoquinone (DDQ) in CH₂Cl₂. Under these conditions, only PMB is removed, while the isopropylidene group remains intact.

In another preferred embodiment, the deprotection of the isopropylidene group on the 6_{D'} hydroxyl moiety of the compound of formula (II) is performed in the presence of 50% aqueous trifluoroacetic acid (TFA). Under these conditions, only (-iPr-) is removed, while the PMB group remains intact.

### Step ii)

According to a preferred embodiment, in step ii), the deprotected 3_{A} hydroxyl moiety of the compounds of formula (II) can be acetylated in the presence of Ac₂O and dimethylaminopyridine (DMAP).

In another preferred embodiment, in step ii), the deprotected 6_{D'} hydroxyl moiety of the compounds of formula (II) - which is a primary alcohol- can be regioselectively acetylated with regard to the 4_{D'} deprotected secondary alcohol by using a molar excess of acetyl chloride in *sym*-collidine.

### Step iii)

According to a preferred embodiment, the remaining Lev (Levulinoyl) groups are cleaved in the presence of hydrazine hydrate in a mixture of pyridine (Py) and AcOH.

Preferably, any remaining -iPr- group is cleaved in the presence of 50% aqueous trifluoroacetic acid (TFA).

Preferably, the remaining benzyl groups (Bn) are cleaved by palladium-catalyzed hydrogenolysis, notably in the presence of 10% Pd/C, EtOH/HOAc 10:1.

It is worth noting that when R' is -Alk-N₃ in formula (II), the palladium-catalyzed hydrogenolysis of the Bn groups allows the concomitant conversion of the azido group -Alk-N₃ into the corresponding amine -Alk-NH₂, ie step (iv).

In a preferred embodiment, the preparation of the decasaccharide of formula (Ia) comprises the steps of:
i) deprotection of the PMB group on the 3_{A} hydroxyl moiety of the compound of formula (II);
ii) acetylation of the resulting deprotected 3_{A} hydroxyl moiety; and
iii) cleavage of all remaining protecting groups ; and
iv) conversion of R' into R, thereby obtaining the decasaccharide of formula (Ia).

In another preferred embodiment, the preparation of the decasaccharide of formula (Ib) comprises the steps of:
i) deprotection of the PMB group on the 3_{A} hydroxyl moiety of the compound of formula (II) ;
ii) acetylation of the resulting deprotected 3_{A} hydroxyl moiety;
iii) deprotection of the isopropylidene group on the 6_{D'} hydroxyl and the 4_{D'} hydroxyl moieties of the compound of formula (II) ;
iv) regioselective acetylation of the 6_{D'} hydroxyl moiety ; and v) cleavage of all remaining protecting groups to produce the decasaccharide of formula (Ib).

In another preferred embodiment, the preparation of the decasaccharide of formula (Ic) comprises the steps of:
i) deprotection of the isopropylidene groups on the 4_{D'} and 6_{D'} hydroxyl moieties of the compound of formula (II);
ii) regioselective acetylation of the resulting deprotected 6_{D'} hydroxyl moiety; and
iii) cleavage of all remaining protecting groups to produce the decasaccharide of formula (Ic).

In another object, the invention provides the use of a decasaccharide of formula (II) for the preparation of a decasaccharide of formula (I).

Method of preparation of decasaccharide of formula (II)

In another object, the invention provides a method of preparation of a decasaccharide of formula (II), said method comprising the steps of:
i) coupling the hexasaccharide D'AB(E)CD of formula (III), wherein R' is as defined hereabove, and the tetrasaccharide A'B'(E')C' of formula (IV), according to a glycosylation reaction; and optionally
ii) recovering the obtained decasaccharide of formula (II).

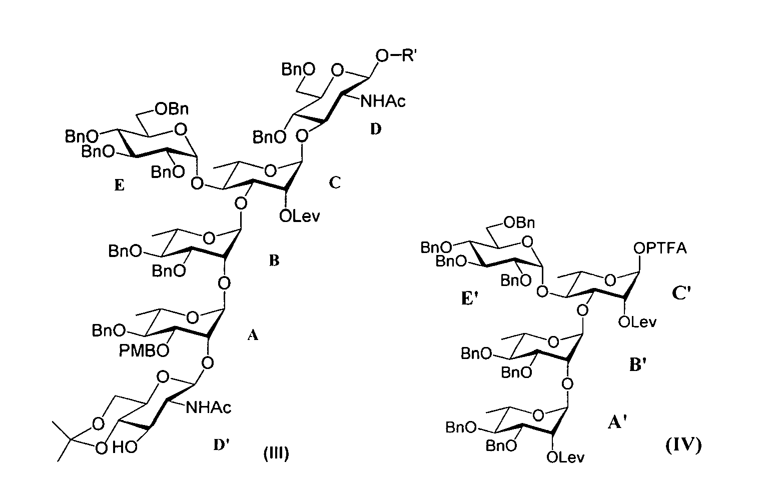

Preferably, the glycosylation reactions herein described are performed in the presence of a promoter such as *tert*-butyldimethylsilyl trifluoromethanesulfonate (TBSOTf), trimethylsilyl trifluoromethanesulfonate (TMSOTf), triflic acid (TfOH), triflic anhydride (Tf₂O), or boron trifluoride diethyletherate (BF₃.OEt₂).

Preferably, the reaction is carried out in a solvent, notably a polar aprotic solvent, such as 1,2-dichloroethane (DCE) or diethylether (Et₂O). The reaction may also be carried out in toluene as a solvent.

In a preferred embodiment, the reaction is performed under anhydrous conditions, preferably in the presence of powdered molecular sieves.

The reaction may be performed in a wide range of temperature, notably in the range of -10°C to 25°C.

Preferably the hexasaccharide D'AB(E)CD of formula (III) is prepared by the method comprising :
i) reacting the pentasaccharide D'AB(E)C of formula (V) with the monosaccharide D of formula (VI) according to a glycosylation reaction: 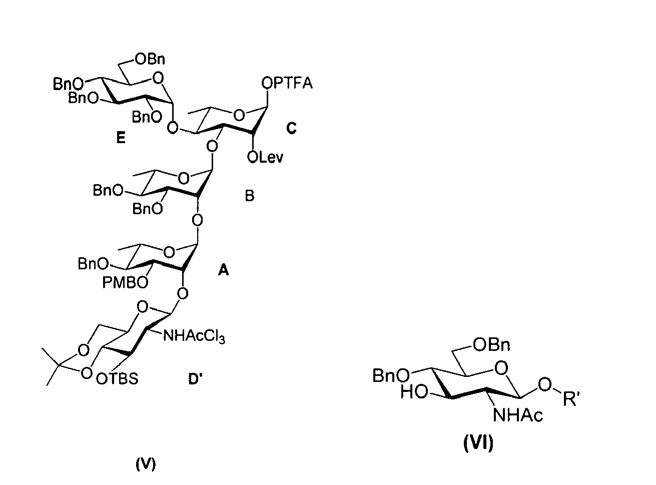 Wherein R' is as defined hereabove for formula (II), and
ii) deprotecting the 3_{D'} hydroxyl group in the obtained hexasaccharide.

Preferably, the pentasaccharide D'AB(E)C of formula (V) is prepared by a method comprising :
i) reacting a tetrasaccharide AB(E)C of formula (VII) with a monosaccharide D' of formula (VIII) according to a glycosylation reaction: 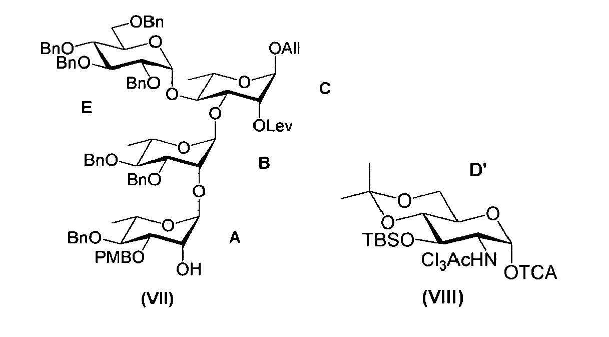
ii) deprotecting the 1_{C} hydroxyl group of the obtained pentasaccharide ; and
iii) converting the deprotected 1_{C} hydroxyl group into a -OPTFA group.

It is worth noting that the PTFA group could be used instead of the TCA group.

Preferably, the tetrasaccharide AB(E)C of formula (VII) is prepared according to a method comprising :
i) reacting a trisaccharide B(E)C of formula (IX) with a monosaccharide A of formula (X) according to a glycosylation reaction: 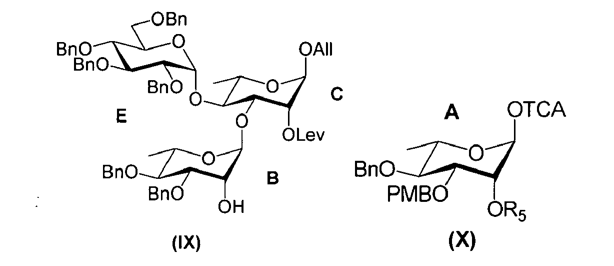 Wherein R₅ is AZMB or chloroacetyl (AcCl),
   and
ii) deprotecting the 2_{A} hydroxyl group in the obtainedtetrasaccharide, that is removing R₅.

Preferably, the trisaccharide B(E)C of formula (IX) is prepared by a method comprising :
i) reacting a disaccharide (E)C of formula (XI) with a monosaccharide B of formula (XII) : 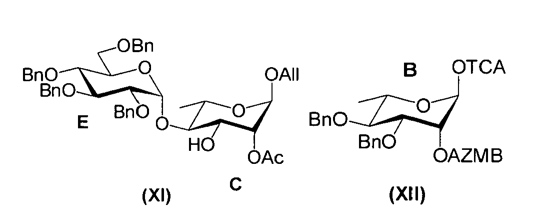
ii) deprotecting the 2_{C} hydroxyl group in the obtained trisaccharide ;
iii) protecting the deprotected 2_{C} hydroxyl group with a Lev group ; and
iv) deprotecting the 2_{B} hydroxyl group.

In another object, the invention provides a hexasaccharide D'AB(E)CD of formula (III):

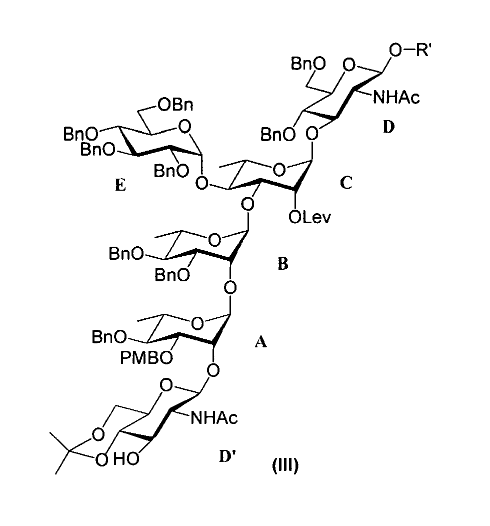

In another object, the invention provides the pentasaccharide D'AB(E)C of formula (V):

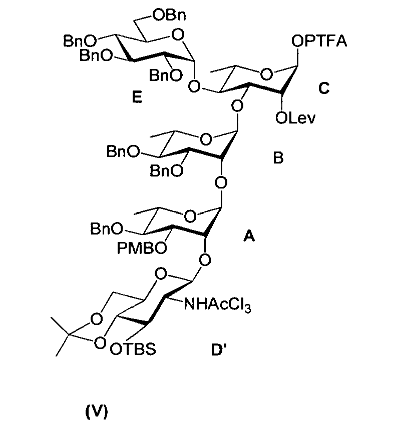

In another object, the invention provides the tetrasaccharide A'B'(E')C' of formula (IV):

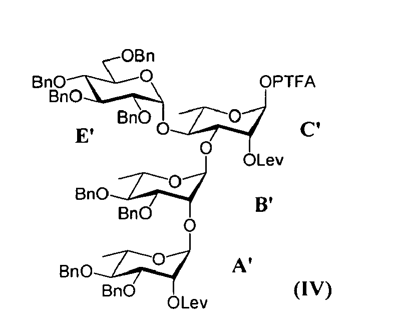

These oligosaccharides (IV) and (V) are particularly useful for the preparation of the decasaccharide intermediates of formula (II).

### Definitions

The following abbreviations, terms and expressions contained herein are defined as follows:

### Abbreviation:

LPS: lipopolysaccharide;
O-SP: O-specific polysaccharide;
DCC: dicyclohexyl carbodiimide;
Rha*p*: rhamnopyranosyl;
Glc*p*: glucopyranosyl;
GlcNA*cp*: 2-acetamido-2-deoxy-glucopyranosyl;
Ac: acetyl: CH₃C(=O)-;
AcCl: chloroacetyl: ClCH₂C(=O)-;
All: allyl;
AZMB: 2-(azidomethyl)benzoyl;
Bn: benzyl;
(-iPr-): isopropylidene ;
Lev: levulinoyl;
PMB: *para*-methoxybenzyl;
PTFA: *N*-(phenyl)trifluoroacetimidoyl;
TBS: *tert*-butyldimethylsilyl;
TCA: trichloroacetimidoyl.

As used herein, the term "alkyl" refers to a straight-chain, or branched alkyl group having 1 to 8 carbon atoms, such as methyl, ethyl, propyl, isopropyl, butyl, isobutyl, *sec*-butyl, *tert*-butyl, pentyl, isoamyl, neopentyl, 1-ethylpropyl, 3-methylpentyl, 2,2-dimethylbutyl, 2,3-dimethylbutyl, hexyl, octyl, etc. Lower alkyl groups, which are preferred, are alkyl groups as defined above which contain 1 to 4 carbons. A designation such as "C₁-C₄ alkyl" refers to an alkyl radical containing from 1 to 4 carbon atoms.

As used herein, the term "alkylene" refers to a branched or straight chained hydrocarbon of 1 to 8 carbon atoms, which is formed by the removal of two hydrogen atoms. A designation such as "C₁-C₄ alkylene" refers to an alkylene radical containing from 1 to 4 carbon atoms. Examples include notably methylene (-CH₂-), propylidene (CH₃CH₂CH=), 1,2-ethanediyl (-CH₂CH₂-).

As used herein, the term "alkenyl" refers to a straight chain, or branched hydrocarbon chains of 2 to 8 carbon atoms having at least one carbon-carbon double bond. A designation "C₂-C₈ alkenyl" refers to an alkenyl radical containing from 2 to 8 carbon atoms. Examples of alkenyl groups include notably ethenyl, propenyl, isopropenyl, 2,4-pentadienyl.

As used herein, the term "halogen" refers to Cl, Br, I or F.

As used herein, the term "heterocycloalkyl" refers to a cycloalkyl group in which one or more ring carbon atoms are replaced by at least one hetero atom such as -O-, -N-, or -S-. The heterocycloakyl groups can be susbstituted by one to three alkyl groups.

As used herein, the term "carrier" refers to any molecule which can be covalently bound to a decasaccharide of formula (I) of the invention to form the glycoconjugates of the invention. It includes notably carriers for preparing diagnostic reagents.

As used herein, the expression "carriers for preparing diagnostic reagents" refers to agents commonly used to immobilize molecules onto a solid phase or to label molecules.

As used herein, the term "a label" refers to any substance which can produce a signal which can be detected by any appropriate mean.

As used herein, the term "glycoconjugate" refers to the decasaccharide of formula (I) covalently bound to a carrier.

Other features of the invention will become apparent in the course of the following description of exemplary embodiments. These examples are given for illustration of the invention and are not intended to be limiting thereof.

### EXAMPLES

### Results and Discussion

Based on previous studies regarding the preparation of non-acetylated dimer and trimer of the branched pentasaccharide repeating unit of *S. flexneri* 2a O-antigen,^{11,27} the mono- and diacetylated decasaccharides **1-3** can be retrosynthetically disconnected into three suitably protected synthons, namely the pentasaccharide donor **D'AB(E)C** (**5**), the tetrasaccharide donor **A'B'(E')C'** (**4**), and the GlcNAc acceptor **D** (**6**). The preparation of synthon **5** would involve the glycosylation of the tetrasaccharide acceptor **AB(E)C** (**7** or **8**) with the glucosaminyl donor **D'** (**9**), while all the tetrasaccharides (**4, 7** and **8**) would be synthesized from coupling between the known disaccharide acceptor (**E**)**C** (**10**) and the rhamnose acceptors **A** or **A' 11-16**. It is worth mentioning that the azidoethyl spacer, precursor of the aminoethyl function, was introduced at the reducing end of residue **D** (**6**) in order to allow further site-selective conjugations, including with a carrier.¹¹ To ensure complete 1,2-*trans*-stereoselectivity during glycosidic bond formation, levulinoyl esters (Lev)²⁸ were chosen as permanent C-2 neighbouring participating groups in view of their orthogonality to acetyl (Ac) groups. Other participating ester groups such as 2-chloroacetyl (AcCl) and the recently disclosed 2-(azidomethyl)benzoyl (AZMB),²⁹ which can be selectively cleaved in the presence of Lev groups, were also employed when necessary. In order to protect the amine function of **D'** donor **9**, the trichloroacetyl moiety (Cl₃Ac)³⁰ was selected since it was shown to provide good stereoselectivity in glycosylation reaction via the formation of a 2-trichloromethyloxazoline intermediate.^{31,32} Moreover, the trichloroacetamide function (NHAcCl₃) can be easily converted to an acetamido (NHAc) group by the concomitant action of tributylstannane (Bu₃SnH) and azobisisobutyronitrile (AIBN) in refluxing toluene.³⁰⁻³² Importantly, the *para*-methoxybenzyl (PMB) and isopropylidene (-iPr-) groups were used for the temporary protection of the 3_{A} and 6_{D'} hydroxyl functions, respectively, in order to allow acetylation at these positions at a later stage of the synthesis. Concerning the anomeric position, the allyl (All) group was chosen for temporary protection since it can be easily removed under mild conditions to regenerate the hemiacetal function. Furthermore, the formation of all glycosidic linkages was based on the trihalogenoacetimidate chemistry. In fact, trichloroacetimidates (TCA)³³ were used for the activation of monosaccharide donors (**9, 11-15**) while *N*-(phenyl)trifluoroacetimidates (PTFA)^{34,35} were selected for the activation of longer sequences, namely tetra- and pentasaccharide donors **4** and **5**, in view of their greater stability to acidic conditions. Finally, benzyl (Bn) groups were used as permanent protecting groups for other hydroxyl functions whenever possible.

The starting point of the synthesis consisted in the preparation of the conveniently protected rhamnose donors **11** and **12**. Accordingly, diol **16**, which was obtained in high yields (86%) at the molar scale (>100 g) following a four-step reported procedure,³⁶ was treated with dibutyltin oxide (Bu₂SnO) in refluxing toluene.³⁷ As shown in Scheme 1, the resulting stannylene acetal intermediate **17** was reacted with benzyl bromide (BnBr) in the presence of both caesium fluoride (CsF) and tetrabutylammonium iodide (TBAI) to yield the known **18**³⁸ (88%) benzylated at both the C-3 and C-4 positions. Regioselective *para*-methoxybenzylation of diol **16** under similar reaction conditions (PMBCl, CsF, TBAI) allowed the formation of the known alcohol **19**³⁹ in good yield (82%). Since an AZMB participating group was planned to be introduced at the C-2 position, the 2-(azidomethyl)benzoyl chloride reagent (AZMBCI, **22**) had to be synthesized following a slightly modified reported procedure (Scheme 2).^{40,41} Briefly, methyl 2-methylbenzoate (**18**) was subjected to Wohl-Ziegler's bromination⁴² to afford **19** in which the bromine was substituted by an azido group (→**20**). The methyl ester function of the latter was saponified under basic conditions allowing the formation of 2-(azidomethyl)benzoic acid (AZMBOH, **21**)²⁹ in 77% yield after three steps. Treatment of AZMBOH (**22**) in refluxing tionyl chloride (SOCl₂) generated AZMBCl (**22**)⁴¹ quantitatively. Then, as depicted in Scheme 3, both alcohols **18** and **19** were reacted with either AZMBCl (**22**) in the presence of 4-dimethylaminopyridine (DMAP) (route a)⁴⁰ or AZMBOH (**21**) in conjunction with dicyclohexylcarbodiimide (DCC) and DMAP (route b)⁴³ to give rhamnoside derivatives **23** and **24**, respectively, in yields ranging from 89-96%. The resulting allyl esters (**23** and **24**) were subsequently converted to TCA donors **11** (68%) and **12** (60%), respectively, following a three-step procedure involving: (i) isomerization of the allyl ether via the utilization of a cationic iridium complex,⁴⁴ (ii) iodine-mediated hydrolysis,⁴⁵ and (iii) activation of the anomeric position under the action of trichloroacetonitrile (CCl₃CN) and catalytic 1,8-diazabicycloundec-7-ene (DBU).³³

The **D'** donor **9** (Scheme **4**) was prepared as follows. Commercially available glucosamine hydrochloride was reacted with trichloroacetic anhydride^{46,47} in the presence of sodium methoxide (NaOMe) to form hemiacetal **25** in which the anomeric position was regioselectively protected with an α-allyl group.⁴⁸⁻⁵⁰ Then, isopropylidenation of the resulting triol (**26**) using 2-methoxypropene under the catalytic action of *para*-toluenesulfonic acid (PTSA)⁵¹ enabled the formation of compound **27** in excellent yield (91%). The latter was treated with *tert-*butyldimethylsilyl chloride (TBSCl)^{52,53} to afford the fully protected **28** (98%). Finally, selective removal of allyl aglycone followed by the activation of the anomeric position using above-mentionned reaction conditions furnished the target derivative **9** in 73% yield after three steps when performed at a 10 g scale.

The synthesis of the **D** acceptor **6** was performed according to the oxazoline chemistry recently exemplified by Bundle and co-workers.^{54,55} Interestingly, they have found that 5,6-*O*-isopropylidene glucofuranosyl oxazoline **29**^{56,57} can be readily converted to corresponding 2-acetamido-2-deoxy-β-D-glucopyranosides with complete 1,2-*trans*-stereoselectiviy when put into reaction with various alcohols in the presence of Brönsted acids such as PTSA and DL-camphor sulfonic acid (CSA). Moreover, even if it was also possible to synthesize unprotected glucopyranosyl oxazoline directly from *N*-acetyl-D-glucosamine as recently shown by Shoda and coworkers,⁵⁸ furanosyl oxazoline **29** was used. Indeed, it appeared to be more suitable, since protecting the 3_{C} hydroxyl group prior to undergo acid-catalyzed oxazoline opening was envisioned.⁵⁵ Therefore, as depicted in Scheme 5, oxazoline **29** obtained in one step from *N*-acetyl-D--glucosamine was reacted under standard conditions to yield three new protected oxazolines featuring TBS (**30**, 53%), PMB (**31**, 51%) or All (**33**, 77%) groups at the C-3 position. It is noticeable that the modest yields of these reactions were attributable to oxazoline degradation during silica gel flash column chromatography. Although the overnight reaction of oxazolines **30** and **31** with anhydrous 2-bromoethanol (BrEtOH) under acidic conditions (CSA, 0.25-1.0 equiv) led to the cleavage of both TBS and PMB protecting groups, the acid-catalyzed opening of C-3 allylated oxazoline **33** resulted in the formation of the target β-glycoside **34** in 55% yield when using less than 0.5 equiv of CSA. However, the reaction time needed in order to achieve complete conversion was quite long, and the use of higher amounts of CSA did not result in any increased yield. Instead, it was found that anomerization occurred with equimolar quantities of CSA affording the α-glycoside **35** in a non-negligible yield of 14% along with **34** (44%) as the major compound. Therefore, Lewis acids were used such as ytterbium (III) trifluoromethanesulfonate [Yb(OTf₃)]⁵⁹ and copper (II) chloride (CuCl₂),⁶⁰ which have been recently shown suitable promoters for the activation of pyranosyl oxazolines. Accordingly, oxazoline **33** was reacted with BrEtOH under the action of CuCl₂ or Yb(OTf)₃ (0.5-1.0 equiv). The yields were thus significantly increased (61% for CuCl₂ and 99% Yb(OTf)₃) as well as the reaction times were shorten (16 h). However, careful 1D and 2D NMR analysis showed that, unexpectedly, the ring of the β-glycosides were stuck in the *furanosyl* configuration (**36**) instead of the pyranosyl one (**34**). Several attemps were made to convert the furanoside **36** into pyranoside **34**,⁵⁶ but isolated yields were not satisfactory and anomerization occurred predominantly (data not shown). With 2-bromoethyl glycoside **34** in hand, its transformation to target acceptor **6** was easily achieved in a four-step process involving: (i) bimolecular substitution of bromine with an azido group⁶¹ (→**37**), (ii) benzylation of both C-4 and C-6 hydroxyl groups at low temperature (0 °C) in order to preserve NHAc integrity⁶² (→**38**, 57%, two steps), and (iii) selective removal of the allyl group (→**6**, 82%, two steps).

Performing the synthesis of the trisaccharide acceptor **43** was the next task. As shown in Scheme 6, the know disaccharidic diol **10**⁶³ was treated with trimethyl orthoacetate under the catalytic action of PTSA to afford the 2,3-ortho ester. The regioselective opening of the latter using 80% aqueous acetic acid (HOAc)^{64,65} proceeded smoothly giving the crude alcohol **39**, which was directly submitted to glycosylation reaction to avoid acetyl migration.⁶⁵ Thus, coupling of crude acceptor **39** with rhamnosyl donor **11** (1.2 equiv) at low temperature (-10 °C) in Et₂O under the promotion of trimethylsilyl trifluoromethanesulfonate (TMSOTf) furnished **40** in excellent yield (88%, three steps from **10**). It was next reasoned that the Ac group should be removed at this step of the synthetic route and replaced with a Lev group, which is orthogonal to acetyls and capable of anchimeric assistance. Therefore, selective deacetylation studies were undertaken on trisaccharide **40** with the aim to obtain alcohol **41.** Several deacetylation conditions were investigated such as the use of tetrafluoroboric acid (HBF₄),^{24,66} PTSA⁶⁷ or guanidine,⁶⁸ but were found unsuccessful. The use of Bu₂SnO (2.0 equiv) in refluxing MeOH (80 mL•mmol⁻¹)⁶⁹⁻⁷¹ led to the formation of the desired trisaccharide **41** (62%), but it was nevertheless difficult to increase the yield due to partial AZMB-loss at the C-2_{B} position. The best result was obtained by reacting **40** with acetyl chloride (AcCl)⁷²⁻⁷⁴ in a solution of MeOH/CH₂Cl₂. In such acidic *trans*-esterification conditions, the target trisaccharide **41** was obtained in good yield (66%) along with unreacted **40** (22%) after six days of reaction at room temperature. Levulinoylation of alcohol **41** was sluggish under usual conditions (LevOH, DCC, DMAP) affording an inseparable mixture of levulinate **42** and starting alcohol **41.** Consequently, trisaccharide **41** was treated with the more reactive levulinic anhydride ^{28,75} in the presence of DMAP giving the desired **42** in a nearly quantitative yield. Then, removal of the AZMB group under Staudinger reaction using tributylphosphine (PBu₃)^{40,76} in THF/H₂O readily furnished trisaccharide acceptor **43** in excellent yield (91%). It is important to note that the utilization of triphenylphosphine (PPh₃) instead of PBu₃ did not lead to any deprotection product (**43**).

As depicted in Scheme 7, tetrasaccharide **44** was synthesized in good yield (80%, slightly contaminated with trichloroacetamide) from the glycosylation of acceptor **43** with rhamnosyl donor **12** (1.5 equiv) catalyzed by the Lewis acid TMSOTf. Replacing Et₂O by CH₂Cl₂ or using Schmidt's inverse procedure⁷⁷ did not result in any increased condensation yield (data not shown). At this step of the synthesis, we attempted to remove the AZMB group using the above-mentionned Staudinger's conditions (PBu₃, THF/H₂O). However, the reaction was sluggish affording the desired compound **7** in only 47% yield (entry 1) along with unreacted **44** (6%). The yield was slightly increased to 61 % (entry 2) when changing PBu₃ for PPh₃ and adding silica gel (SiO₂) to the reaction mixture.⁷⁸ Surprisingly, it was found that, when water was subsequently added after the formation of the iminophosphorane intermediate (entry 2), 1-*O*-(tributylphosphonium)isoindol-1-yl derivative (**46**) was majoritarly produced (49%). Moreover, refluxing the reaction mixture led to the exclusive formation of the triphenylphosphonium derivative **45** (entry 4). Although the reasons for the formation of these unexpected polar phosphonium salts (**45** and **46**) were unclear, their structures were unambiguously proved by ¹H, ¹³C and ³¹P NMR analysis as well as by HR-ESI-TOF-MS (**45**: *mlz* 1900.8340 [M]⁺, calcd for C₁₁₅H₁₂₃NO₂₂P, 1900.8274; **46**: *m*/*z* 1840.9080 [M]⁺, calcd for C₁₀₉H₁₃₅NO₂₂P, 1841.9247). Therefore, in order to obtain exclusively the desired tetrasaccharide acceptor **7**, other azide reduction conditions (entry 5-8) were considered, which did not involve the utilization of phosphine derivatives, such as the use of hydrogen sulfide (H₂S),^{79,80} 1,3-propanedithiol⁸¹ or sodium borohydride (NaBH₄) in conjunction with nickel chloride hexahydrate (NiCl₂.6H₂O).^{82,83} Unfortunately, all these reactions provided desired alcohol **7** in unsatisfactory yields (0-44%). Although AZMB cleavage often involves mild reaction conditions, these results as well as those of Seeberger and co-workers,⁷⁶ who encountered similar difficulties for the removal of an AZMB group on a tetrasaccharidic derivative, clearly showed that AZMB may not be always suitable as an orthogonal protecting group in the synthesis of complex oligosaccharides and thus, alternatives must be envisioned.

As a consequence, a different synthetic route was investigated involving: (i) cleavage of the C-2_{C} Ac group at a later stage of the synthesis, and (ii) utilization of Lev instead of AZMB at the C-2_{A} position on the tetrasaccharide backbone. As illustrated in Scheme 8, fully protected trisaccharide **40** was subjected to Staudinger reaction (PBu₃, THF then H₂O to afford acceptor **47** (88%), which was glycosylated with rhamnose donor **15** at low temperature (-10 °) in the presence of TMSOTf. It should be noted that, although tetrasaccharide **48** was obtained in good yield (76%, slightly contaminated with trichloroacetamide), two equivalents of donor were needed to achieve complete conversion. Subsequent delevulinoylation of compound **48** using hydrazine hydrate in Py/HOAc (3:2 gave tetrasaccharide acceptor **8** in good yield (85%). The latter was coupled with the **D'** donor **9** (1.5 equiv) under TMSOTf promotion at room temperature to produce the fully protected pentasaccharide **49** (75%) (Scheme 9). Importantly, non-acid washed powdered molecular sieves were needed in order to avoid the cleavage of acid-sensitive groups such as PMB, TBS and isopropylidene. At this stage, it was aimed to remove the Ac group at the C-2_{C} position of pentasaccharide **49** and replace it with a Lev group. Unfortunately, all deacetylation attempts failed to yield alcohol **50**. Indeed, acidic conditions had to be avoided because of the presence of the PMB and isopropylidene groups.Moreover, all basic and neutral conditions that were tried, such as K₂CO₃ (2.0 equiv),⁸⁴ Zemplen deacetylation (MeONa) or Bu₂SnO (2.0 equiv)⁶⁹ in refluxing MeOH, but all led preferentially to the cleavage of both the Ac and TBS groups (data not shown). This alternative synthetic route was thus abandoned.

The utilization of a chloroacetyl group (AcCl) at the C-2_{A} position of the **AB(E)C** tetrasaccharide, as a neighboring participating group that can be selectively removed in the presence of a Lev group, was next examined. To this end, it was necessary to synthesize the rhamnose donor **13** from the known precursor **19**. As depicted in Scheme 10, 2-chloroacetylation⁸³ of compound **19** allowed the quantitative formation of monosaccharide **51** (99%). Selective removal of the All group followed by activation of the anomeric position under above-mentionned conditions furnished the rhamnose donor **13** in excellent yield (89%, three steps). TMSOTF-mediated coupling of the latter with acceptor **43** at low temperature (-10 °C) gave tetrasaccharide **52** in a nearly quantitative yield (98%). However, as for all the tetrasaccharides reported in the present study, compound **52** was slightly contaminated with trichloroacetamide, and two equivalents of donor **13** were needed to achieve complete conversion. Finally, treatment of **52** with thiourea in a mixture of Py/MeOH afforded alcohol 7 in good yield (84%) as expected.

With tetrasaccharide 7 in hand, the construction of pentasaccharide donor **5** was straightforward (Scheme 11). Firstly, glycosylation of acceptor **7** with **D'** donor **9** (1.5 equiv) catalyzed by TMSOTf proceeded well at a cryogenic temperature (-78 °C) providing fully protected pentasaccharide **53** in excellent yield (89%). Thereafter, reductive free-radical dechlorination by using Bu₃SnH in conjunction with AIBN in refluxing toluene³¹ allowed the conversion of NHAcCl₃ into a NHAc group. However, it was quite difficult to purify the product by silica gel chromatography at this step because of the contamination with tin salts. Thus, selective removal of the All group was accomplished on the crude product to provide hemiacetal **54** (58%, three steps from **53**). The anomeric position of the latter was then activated with using N-(phenyl)trifluoroacetimidoyl chloride (PTFACl) in the presence of caesium carbonate (Cs₂CO₃) as a base in wet acetone⁸⁵ furnishing the pentasaccharide donor **5** in excellent yield (89%).

The elaboration of the **C-D** glycosidic linkage was the next mission. After optimization of the glycosylation conditions based on previous studies" using different promoters (TMSOTf, TBSOTf, BF₃.OEt₂, TfOH, Yb(OTf₃)), solvents (CH₂Cl₂, DCE, toluene) and temperatures (data not shown), it was found that the PTFA pentasaccharide donor **5** could be coupled efficiently with the acceptor **6** (1.5 equiv), thus providing access to the fully protected hexasaccharide **55** in excellent yield (84%) when the reaction was run in hot toluene (75 °C) in the presence of powdered molecular sieves under the promotion of *tert*-butyldimethylsilyl trifluoromethanesulfonate (TBSOTf) (Scheme 12). To avoid acidic degradation of donor **5**, Schmidt's inverse procedure⁷⁷ was particularly preferred, i.e. donor **5** is added last to the reaction mixture at elevated temperature. Noteworthy, in our hands the utilization of the more stable PTFA donor **5** resulted in better yields than that obtained with a similar TCA donor.¹¹ In order to obtain the hexasaccharide acceptor **56**, compound **55** was treated with tetrabutylammonium fluoride (TBAF) in THF. However, as revealed by ¹H NMR analysis, owing to the high basicity of fluoride ions both the TBS and Lev groups were removed . Alternative less basic desilylation conditions were thus investigated, namely HF-Py⁸⁶ and TBAF buffered with HOAc.^{53,87,88} Although the reaction of **55** with HF-Py did not lead to any deprotection product, desilylation using a mixture of TBAF/HOAc 2:1 in THF afforded hexasaccharide acceptor **56** in good yield (85%) along with unreacted **55** (7%) without any detectable trace of delevulinoylation product after five days of reaction.

The construction of the fully protected decasaccharide **59** required the beforehand preparation of the **A'B'(E')C'** tetrasaccharide donor (**4**). As shown in Scheme 13, the acceptor **43** was reacted with the known donor **14**⁶⁵ under the promotion of TMSOTf at low temperature (-10 °C) to provide tetrasaccharide **57** (92%). Conversion of the All group into hemiacetal **58** (92%, two steps) followed by activation of the anomeric position using PTFACl in the presence of Cs₂CO₃ afforded tetrasaccharide donor **4** in excellent yield (93%). Condensation of the latter with the acceptor **56** proceeded smoothly after three hours at low temperature (-10 °C) with TBSOTf as catalyst in the presence of powdered molecular sieves to give decasaccharide **59** in a satisfactory yield of 73% (Scheme 14).

Having synthesized the fully protected decasaccharide **59**, the last part of the work consisted in: (i) orthogonal deprotection of the PMB and/or isopropylidene groups, (ii) regioselective acetylation of the 3_{A} and/or 6_{D'} hydroxyl moieties, and (iii) final cleavage of all remaining protecting groups such as Lev and Bn to produce target mono- and diacetylated decasaccharides (**1-3**). Accordingly, compound **59** was subjected to PMB deprotection by the action of 2,3-dichloro-5,6-dicyanobenzoquinone (DDQ)^{89,90} in wet CH₂Cl₂ affording alcohol **60** (70%) along with unreacted **59** (13%). It is important to note that reaction times superior to 90 min. led to degradation products. Thereafter, the alcohol **60** was acetylated using excess of acetic anhydride (Ac₂O) in conjunction with DMAP, the isopropylidene group was removed by treatment with 50% aqueous trifluoroacetic acid (TFA)¹¹ and the three Lev groups were cleaved under standard conditions (hydrazine hydrate in Py/HOAc 3:2) leading to the formation of monoacetylated decasaccharide **61** in 79% yield (three steps from **60**). Acetylation of alcohol **60**, cleavage of the isopropylidene group, Yamamoto's regioselective acetylation ⁹¹⁻⁹³ of the 6_{D'} primary alcohol using an excess of acetyl chloride (AcCl) in *sym*-collidine followed by the deprotection of the Lev groups allowed the formation of diacetylated decasaccharide **62** in 74% yield (four steps from **60**). The regioselectivity of the acetylation reaction was confirmed from the downfield shift in the ¹H NMR signal of both the 6a_{D'} and 6b_{D'} protons. In another reaction, the fully protected decasaccharide **59** was treated with 50% aqueous TFA to furnish diol **63** in excellent yield (90%). It was found that, under these acidic conditions, the PMB group remained intact. The primary alcohol of compound **63** was regioselectivity acetylated (acetyl chloride, *sym*-collidine), then the Lev groups were removed affording monoacetylated decasaccharide **64** in 81 % yield (two steps from **63**). Finally, palladium-catalyzed hydrogenolysis (10% Pd/C, EtOH/HOAc 10:1)⁹⁴ of the remaining benzyl groups along with concomitant conversion of the azido group into the corresponding amine using H-Cube™ in full-H₂ mode⁹⁵ provided target decasaccharides **1** (58%), **2** (75%) and **3** (85%) in pure forms after HPLC purification and repeated freeze-drying.

### Conclusion

In summary, the preparation of three aminoethyl mono- and di-*O-*acetylated decasaccharides (**1-3**) representative of two repeating units of *S*. *flexneri* serotype 2a O-antigen was accomplished with success for the first time. A highly modular synthetic route has been developed in order to gain access to the three diversely O-acetylated oligosaccharide targets from the common fully protected intermediate **59**. The preparation of the latter was based on a blockwise strategy involving three conveniently protected synthons, namely the pentasaccharide donor **D'AB(E)C** (**5**), the tetrasaccharide donor **A'B'(E')C'** (**4**) and the acceptor **D** (**6**). L-Rhamnose monohydrate, D-glucosamine hydrochloride and *N*-D-acetylglucosamine were used as starting materials. The presence of an aminoethyl spacer at the reducing end of each acetylated decasaccharides will enable further site-selective conjugation at the reducing end of the new decasaccharides.

### Experimental

### General methods.

Chemical reagents were purchased from Sigma-Aldrich, Fluka, Alfa Aesar or TCI Europe and were used as received. The usual solvents were obtained from VWR International and were used as received. Air and water sensitive reactions were performed in dried glassware under argon atmosphere. Moisture sensitive reagents were introduced via a dry syringe. Anhydrous toluene (Tol), diethyl ether (Et₂O), dichloromethane (CH₂Cl₂), 1,2-dichloroethane (DCE), tetrahydrofuran (THF), dimethylformamide (DMF), acetonitrile (CH₃CN), ethanol (EtOH), methanol (MeOH) and pyridine (Py) were delivered on molecular sieves and were used as received. Additional solvents commonly cited in the text are abbreviated as Chex (cyclohexane) and CCl₄ (carbon tetrachloride). 4Å molecular sieves were activated before use by heating at 250 °C under vacuum. Analytical thin-layer chromatography was performed with silica gel 60 F₂₅₄, 0.25 mm pre-coated TLC plates (Merck). Compounds were visualized using UV₂₅₄ and/or orcinol (1 mg·mL⁻¹) in 10% aqueous H₂SO₄ with charring. Flash column chromatography was carried out using silica gel (Merck, particle size 0.040-0.063 mm). All of the chemical yields generally represent the highest result obtained for a minimum of three independent experiments. Nuclear magnetic resonance (NMR) spectra were recorded at 303 K on a Bruker Avance spectrometer at 400 MHz (¹H) and 100 MHz (¹³C) equipped with a BBO probe. Elucidations of chemical structures were based on ¹H, COSY, DEPT-135, HSQC, ¹³C, ¹³C gated decoupling and HMBC experiments. Signals are reported as m (multiplet), s (singlet), d (doublet), t (triplet), dd (doublet of doublet), dq (doublet of quadruplet), ddd (doublet of doublet of doublet), ddt (doublet of doublet of triplet), br s (broad singlet), br d (broad doublet) and coupling constants are reported in hertz (Hz). The chemical shifts are reported in ppm (δ) relative to residual solvent peak. Optical rotations were obtained using sodium D line at ambient temperature (25 °C) on a Bellingham + Stanley Ltd. ADP220 polarimeter. Of the two magnetically non-equivalent geminal protons at C-6, the one resonating at lower field is denoted H-6a, and the one at higher field is denoted H-6b. Interchangeable assignments are marked with an asterisk in the listing of signal assignments. Sugar residues are serially lettered according to the lettering of the repeating unit of the *S. flexneri* 2a O-Ag and identified by a subscript in the listing of signal assignments. High resolution electrospray ionisation/time-of-flight mass spectra (HR-ESI-TOF-MS) were recorded in the positive-ion mode with 1:1 CH₃CN/H₂O containing 0.1% formic acid as the ESI-TOF spectrometer solution. High resolution matrix-assisted laser desorption-ionisation/time-of-flight mass spectra (HR-MALDI-TOF-MS) were recorded in the positive-ion mode and were obtained at Institut de Chimie des Substances Naturelles, Gif sur Yvettes, France.

### Allyl 4-O-benzyl-α-L-rhamnopyranoside (16).

AcCl (98 mL, 1.4 mol, 2.5 equiv) was added dropwise to AllOH (1250 mL) at 0 °C. The solution was stirred for 1 h at this temperature, then L-rhamnose monohydrate (100 g, 548 mmol) was added. The reaction was stirred for 2 h at 70 °C, then overnight at 40 °C. The temperature was lowered to 0 °C, the mixture was neutralized by the addition of solid NaHCO₃, filtered over Celite^{®} and the solvents were evaporated under reduced pressure and co-evaporated with toluene. The resulting brown oily residue was dissolved in anhydrous acetone (604 mL), then 2,2-dimethoxypropane (203 mL, 1.65 mol, 3.0 equiv) and PTSA (5.22 g, 27.4 mmol, 0.05 equiv) were successively added. The mixture was stirred for 2 h at room temperature under an argon atmosphere. Then, the reaction was neutralized by the addition of Et₃N and the solvents were evaporated under reduced pressure. The residue was taken up in CH₂Cl₂ (1000 mL) and washed with H₂O (3×250 mL) and brine (1×250 mL). The organic layer was dried over anhydrous Na₂SO₄, filtered and concentrated to dryness. The resulting residue was dissolved in anhydrous DMF (1590 mL), the temperature was lowered to -5 °C (acetone/ice water bath) and NaH (60% oil dispersion, 55.3 g, 1.65 mol, 3.0 equiv) was carefully added. The mixture was stirred for 1 h at room temperature, then BnBr (130 mL, 1.10 mol, 2.0 equiv) was added dropwise at -5 °C. The reaction was stirred overnight at room temperature under an argon atmosphere. The temperature was lowered to 0 °C and MeOH was carefully added to 3 h,quench the reaction. Then, the solvents were concentrated under reduced pressure and the volatiles were co-evaporated with toluene. The residue was taken up in EtOAc (1000 mL) and washed with H₂O (3×250 mL) and brine (1×250 mL). The organic layer was dried over anhydrous Na₂SO₄, filtered and concentrated to dryness. The residue was dissolved in 80% aqueous HOAc (1370 mL) and the mixture was stirred for 3 h at 60 °C. The solvents were then evaporated under reduced pressure and the volatiles were co-evaporated to dryness with toluene to give a dark yellow solid residue. The crude product was recrystallized twice from diisopropyl ether/petroleum spirit 4:1 and the filtrate was further purified by flash chromatography (CH₂Cl₂/EtOAc 9:1 to 8:2) to afford diol **16** (112 g, 86%, 4 steps) as a white crystalline powder. ¹H and ¹³C NMR spectral data of **16**⁹⁶ were in agreement with those published in the literature.

### Allyl 3,4-di-O-benzyl-α-L-rhamnopyranoside (17).

To a solution of diol **16** (50.0 g, 170 mmol) in anhydrous toluene (680 mL) was added Bu₂SnO (46.5 g, 187 mmol, 1.10 equiv). The mixture was stirred for 3 h at reflux using a "Dean-Stark" apparatus. After cooling to room temperature, dry CsF (26.3 g, 173 mmol, 1.02 equiv), dry TBAI (75.2 g, 204 mmol, 1.20 equiv) and BnBr (24.2 mL, 204 mmol, 1.20 equiv) were successively added. The reaction was stirred overnight at 60 °C, then the temperature was lowered to 0 °C. The salts were removed by filtration, washed with toluene and the solvents of the filtrate were evaporated under reduced pressure. The residue was directly purified by flash chromatography (Chex/EtOAc 95:5 to 1:1) to furnish **17** (57.7 g, 88%, 2 steps) as a yellow oil. *R*_{f} 0.41 (Chex/EtOAc 7:3); [α]²⁵_{D} -37° (*c* 1.0, CHCl₃); ¹H and ¹³C NMR spectral data of **17**³⁸ were in agreement with those published in the literature.

### Allyl 4-O-benzyl-3-O-para-methoxybenzyl-α-L-rhamnopyranoside (19).

To a solution of diol **16** (13.2 g, 44.8 mmol) in anhydrous toluene (555 mL) was added Bu₂SnO (11.4 g, 45.6 mmol, 1.02 equiv). The mixture was stirred for 3 h at reflux using a "Dean-Stark" apparatus. After cooling to room temperature, dry CsF (6.92 g, 45.6 mmol, 1.02 equiv), dry TBAI (16.8 g, 45.6 mmol, 1.02 equiv) and PMBCl (6.7 mL, 49 mmol, 1.1 equiv) were successively added. The reaction was stirred for 2 h at reflux, then the temperature was lowered to 0 °C. The precipitate was removed by filtration and the solvents of the filtrate were evaporated under reduced pressure. The residue was directly purified by flash chromatography (Chex/EtOAc 9:1 to 7:3) to furnish **19** (15.2 g, 82%, 2 steps) as a yellow oil. *R*_{f} 0.31 (Chex/EtOAc 7:3); [α]²⁵_{D} -63° (*c* 1.0, CHCl₃); ¹H NMR (CDCl₃, 400 MHz) δ: 7.38-7.27 (m, 7H, C*H*_{Ph}), 6.90-6.86 (m, 2H, C*H*_{Ph}), 5.92 (high-order m, 1H, H-2_{All}), 5.30 (ddd, *J* = 17.2, 3.3, 1.5 Hz, 1H,H-3a_{All}),5.21 (ddd, *J* = 10.3, 5.2, 1.3 Hz, 1H, H-3b_{All}), 4.92 (d, *J* = 11.1 Hz, 1H, C*H*₂Ph), 4.87 (d, *J*_{1,2} = 1.4 Hz, 1H, H-1), 4.67-4.61 (m, 3H, C*H*₂Ph), 4.17 (ddt, *J* = 13.0, 5.1, 1.4 Hz, 1H, H-1a_{All}), 4.05 (dd, *J*_{2,3} = 4.5 Hz, *J*_{1,2} = 2.8 Hz, 1H, H-2), 3.97 (ddt, *J* = 13.0, 6.0, 1.3 Hz, 1H, H-1b_{All}), 3.89 (dd, *J*_{3,4} = 9.2 Hz, *J*_{2,3} = 3.2 Hz, 1H, H-3), 3.80 (high-order m, 1H, H-5), 3.74 (s, 3H, OC*H*₃), 3.54 (t, *J*_{4,5} = *J*_{3,4} = 9.3 Hz, 1H, H-4), 3.19 (d, *J*_{O*H,*2} = 2.5 Hz, 1H, O*H*), 1.37 (d, *J*_{5,6} = 6.2 Hz, 3H, H-6). ¹³C NMR (CDCl₃, 100 MHz) δ: 159.1 (C_{Ph}), 138.4 (C-2_{All}), 133.8 (C_{Ph}), 129.9-127.3 (*C*H_{Ph}), 116.8 (C-3_{All}), 113.6 (*C*H_{Ph}), 113.5 (*C*Hpₕ), 79.8 (C-4), 79.5 (C-3), 74.9 (*C*H₂Ph), 71.3 (*C*H₂Ph), 68.2 (C-2), 67.5 (C-1_{All}), 67.3 (C-5), 54.8 (O*C*H₃), 17.7 (C-6). HR-ESI-TOF-MS *m*/*z* 437.1929 [M + Na]⁺ (calcd for C₂₄H₃₀O₆Na, 437.1940).

### 2-(Azidomethyl)benzoic acid (21).

To a solution of methyl 2-methylbenzoate (**18**, 85.0 g, 566 mmol) in anhydrous CCl₄ (1.00 L) was added NBS (102 g, 572 mmol, 1.01 equiv) and benzoyl peroxide (1.37 g, 6.66 mmol, 0.01 equiv). CAUTION! This reaction is very exothermic, thus care must be taken in order to minimize the risks of runaway reaction. The mixture was stirred for 24 h at reflux under an argon atmosphere. Then, the temperature was lowered to 0 °C and the precipitate was removed by filtration. The solvents were evaporated under reduced pressure to dryness to afford a residue, which was dissolved in anhydrous EtOH (1.59 L). NaN₃ (37.2 g, 572 mmol, 1.01 equiv) was added and the mixture was stirred for 3 h at reflux under an argon atmosphere. The reaction was then quenched with brine (250 mL) and the mixture was concentrated (to 1/3 volume) under reduced pressure. The aqueous phase was extracted with CH₂Cl₂ (3×500 mL), the organic layer was dried over anhydrous Na₂SO₄, filtered, and concentrated to dryness. The resulting residue was dissolved in MeOH/H₂O (1.19 L, 5:2 v/v) and NaOH (51.0 g) was added. The mixture was stirred for 2 h at room temperature, then concentrated (to 1/3 volume) under reduced pressure. The aqueous phase was extracted with CH₂Cl₂ (3×500 mL) and acidified with 6 N aqueous HCl. The precipitated acid was then extracted with CH₂Cl₂ (3×250 mL), the organic phases were pooled and concentrated to dryness to afford a yellow solid residue. The crude product was recrystallized from Chex to furnish **21** (77.3 g, 77%, 3 steps) as a white crystalline powder. Physical and analytical data of **21**⁴¹ were in agreement with those published in the literature.

### 2-(Azidomethyl)benzoyl chloride (22).

AZMBOH (**21**, 7.00 g, 41.2 mmol) was dissolved in SOCI₂ (6.0 mL, 82 mmol, 2.0 equiv) and the mixture was stirred at reflux for 1 h under an argon atmosphere. The volatiles were then evaporated under reduced pressure and co-evaporated with toluene to dryness to afford crude AZMBC1 (**22**) as a yellow oil, which was used directly for the next step without further purification. Physical and analytical data of **22**⁴¹ were in agreement with those published in the literature.

### Allyl 2-O-(azidomethyl)benzoyl-3,4-di-O-benzyl-α-L-rhamnopyranoside (23).

**Route a.** To a solution of **18** (5.00 g, 13.0 mmol) in anhydrous CH₂Cl₂ (130 mL) was added DMAP (3.18 g, 26.0 mmol, 2.0 equiv) and AZMBC1 (**22**, 3.02 g, 26.0 mmol, 2.0 equiv). The mixture was stirred for 1 h at room temperature under an argon atmosphere. The organic phase was washed with saturated aqueous NaHCO₃ (2×50 mL) and brine (1×50 mL). Then, the solution was dried over anhydrous Na₂SO₄, filtered and the solvents were removed under reduced pressure. The residue was purified by flash chromatography (Chex/EtOAc 95:5 to 9:1) to give **23** (6.78 g, 94%) as a colorless oil.

**Route b.** To a solution of **18** (34.2 g, 88.9 mmol) in anhydrous CH₂Cl₂ (910 mL) was added DMAP (10.9 g, 88.8 mmol, 1.0 equiv), DCC (36.7 g, 178 mmol, 2.0 equiv) and AZMBOH (**21**, 23.6 g, 133 mmol, 1.5 equiv). The mixture was stirred for **4** h at reflux under an argon atmosphere, then the temperature was lowered to room temperature. The mixture was filtered over Celite^{®}, washed with several portions of CH₂Cl₂ and the solvents were removed under reduced pressure. The residue was purified by flash chromatography (Chex/EtOAc 95:5 to 7:3) to afford **23** (43.1 g, 89%) as a light yellow oil. *R*_{f} 0.84 (Chex/EtOAc 7:3); [α]²⁵_{D}+16° (*c* 1.0, CHCl₃); ¹H NMR (CDCl₃, 400 MHz) δ: 8.14-8.11 (m, 1H, C*H*_{Ph}), 7.64-7.29 (m, 13H, C*H*_{Ph}), 5.98 (high-order m, 1H, H-2_{All}), 5.71 (dd, *J*_{2,3} = 3.2 Hz, *J*_{1,2} = 1.8 Hz, 1H, H-2) 5.38 (*ddd, J* = 17.2, 3.1,1.5 Hz, 1H, H-3a_{All}), 5.29 (*ddd, J* = 10.4, 2.5, 1.3 Hz, 1H, H-3b_{All}), 5.02 (d, *J* = 10.9 Hz, 1H, C*H*₂Ph), 5.02 (d, *J*_{1,2} = 1.8 Hz, 1H, H-1), 4.89-4.82 (m, 3H, C*H*₂Ph, C*H*₂N₃), 4.74 (d, *J* = 10.9 Hz, 1H, C*H*₂Ph), 4.69 (d, *J* = 11.3 Hz, 1H, C*H*₂Ph), 4.26 (ddt, *J* = 12.9, 5.3, 1.4 Hz, 1H, H-1a_{All}), 4.18 (dd, *J*_{3,4} = 9.4 Hz, *J*_{2,3} = 3.3 Hz, 1H, H-3), 4.09 (ddt, *J* = 12.9, 6.0, 1.3 Hz, 1H, H-1 b_{All}), 3.94 (high-order m, 1H, H-5), 3.64 (t, *J*_{3,4} = *J*_{4,5} = 9.4 Hz, 1H, H-4), 1.45 (d, *J*_{5,6} = 6.1 Hz, 3H, H-6). ¹³C NMR (CDCl₃, 100 MHz) δ: 166.0 (*C*=O), 138.5 (PPh), 138.1 (C_{Ph}), 137.3 (C_{Ph}) 133.6 (C-2_{All}), 132.8-127.7 (*C*H_{Ph}), 117.7 (C-3_{All}), 96.8 (C-1), 80.2 (C-4), 78.2 (C-3), 75.5 (*C*H₂Ph), 71.8 (*C*H₂Ph), 70.0 (C-2), 68.2 (C-1_{All}), 67.9 (C-5), 53.0 (*C*H₂N₃), 18.2 (C-6). HR-ESI-TOF-MS *m*/*z* 566.2254 [M + Na]⁺ (calcd for C₃₁H₃₃N₃O₆Nₐ, 566.2267).

### Allyl 2-O-(azidomethyl)benzoyl-4-O-benzyl-3-O-para-methoxybenzyl-α-L-rhamnopyranoside (24).

**Route a.** To a solution of **19** (5.00 g, 12.1 mmol) in anhydrous CH₂Cl₂ (121 mL) was added DMAP (2.95 g, 24.1 mmol, 2.0 equiv) and AZMBC1 (**22**, 2.80 g, 24.1 mmol, 2.0 equiv). The mixture was stirred for 1 h at room temperature under an argon atmosphere. The organic phase was washed with saturated aqueous NaHCO₃ (2×50 mL) and brine (1×50 mL). Then, the solution was dried over anhydrous Na₂SO₄, filtered and the solvents were removed under reduced pressure. The residue was purified by flash chromatography (Chex/EtOAc 95:5 to 8:2) to give **24** (6.26 g, 91 %) as a colorless oil.

**Route b.** To a solution of **19** (14.0 g, 33.8 mmol) in anhydrous CH₂Cl₂ (338 mL) was added DMAP (4.13 g, 33.8 mmol, 1.0 equiv), DCC (13.9 g, 67.6 mmol, 2.0 equiv) and AZMBOH (**21**, 8.98 g, 50.7 mmol, 1.5 equiv). The mixture was stirred for 4 h at reflux under an argon atmosphere, then the temperature was lowered to room temperature. The mixture was filtered under Celite^{®}, washed with several portions of CH₂Cl₂ and the solvents were removed under reduced pressure. The residue was purified by flash chromatography (Chex/EtOAc 95:5 to 7:3) to afford **24** (18.6 g, 96%) as a light yellow oil. *R*_{f} 0.79 (Chex/EtOAc 7:3); [α]²⁵_{D} +32° (*c* 1.0, CHCl₃); ¹H NMR (CDCl₃, 400 MHz) δ: 8.05-8.02 (m, 1H, C*H*_{Ph}), 7.60-7.56 (m, 1H, C*H*_{Ph}), 7.53-7.50 (m, 1H, C*H*_{Ph}), 7.45-7.40 (m, 1H, C*H*_{Ph}), 7.36-7.22 (m, 7H, C*H*_{Ph}), 6.83-6.78 (m, 2H, C*H*_{Ph}), 5.91 (high-order m, 1H, H-2_{All}), 5.60 (dd, *J*_{2,3} = 3.3 Hz, *J*_{1,2} = 1.8 Hz, 1H, H-2), 5.30 (ddd, *J* = 17.2, 3.2, 1.6 Hz, 1H, H-3a_{All}), 5.22 (ddd, *J* = 10.4, 2.8, 1.3 Hz, 1H, H-3b_{All}), 4.92 (d, *J* = 10.9 Hz, 1H, C*H*₂Ph), 4.92 (d, *J*_{1,2} = 1.7 Hz, 1H, H-1), 4.78 (br s, 2H, CH₂N₃)**,** 4.71 (d, *J* = 10.9 Hz, 1H, C*H*₂Ph), 4.64 (d, *J* = 11.0 Hz, 1H, C*H*₂Ph), 4.54 (d, *J* = 10.9 Hz, 1H, C*H*₂Ph), 4.19 (ddt, *J* = 12.8, 5.1, 1.5 Hz, 1H, H-1a_{All}), 4.07 (dd, *J*_{3,4} = 9.3 Hz, *J*_{2,3} = 3.4 Hz, 1H, H-3), 4.01 (ddt, *J* = 12.8, 6.1, 1.3 Hz, 1H, H-1b_{All}), 3.84 (high-order m, 1H, H-5), 3.77 (s, 3H, OC*H*₃), 3.52 (t, *J_{3,4}* = *J_{4,5}* = 9.4 Hz, 1H, H-4), 1.35 (d, *J*_{5,6} = 6.3 Hz, 3H, H-6). ¹³C NMR (CDCl₃, 100 MHz) δ: 166.1 (*C*=O), 159.4 (C_{Ph}), 138.6 (C_{Ph}), 137.4 (C_{Ph}), 133.7 (C-2_{All}), 132.9-127.8 (*C*H_{Ph}), 117.8 (C-3_{All}), 113.9 (*C*H_{Ph}), 96.9 (C-1), 80.3 (C-4), 78.0 (C-3), 75.5 (*C*H₂Ph), 71.6 (*C*H₂Ph), 70.1 (C-2), 68.3 (C-1_{All}), 67.9 (C-5), 55.4 (OCH₃), 53.1 (*C*H₂N₃), 18.2 (C-6). HR-ESI-TOF-MS *m*/*z* 596.2415 [M + Na]⁺ (calcd for C₃₂H₃₅N₃O₇Na, 596.2372).

### 2-O-2-(Azidomethyl)benzoyl-3,4-di-O-benzyl-α-L-rhamnopyranosyl trichloroacetimidate (11).

1,5-Cyclooctadiene-bis(methyldiphenylphosphine)-iridium hexafluorophosphate (**467** mg, 0.55 mmol, 0.05 equiv) was dissolved in anhydrous THF (81 mL) and the resulting red solution was degassed under an argon atmosphere. Hydrogen was bubbled through the solution for 5 min, then the resulting yellow solution was once again degassed under an argon atmosphere. A solution of **23** (6.00 g, 11.0 mmol) in anhydrous THF (110 mL) was then added. The mixture was stirred for 2 h at room temperature under an argon atmosphere. A solution of iodine (5.60 g, 22.1 mmol, 2.0 equiv) in THF/H₂O (72 mL, 4:1 v/v) was added to the mixture, which was stirred for 1 h at room temperature. The reaction was then quenched by the addition of a freshly prepared solution of 10% aqueous sodium bisulfite (75 mL). The mixture was concentrated (to 1/3 volume) under reduced pressure and the aqueous phase was extracted three times with CH₂Cl₂ (3×150 mL). The organic layer was washed with H₂O (2×100 mL), brine (1×100 mL), dried over anhydrous Na₂SO₄, filtered and evaporated to dryness. The resulting oily residue was dissolved in anhydrous DCE (99 mL), then CCl₃CN (5.5 mL, 55 mmol, 5.0 equiv) and DBU (0.46 mL, 3.1 mmol, 0.28 equiv) were successively added. The mixture was stirred for 1h at room temperature under an argon atmosphere, then the solvents were evaporated under reduced pressure. The crude product was purified by flash chromatography (Chex/EtOAc 95:5 to 8:2 + 0.5% Et₃N) to afford **11** (4.84 g, 68%, 3 steps) as a yellow oil. *R*_{f} 0.81 (Chex/EtOAc 7:3); [CC]²¹_{D} -10° (*c* 1.0, CHCl₃); ¹H NMR (CDCl₃, 400 MHz) δ: 8.70 (s, 1H, N*H*), 8.09-8.06 (m, 1H, C*H*_{Ph}), 7.64-7.59 (m, 1H, C*H*_{Ph}), 7.56-7.53 (m, 1H, C*H*_{Ph}), 7.49-7.44 (m, 1H, C*H*_{Ph}), 7.37-7.25 (m, 10H, C*H*_{Ph}), 6.33 (d, *J*_{1,2} = 2.0 Hz, 1H, H-1), 5.71 (d, *J*_{2,3} = 3.2 Hz, *J*_{1,2} = 2.1 Hz, 1H, H-2), 4.95 (d, *J* = 10.9 Hz, 1H, C*H*₂Ph), 4.80 (d, *J* = 11.4 Hz, 1H, C*H*₂Ph), 4.78 (br s, 2H, C*H*₂N₃), 4.68 (d, *J* = 10.9 Hz, 1H, C*H*₂Ph), 4.66 (d, *J* = 11.4 Hz, 1H, C*H*₂Ph), 4.12 (dd, *J*_{3,4} = 9.4 Hz, *J*_{2,3} = 3.2 Hz, 1H, H-3), 4.02 (high-order m, 1H, H-5), 3.64 (t, *J*_{3,4} = *J*_{4,5} = 9.6 Hz, 1H, H-4), 1.38 (d, *J*_{5,6} = 5.7 Hz, 3H, H-6). ¹³C NMR (CDCl₃, 100 MHz) δ: 165.7 (*C*=O), 160.3 (*C*=NH), 138.2 (C_{Ph}), 137.7 (2×C_{Ph}), 133.3-128.1 (*C*H_{Ph}), 95.3 (C-1), 91.0 (*C*Cl₃), 79.6 (C-4), 77.4 (C-3), 75.8 (*C*H₂Ph), 72.2 (*C*H₂Ph), 70.9 (C-5), 68.7 (C-2), 53.1 (*C*H₂N₃),18.3 (C-6).

### 2-O-2-(Azidomethyl)benzoyl-4-O-benzyl-3-O-para-methoxylbenzyl-α-L-rhamnopyranosyl trichloroacetimidate (12).

1,5-Cyclooctadiene-bis(methyldiphenylphosphine)-iridium hexafluorophosphate (663 mg, 0.784 mmol, 0.03 equiv) was dissolved in anhydrous THF (131 mL) and the resulting red solution was degassed under an argon atmosphere. Hydrogen was bubbled through the solution for 5 min, then the resulting yellow solution was once again degassed under an argon atmosphere. A solution of **24** (15.0 g, 26.2 mmol) in anhydrous THF (131 mL) was then added. The mixture was stirred for 2 h at room temperature under an argon atmosphere. A solution of iodine (13.3 g, 52.3 mmol, 2.0 equiv) in THF/H₂O (157 mL, 4:1 v/v) was added to the mixture, which was stirred for 3 h at room temperature. The reaction was then quenched by the addition of a freshly prepared solution of 10% aqueous sodium bisulfite (100 mL). The mixture was concentrated (to 1/3 volume) under reduced pressure and the aqueous phase was extracted three times with CH₂Cl₂ (3×250 mL). The organic layer was washed with H₂O (1×00 mL), brine (1×250 mL), dried over anhydrous Na₂SO₄, filtered and evaporated to dryness. The resulting oily residue was dissolved in anhydrous DCE (114 mL), then CCl₃CN (13.1 mL, 131 mmol, 5.0 equiv) and DBU (1.1 mL, 7.3 mmol, 0.28 equiv) were successively added. The mixture was stirred for 1 h at room temperature under an argon atmosphere, then the solvents were evaporated under reduced pressure. The crude product was purified by flash chromatography (isocratic CH₂Cl₂ + 0.5% Et₃N) to afford **12** (10.7 g, 60%, 3 steps) as a yellow oil. *R*_{f} 0.76 (Chex/EtOAc 7:3); [α]²⁵_{D} -3° (*c* 1.0, C*H*Cl₃); ¹H NMR (CDCl₃, 400 MHz) δ: 8.71 (s, 1H, N*H*), 8.09-8.06 (m, 1H, C*H*_{Ph}), 7.65-7.61 (m, 1H, C*H*_{Ph}), 7.58-7.55 (m, 1H, C*H*_{Ph}), 7.50-7.45 (m, 1H, C*H*_{Ph}), 7.39-7.25 (m, 7H, C*H*_{Ph}), 6.85-6.81 (m, 2H, C*H*_{Ph}), 6.33 (d, *J*_{1,2} = 1.9 Hz, 1H, H-1), 5.70 (dd, *J*_{2,3} = 3.3 Hz, *J*_{1,2} = 2.0 Hz, 1H, H-2), 4.95 (d, *J* = 10.8 Hz, 1H, C*H*₂Ph), 4.81 (br s, 2H, C*H*₂N₃), 4.74 (d, *J* = 11.0 Hz, 1H, C*H*₂Ph), 4.68 (d, *J* = 10.8 Hz, 1H, C*H*₂Ph), 4.61 (d, *J* = 11.0 Hz, 1H, C*H*₂Ph), 4.11 (dd, *J*_{3,4} = 9.5 Hz, *J*_{2,3} = 3.3 Hz, 1H, H-3), 4.01 (high-order m, 1H, H-5), 3.80 (s, 3H, OC*H*₃), 3.63 (t, *J*_{3,4} = *J*_{4,5} = 9.6 Hz, 1H, H-4), 1.39 (d, *J*_{5,6} = 6.0 Hz, 3H, H-6). ¹³C NMR (CDCl₃, 100 MHz) δ: 165.7 (*C*=O), 160.3 (*C*=NH), 159.6 (PPh), 138.3 (C_{Ph}), 137.7 (PPh), 133.2-128.0 (*C*H_{Ph}), 114.0 (C_{Ph}), 95.3 (C-1), 91.0 (*C*Cl₃), 79.5 (C-4), 77.0 (C-3), 75.8 (CH₂Ph), 71.8 (CH₂Ph), 70.9 (C-5), 68.7 (C-2), 55.4 (OCH₃), 53.2 (CH₂N₃), 18.3 (C-6).

### Allyl 4-O-benzyl-2-O-chloroacetyl-3-O-para-methoxybenzyl-α-L-rhamnopyranoside (51).

To a cold (ice/water bath) solution of **19** (250 mg, 0.603 mmol) in anhydrous CH₂Cl₂/Py (5.4 mL, 8:1 v/v) was carefully added chloroacetyl chloride (192 µL, 2.41 mmol, 4.0 equiv). The mixture was stirred for 3 h at room temperature under an argon atmosphere, then diluted with CH₂Cl₂ (100 mL). The mixture was washed with 10% aqueous HCl (1 × 50 mL), saturated NaHCO₃ (1 x50 mL) and brine (1×50 mL). The solvents of the dried (anhydrous Na₂SO₄) were removed under reduced pressure and the resulting residue was purified on a short pad of silica gel (100% CH₂Cl₂, then Chex/EtOAc 7:3) to furnish **51** (296 mg, 100%) as a yellow oil. *R_{f}* 0.75 (Chex/EtOAc 7:3); [α]²⁵_{D} -22° (*c* 1.0, CHCl₃); ¹H NMR (CDCl₃, 400 MHz) δ: 7.37-7.23 (m, 7H, C*H*_{Ph}), 6.87-6.83 (m, 2H, C*H*_{Ph}), 5.88 (high-order m, 1H, H-2_{All}), 5.44 (dd, *J*_{2,3} = 3.3 Hz, *J*_{1,2} *=* 1.8 Hz, 1H, H-2), 5.28 (ddd, *J*= 17.2, 3.2, 1.5 Hz, 1H, H-3a_{All}), 5.21 (ddd, *J* = 10.3, 2.6, 1.3 Hz, 1H, H-3a_{All}), 4.89 (d, *J* = 10.8 Hz, 1H, C*H*₂Ph), 4.80 (d, *J*_{1,2} = 1.7 Hz, 1H, H-1), 4.64 (d, *J* = 10.8 Hz, 1H, C*H*₂Ph), 4.60 (d, *J* = 10.8 Hz, 1H, C*H*₂Ph), 4.47 (d, *J* = 10.7 Hz, 1H, C*H*₂Ph), 4.16 (ddt, *J* = 12.7, 5.3, 1.5 Hz, 1H, H-1a_{All}), 4.16 (s, 2H, C*H*₂Cl), 4.01-3.95 (m, 2H, H-1b_{All}, H-3), 3.82-3.75 (high-order m, 1H, H-5), 3.80 (s, 3H, OC*H*₃), 3.39 (t, *J*_{3,4} = *J*_{4,5} *=* 9.6 Hz, 1H, H-4), 1.32 (d, *J*_{5,6} = 6.1 Hz, 3H, H-6). ¹³C NMR (CDCl₃, 100 MHz) δ: 167.0 (C=O), 159.5 (C_{Ph}), 138.5 (C_{Ph}), 133.5 (C-2_{All}), 129.9-127.8 (*C*H_{Ph}), 117.9 (C-3_{All}), 114.0 (*C*H_{Ph}), 96.5 (C-1), 80.0 (C-4), 77.8 (C-3), 75.5 (*C*H₂Ph), 71.8 (*C*H₂Ph), 71.1 (C-2), 68.2 (C-1Aₗₗ), 67.9 (C-5), 55.4 (OCH₃), 41.1 (CH₂Cl), 18.0 (C-6). HR-ESI-TOF-MS *m*/*z* 513.1653 [M + Na]⁺ (calcd for C₂₆H₃₁ClO₇Na, 513.1656).

### 4-O-Benzyl-2-O-chloroacetyl-3-O-para-methoxybenzyl-α-L-rhamnopyranosyl trichloroacetimidate (13).

1,5-Cyclooctadiene-bis(methyldiphenylphosphine)-iridium hexafluorophosphate (122 mg, 0.14 mmol, 0.05 equiv) was dissolved in anhydrous THF (14 mL) and the resulting red solution was degassed under an argon atmosphere. Hydrogen was bubbled through the solution for 15 min, then the resulting yellow solution was once again degassed under an argon atmosphere. A solution of 51 (1.42 g, 2.89 mmol) in anhydrous THF (14 mL) was then added. The mixture was stirred overnight at room temperature under an argon atmosphere. A solution of iodine (1.47 g, 5.78 mmol, 2.0 equiv) in THF/H₂O (6.0 mL, 4:1 v/v) was added to the mixture, which was stirred for 1 h at room temperature. The reaction was then quenched by the addition of a freshly prepared solution of 10% aqueous sodium bisulfite. The mixture was concentrated (to 1/3 volume) under reduced pressure and the aqueous phase was extracted three times with CH₂Cl₂. The organic layer was washed with H₂O (2×), brine (1×), dried over anhydrous Na₂SO₄, filtered and evaporated to dryness. The resulting oily residue was dissolved in anhydrous DCE (5.8 mL), then CCl₃CN (1.5 mL, 14 mmol, 5.0 equiv) and DBU (130 µL, 0.87 mmol, 0.30 equiv) were successively added. The mixture was stirred for 1 h at room temperature under an argon atmosphere, then the mixture was purified by flash chromatography (Chex/EtOAc 9:1 to 8:2 + 1% Et₃N) to afford 13 (1.53 g, 89%, 3 steps) as a yellow oil. *R*_{f} 0.73 (Chex/EtOAc 7:3); [×]²⁵_{D} -31° (c 1.0, CHCl₃); ¹H NMR (CDCl₃, 400 MHz) δ (α): 8.68 (s, 1H, N*H*), 7.38-7.22 (m, 7H, C*H*_{PH}), 6.87-6.82 (m, 2H, C*H*_{Ph}), 6.20 (br s, 1H, H-1), 5.51 (br s, 1H, H-2), 4.91 (d, *J* = 10.8 Hz, 1H, C*H*₂Ph), 4.66 (d, *J* = 11.9 Hz, 1H, C*H*₂Ph), 4.63 (d, *J* = 11.8 Hz, 1H, C*H*₂Ph), 4.52 (d, *J* = 10.9 Hz, 1H, C*H*₂Ph), 4.19 (s, 2H, C*H*₂Cl), 4.02-3.91 (m, 2H, H-3, H-5), 3.80 (s, 3H, OC*H*₃), 3.48 (t, *J*_{3,4} = *J*_{4,5} = 9.6 Hz, 1H, H-4), 1.34 (d, *J*_{5,6} = 6.1 Hz, 3H, H-6). ¹³C NMR (CDCl₃, 100 MHz) δ (α): 166.7 (*C*=O), 160.1 (*C*=NH), 159.6 (C_{Ph}), 138.1 (C_{Ph}), 130.2-128.1 (CH_{Ph}), 114.0 (CH_{Ph}), 94.8 (C-1), 90.9 (CCl₃), 79.1 (C-4), 76.7 (C-3), 75.8 (CH₂Ph), 72.0 (CH₂Ph), 70.9 (C-5), 69.6 (C-2), 55.4 (OCH₃), 40.9 (CH₂Cl), 18.0 (C-6).

### Allyl 2-deoxy-2-trichloroacetamido-α-D-glucopyranoside (26).

Glucosamine hydrochloride (10.0 g, 46.4 mmol) was dissolved in anhydrous MeOH (199 mL) and the mixture was stirred for 1 h at room temperature under an argon atmosphere. The temperature was lowered to 0 °C, then a solution of 25% NaOMe in MeOH (32 mL) was added. After 15 min, trichloroacetic anhydride (12.7 mL, 69.6 mmol, 1.5 equiv) was added dropwise and the mixture was stirred for 2 h at 0 °C. The reaction was neutralized with DOWEX H⁺ form resin, filtered and the solvents were evaporated under reduced pressure. The resulting residue was dissolved in a solution of AllOH (3.1 mL) in which AcCl (0.37 mL, 5.2 mmol, 3.4 equiv) was slowly added at 0 °C. The mixture was stirred for 3 h at 70 °C, then diluted with MeOH and solid NaHCO₃ was added until neutrality was reached. After filtration, the solvents were evaporated under reduced pressure and the residue was purified by flash chromatography (CH₂Cl₂/MeOH 95:5 to 9:1) to afford **26** (7.53 g, 45%, 2 steps) as an amorphous brownish solid. *R_{f}* 0.16 (CH₂Cl₂/MeOH 95:5); [α]²⁵_{D} +113° (c 1.0, MeOH); ¹H NMR (CDCl₃, 400 MHz) δ: 7.14 (d, *J_{NH,2}* = 8.8 Hz, 1H, *NH),* 5.85 (high-order m, 1H, H-2_{All}), 5.29 (ddd, 1H, *J*= 17.1, 3.0, 1.5 Hz, 1H, H-3a_{All}), 5.21 (ddd, 1H, *J =* 10.4, 2.5, 1.1 Hz, 1H, H-3b_{All}), 4.95 (d, *J*_{1,2} = 3.6 Hz, 1H, H-1), 4.19 (ddt, *J =* 13.0, 5.2, 1.4 Hz, 1H, H-1a_{All}), 4.08 (td, *J_{2,3} = J_{NH,2} =* 9.5 Hz, *J*_{1,2} = 3.6 Hz, 1H, H-2), 4.00 (ddt, *J* = 13.0, 6.3, 1.2 Hz, 1H, H-1b_{All}), 3.94 (dd, *J*_{6a,6b} = 12.5 Hz, *J*_{5,6a} = 2.3 Hz, 1H, H-6a), 3.88-3.81 (m, 2H, H-6b, H-3), 3.77 (t, *J_{3,4} = J_{4,5} =* 9.3 Hz, 1H, H-4), 3.64 (dt, *J_{4,5}* = 9.6 Hz, *J*_{5,6a} = *J*_{5,6b} = 2.5 Hz, 1H, H-5). ¹³C NMR (CDCl₃, 100 MHz) δ: 162.9 (*C*=O), 133.3 (C-2_{All}), 118.5 (C-3_{All}), 96.1 (C-1), 92.6 (*C*Cl₃), 72.8 (C-3), 72.0 (C-5), 70.1 (C-4), 68.7 (C-1_{All}), 61.3 (C-6), 55.5 (C-2). HR-ESI-TOF-MS *m*/*z* 364.0120 [M + H]⁺ (calcd for C₁₂H₁₇ChNO₆, 364.0121).

### Allyl 2-deoxy-4,6-O-isopropylidene-2-trichloroacetamido-α-D-glucopyranoside (27).

To a solution of **26** (8.07 g, 22.1 mmol) in anhydrous DMF (75 mL) was added 2-methoxypropene (4.2 mL, 44.4 mmol, 2.0 equiv) followed by PTSA (422 mg, 2.22 mmol, 0.1 equiv). The mixture was stirred for 1 h at room temperature under an argon atmosphere. Et₃N (3.0 mL) was then added to quench the reaction and the solvents were evaporated under reduced pressure. The resulting residue was purified by flash chromatography (Chex/EtOAc 8:2 to 7:3) to give **27** (8.09 g, 90%) as a white amorphous powder. *R_{f}* 0.36 (Chex/EtOAc 7:3); [α]²⁵D +95° (*c* 1.0, CHCl₃); ¹H NMR (Pyr-d₅, 400 MHz) δ: 10.35 (d, *J*_{NH,2} = 8.1 Hz, 1H, N*H*), 7.69 (d, *J*_{OH,3} = 5.3 Hz, 1H, O*H*), 6.01 (high-order m, 1H, H-2_{All}), 5.36 (ddd, *J* = 17.2, 3.2, 1.6 Hz, 1H, H-3a_{All}), 5.36 (d, *J_{1,2}* = 3.8 Hz, 1H, H-1), 5.18 (ddd, *J* = 10.3, 2.8, 1.3 Hz, 1H, H-3b_{All}), 4.72 (ddd, *J*_{2,3} = 10.2 Hz, *J*_{2,NH} *=* 8.3 Hz, *J*_{1,2} = 3.6 Hz, 1H, H-2), 4.50 (high-order m, 1H, H-3), 4.28 (ddt, *J =* 13.0, 5.3, 1.4 Hz, 1H, H-1a_{All}), 4.09 (ddt, *J* = 13.0, 6.2, 1.3 Hz, 1H, H-1b_{All}), 4.04-3.90 (m, 4H, H-4, H-5, H-6a, H-6b), 1.49 (s, 3H, C(CH₃)₂), 1.44 (s, 3H, C(*C*H₃)₂). ¹³C NMR (Pyr-d₅, 100 MHz) δ: 163.6 (*C*=O), 134.9 (C-2_{All}), 118.2 (C-3_{All}), 100.2 (*C*(CH₃)₂), 97.8 (C-1), 94.5 (*C*Cl₃), 76.6 (C-4), 69.3 (C-1_{All}), 68.8 (C-3), 65.0 (C-5), 63.0 (C-6), 58.1 (C-2), 29.7 (C(CH₃)₂), 19.6 (C(CH₃)₂). HR-ESI-TOF-MS *m*/*z* 404.0441 [M + H]⁺ (calcd for C₁₅H₂₁Cl₃NO, 404.0435).

### Allyl 3-O-tert-butyldimethylsilyl-2-deoxy-4,6-O-isopropylidene-2-trichloroacetamido-α-D-glucopyranoside (28).

To a solution of **27** (7.98 g, 19.7 mmol) in anhydrous THF (59 mL) was added DMAP (241 mg, 2.0 mmol, 0.1 equiv), imidazole (3.36 g, 49.3 mmol, 2.5 equiv) and TBSCl (8.92 g, 59.2 mmol, 3.0 equiv). The mixture was stirred for 2 d at reflux under an argon atmosphere. After cooling to room temperature, the reaction was filtered and the filtrate was evaporated to dryness. The resulting residue was purified by flash chromatography (100% Chex to Chex/EtOAc 9:1) to give **28** (10.0 g, 98%) as a white amorphous powder. *R_{f}* 0.82 (Chex/EtOAc 7:3); [a]²⁵_{D} +73° (c 1.0, CHCl₃); ¹H NMR (Pyr-d₅, 400 MHz) δ: 8.91 (d, *J*_{NH,2} = 9.1 Hz, 1H, N*H*), 5.90 (high-order m, 1H, H-2_{All}), 5.27 (br dd, *J* = 17.2, 2.9 Hz, 1H, H-3a_{All}), 5.16-5.11 (m, 2H, H-1, H-3b_{All}), 4.54 (td, *J*_{N*H*,2} = *J*_{2,3} = 9.5 Hz, *J*_{1,2} = 3.8 Hz, 1H, H-2), 4.26 (t, *J*_{2,3} = *J_{3,4} =* 9.3 Hz, 1H, H-3), 4.22 (ddt, *J* = 13.0, 5.5, 1.3 Hz, 1H, H-1a_{All}), 4.06-3.99 (m, 2H, H-1b_{All}, H-6a), 3.97-3.85 (m, 2H, H-5, H-6b), 3.71 (t, *J*_{3,4}=*J*_{4,5}= 8.9 Hz, 1H, H-4), 1.52 (s, 3H, *C*(C*H*₃)₂)*,* 1.49 (s, 3H, C(C*H*₃)₂), 1.00 (s, 9H, C(C*H*₃)₃), 0.16 (s, 3H, Si(C*H*₃)₂), 0.14 (s, 3H, Si(C*H*₃)₂). ¹³C NMR (Pyr-d₅, 100 MHz) δ: 163.1 (*C*=O), 134.5 (C-2_{All}), 118.8 (C-3_{Alll}), 100.1 (*C*(CH₃)₂), 97.5 (C-1), 94.0 (CCl₃), 75.8 (C-4), 71.4 (C-3), 69.3 (C-1_{All}), 64.5 (C-5), 62.8 (C-6), 57.9 (C-2), 29.7 (C(*C*H₃)₂), 26.6 (C(*C*H₃)₃, 3C), 19.5 (C(*C*H₃)₂), 18.9 (*C*(CH₃)₃), -3.2 (Si(*C*H₃)₂), -4.3 (Si(*C*H₃)₂). HR-ESI-TOF-MS *m*/*z* 518.1277 [M + H]⁺ (calcd for C₂₀H₃₅Cl₃NO₆Si, 518.1299).

### 3-O-tert-Butyldimethylsilyl-2-deoxy-4,6-O-isopropylidene-2-trichloroacetamido-α-D-glucopyranosyl trichloroacetimidate (9).

1,5-Cyclooctadiene-bis(methyldiphenylphosphine)-iridium hexafluorophosphate (815 mg, 0.96 mmol, 0.05 equiv) was dissolved in anhydrous THF (96 mL) and the resulting red solution was degassed under an argon atmosphere. Hydrogen was bubbled through the solution for 5 min, then the resulting yellow solution was once again degassed under an argon atmosphere. A solution of **28** (10.0 g, 19.3 mmol) in anhydrous THF (96 mL) was then added. The mixture was stirred overnight at room temperature under an argon atmosphere. A solution of iodine (9.78 g, 38.5 mmol, 2.0 equiv) in THF/H₂O (115 mL, 4:1 v/v) was added to the mixture, which was stirred for 1 h at room temperature. The reaction was then quenched by the addition of a freshly prepared solution of 10% aqueous sodium bisulfite (75 mL). The mixture was concentrated (to 1/3 volume) under reduced pressure and the aqueous phase was extracted three times with CH₂Cl₂ (3×250 mL). The organic layer was washed with H₂O (2×250 mL), brine (1×250 mL), dried over anhydrous Na₂SO₄, filtered and evaporated to dryness. The residue was purified by flash chromatography (Chex/EtOAc 9:1 to 6:4) to afford 3-*O-tert*-butyldimethylsilyl-2-deoxy-4,6-*O-*isopropylidene-2-trichloroacetamido-α,β-D-glucopyranose (7.99 g, 87%, 2 steps, ratio α/β ≈ 7:1) as a yellow amorphous powder. R_{f} 0.46 (Chex/EtOAc 7:3); [a]²⁵D +22° (c 1.0, CHCl₃); ¹H NMR (Pyr-d₅, 400 MHz) δ (α): 9.31 (br s, 1H, N*H*), 8.37 (br s, 1H, O*H*), 5.65 (t, *J*_{1,2} = *J*_{1,OH} 3.4 Hz, 1H, H-1), 4.55 (td, *J*_{2,3} *= J*_{2,NH} *=* 9.5 Hz, *J*_{1,2} = 3.7 Hz, 1H, H-2), 4.39 (t, *J*_{2,3} = *J*_{3,4} = 9.0 Hz, 1H, H-3), 4.34 (dt, *J*_{4,5} = 15.0 Hz, *J*_{5,6a} = *J*_{5,6b} = 5.1 Hz, 1H, H-5), 4.02 (dd, *J*_{6a,66} = 10.7 Hz, *J*_{5,6a} = 5.4 Hz, 1H, H-6a), 3.92 (t, *J*_{5,6a} = *J*_{6a,6b} = 10.6 Hz, 1H, H-6b), 3.79 (t, *J*_{3,4} = *J*_{4,5} = 9.2 Hz, 1H, H-4), 1.55 (s, 3H, C(C*H*₃)₂), 1.53 (s, 3H, C(C*H*₃)₂), 1.06 (s, 9H, C(C*H*₃)₃), 0.25 (s, 6H, Si(C*H*₃)₂). ¹³C NMR (Pyr-d₅, 100 MHz) δ (α): 163.0 (*C*=O), 100.1 (*C*(CH₃)₂), 94.2 (*C*Cl₃), 92.7 (C-1), 76.2 (C-4), 71.6 (C-3), 64.1 (C-5), 63.3 (C-6), 58.7 (C-2), 29.7 (C(*C*H₃)₂), 26.6 (C(*C*H₃)₃), 19.5 (C(*C*H₃)₂), 19.0 (*C*(CH₃)₃), -3.2 (Si(*C*H₃)₂), -4.2 (Si(*C*H₃)₂). HR-ESI-TOF-MS *m*/*z* 478.0997 [M + H]⁺ (calcd for C₁₇H₃₁ChNO₆Si, 478.0968). To a solution of 3-*O-tert*-butyldimethylsilyl-2-deoxy-4,6-*O*-isopropylidene-2-trichloroacetamido-α,β,-D-glucopyranose (7.88 g, 16.5 mmol) in anhydrous DCE (33 mL) was added CCl₃CN (8.3 mL, 82 mmol, 5.0 equiv) and DBU (0.74 mL, 4.9 mmol, 0.30 equiv). The mixture was stirred for 1 h at room temperature under an argon atmosphere, then the mixture was purified by flash chromatography (isocratic Chex/EtOAc 9:1 + 1% Et₃N) to afford 9 (8.62 g, 84%, α/β 7:1) as a white foam. *R_{f}* 0.74 (Chex/EtOAc 7:3); [α]²⁵_{D} +63° (*c* 1.0, CHCl₃); ¹H NMR (Pyr-d₅, 400 MHz) δ (α): 10.48 (s, 1H, C=N*H*), 8.26 (d, *J*_{NH,2} = 8.7 Hz, 1H, N*H*), 6.97 (d, *J*_{1,2} = 3.9 Hz, 1H, H-1), 4.78 (td, *J*_{NH,2} = *J*_{2,3} = 9.4 Hz, *J*_{1,2} = 4.0 Hz, 1H, H-2), 4.48 (t, *J*_{3,4} = *J*_{2,3} = 9.3 Hz, 1H, H-3), 4.22 (high-order m, 1H, H-5), 4.06 (dd, *J*_{6a,6b} = 10.7 Hz, *J*_{5,6a} = 5.1 Hz, 1H, H-6a), 3.95-3.87 (m, 2H, H-6b, H-4), 1.55 (s, 3H, C(C*H*₃)₂), 1.51 (s, 3H, C(C*H*₃)₂), 1.00 (s, 9H, C(C*H*₃)₃), 0.21 (s, 3H, Si(C*H*₃)₂), 0.18 (s, 3H, Si(C*H*₃)₂). ¹³C NMR (Pyr-d₅, 100 MHz) δ (α): 163.0 (*C*=O), 160.1 (*C*=NH), 100.5 (*C*(CH₃)₂), 95.6 (C-1), 93.5 (*C*Cl₃), 91.7 (*C*Cl₃), 74.9 (C-4), 71.6 (C-3), 67.1 (C-5), 62.5 (C-6), 57.2 (C-2), 29.5 (C(*C*H₃)₂), 26.5 (C(*C*H₃)₃, 3C), 19.5 (C(*C*H₃)₂), 18.9 (*C*(CH₃)₃), -3.2 (Si(*C*H₃)₂), -4.3 (Si(*C*H₃)₂).

### 2-Methyl-(1,2-dideoxy-5,6-O-isopropylidene-α-D-glucofurano)-[2,1-d]-2-oxazoline (29).

To a solution of *N*-acetyl-D-glucosamine (30.0 g, 136 mmol) in anhydrous acetone (600 mL) was carefully added dry FeCl₃ (44.0 g, 271 mmol, 2.0 equiv). The mixture was stirred for 30 min at reflux under an argon atmosphere. After cooling to room temperature, saturated aqueous Na₂CO₃ was added until neutralization. The mixture was filtered, the solid was washed several times with CH₂Cl₂ and the organic solvents were evaporated under reduce pressure. The aqueous phase was then extracted with CH₂Cl₂ (3×250 mL). The organic layer was washed with brine (1×250 mL), dried over anhydrous Na₂SO₄, filtered and evaporated to dryness to give a brown oil (**29**, 23.4 g, 71 %), which was >95% pure by NMR and used directly for the next step without further purification. Physical and analytical data of **29⁵⁷** were in agreement with those published in the literature. HR-ESI-TOF-MS *m*/*z* 244.1140 [M + H]⁺ (calcd for C₁₁H₁₈NO₅, 244.1185).

### 2-Methyl-(3-O-tert-butyldimethylsilyl-1,2-dideoxy-5,6-O-isopropylidene-α-D-glucofurano)-[2,1-d]-2-oxazoline (30).

To a solution of oxazoline **29** (4.00 g, 16.4 mmol) in anhydrous THF (49 mL) was added DMAP (201 mg, 1.64 mmol, 0.1 equiv), imidazole (2.80 g, 41.1 I mmol, 2.5 equiv) and TBSCI (4.96 g, 32.9 mmol, 2.0 equiv). The mixture was stirred for 2 h at reflux under an argon atmosphere. After cooling to room temperature, the precipitate was filtered and the filtrate was concentrated under reduced pressure. The resulting residue was purified by flash chromatography (Chex/EtOAc 9:1 to 7:3) to afford **30** (3.05 g, 53%) as a yellow amorphous solid. [α]²⁵_{D} -27° (*c* 1.0, CHCl₃); ¹H NMR (CDCl₃, 400 MHz) δ: 6.09 (d, *J*_{1,2} = 5.1 Hz, 1H, H-1), 4.32-4.28 (m, 2H, H-2, H-3), 4.23 (high order m, 1H, H-5), 4.08 (dd, *J*_{6a,6b}=8.4 Hz, *J*_{5,6a} = 6.2 Hz, 1H, H-6a), 3.96 (dd, *J*_{6a,6b} = 8.5 Hz, *J*_{5,6b =} 5.7 Hz, 1H, H-6b), 3.69 (dd, *J*_{4,5} = 7.7 Hz, *J*_{3,4} = 2.8 Hz, 1H, H-4), 2.01 (d, *J* = 1.5 Hz, 3H, C*H*₃), 1.37 (s, 3H, C(C*H*₃)₂), 1.31 (s, 3H, C(C*H*₃)₂), 0.91 (s, 9H, C(C*H*₃)₃), 0.15 (s, 3H, Si(C*H*₃)₂), 0.12 (s, 3H, Si(C*H*₃)₂). ¹³C NMR (CDCl₃, 100 MHz) δ: 166.9 (*C*=N), 109.1 (*C*(CH₃)₂), 107.1 (C-1), 82.7 (C-4), 79.2 (C-3), 75.7 (C-2), 72.4 (C-5), 67.5 (C-6), 26.9 (C(*C*H₃)₂), 25.9 (C(*C*H₃)₃, 3C), 25.4 (C(*C*H₃)₂), 18.3 (*C*(CH₃)₃), 14.3 (*C*H₃), -4.8 (Si(*C*H₃)₂), -5.0 (Si(*C*H₃)₂). HR-ESI-TOF-MS *m*/*z* 358.2017 [M + H]⁺ (calcd for C₁₇H₃₂NO₅Si, 358.2050).

### 2-Methyl-(1,2-dideoxy-5,6-O-isopropylidene-3-O-para-methoxybenzyl-α-D-glucofurano)-[2,1-d]-2-oxazoline (31).

To a solution of oxazoline **29** (1.00 g, 4.11 mmol) in anhydrous THF/DMF (37 mL, 2:1 v/v) was added NaH (60% oil dispersion, 166 mg, 4.93 mmol, 1.2 equiv) at 0 °C. After 1 h at this temperature, catalytic TBAI (76 mg, 0.21 mmol, 0.05 equiv) and PMBCl (669 µL, 4.93 mmol, 1.2 equiv) were added and the mixture was stirred for 4 h at room temperature under an argon atmosphere. After cooling to 0 °C, MeOH was carefully added to quench the reaction. MeOH and THF were then evaporated under reduced pressure, H₂O (50 mL) was added and the aqueous phase was extracted with CH₂Cl₂ (3×100 mL). The organic layer was dried over anhydrous Na₂SO₄ filtered and evaporated to dryness. The resulting residue was purified by flash chromatography (Chex/EtOAc 5:5 to 2:8) to furnish **31** (759 mg, 51%) as a yellow oil. *R_{f}* 0.33 (CH₂Cl₂/MeOH 95:5); [α]²⁵D -8° (c 1.0, CHCl₃); ¹H NMR (CDCl₃, 400 MHz) δ: 7.30-7.26 (m, 2H, C*H*_{Ph}), 6.89-6.85 (m, 2H, C*H*_{Ph}), 6.11 (d, *J*_{1,2} = 5.1 Hz, 1H, H-1), 4.61, (dd, *J* = 26.0, 11.4 Hz, 2H, C*H*₂Ph), 4.49 (high order m, 1H, H-2), 4.34 (dd, *J*_{4,5} = 12.9 Hz, *J*_{5,6ab} = 6.1 Hz, 1H, H-5), 4.10-4.06 (m, 2H, H-6a, H-3), 4.00 (dd, *J*_{6a,6b} = 8.6 Hz, *J*_{5,6b} = 5.6 Hz, 1H, H-6b), 3.83 (dd, *J*_{4,5} = 6.8 Hz, *J*_{3,4} = 3.0 Hz, 1H, H-4), 3.80 (s, 3H, OC*H*₃), 2.01 (d, *J* = 1.5 Hz, 3H, C*H*₃), 1.41 (s, 3H, C(C*H*₃)₂), 1.37 (s, 3H, C(C*H*₃)₂). ¹³C NMR (CDCl₃, 100 MHz) δ: 167.0 (*C*=N), 159.6 (C_{Ph}), 129.9 (C_{Ph}), 129.6 (2×*C*H_{Ph}), 114.0 (2×*C*H_{Ph}), 109.1 (*C*(CH₃)₂), 107.2 (C-1), 81.7 (C-4), 81.4 (C-3), 75.8 (C-2), 72.9 (C-5), 72.1 (*C*H₂Ph), 67.1 (C-6), 55.4 (O*C*H₃), 26.9 (C(*C*H₃)₂), 25.5 (C(*C*H₃)₂), 14.3 (CH₃). HR-ESI-TOF-MS *m*/*z* 364.1762 [M + H]⁺ (calcd for C₁₉H₂₆NO₆, 364.1760).

### 2-Methyl-(3-O-allyl-1,2-dideoxy-5,6-O-isopropylidene-α-D-glucofurano)-[2,1-d]-2-oxazoline (33).

To a solution of oxazoline **29** (1.00 g, 4.11 mmol) in anhydrous DMF (12.3 mL) was added NaH (60% oil dispersion, 414 mg, 12.3 mmol, 3.0 equiv) at 0 °C. After 1 h at this temperature, AllBr (1.07 mL, 12.3 mmol, 3.0 equiv) was added dropwise and the mixture was stirred overnight at room temperature under an argon atmosphere. After cooling to 0 °C, MeOH was carefully added to quench the reaction. H₂O (50 mL) was added and the aqueous phase was extracted with CH₂Cl₂ (3×100 mL). The organic layer was dried over anhydrous Na₂SO₄, filtered and evaporated to dryness. The resulting residue was purified by flash chromatography (Chex/EtOAc 7:3 to 2:8) to give **33** (892 mg, 77%) as a yellow oil. *R_{f}* 0.28 (CH₂Cl₂/MeOH 95:5); [α]²⁵_{D} -46° (*c* 1.0, CHCl₃); ¹H NMR (CDCl₃, 400 MHz) δ: 6.10 (d, *J_{1,2} =* 5.1 Hz, 1H, H-1), 5.90 (high-order m, 1H, H-2_{All}), 5.31 *(ddd, J =* 17.3, 3.3, 1.5 Hz, 1H, H-3a_{All}), 5.20 (ddd, *J* = 10.5, 2.9, 1.4 Hz, 1H, H-3b_{All}), 4.48 (high-order m, 1H, H-2), 4.32 (dd, *J*_{4,5} *=* 12.9 Hz, *J*_{5,6ab} = 6.1 Hz, 1H, H-5), 4.18 (ddt, *J =* 12.9, 5.3, 1.4 Hz, 1H, H-1a_{All}), 4.14-4.04 (m, 4H, H-1b_{All}, H-6a, H-3, H-6b), 3.81 (dd, *J*_{4,5} = 7.1 Hz, *J*_{3,4} = 3.1 Hz, 1H, H-4), 2.01 (d, *J* = 1.5 Hz, 3H, C*H*₃), 1.40 (s, 3H, C(C*H*₃)₂), 1.34 (s, 3H, C(C*H*₃)₂). ¹³C NMR (CDCl₃, 100 MHz) δ: 167.0 (*C*=N), 134.3 (C-2_{All}), 117.5 (C-3_{All}), 109.1 (*C*(CH₃)₂), 107.1 (C-1), 81.6 (C-4), 81.5 (C-3), 75.8 (C-2), 72.7 (C-5), 71.2 (C-1_{All}), 67.0 (C-6), 26.9 (C(*C*H₃)₂), 25.5 (C(*C*H₃)₂), 14.3 (*C*H₃). HR-ESI-TOF-MS *m*/*z* 284.1468 [M + H]⁺ (calcd for C₁₄H₂₂NO_{5,} 284.1498).

### 2-Bromoethyl 2-acetamido-2-deoxy-β-D-glucopyranoside (32).

To a solution of **30** (1.00 g, 2.80 mmol) in anhydrous 2-bromoethanol (5.6 mL) was added anhydrous PTSA (482 mg, 2.80 mmol, 1.0 equiv) and 4 Å AW molecular sieves (150 mg). The mixture was stirred overnight at room temperature under an argon atmosphere. NaHCO₃ was added until neutrality was reached, then the mixture was filtered and the solvents were evaporated under reduced pressure. The resulting residue was purified by flash chromatography (CH₂Cl₂/MeOH 9:1 to 8:2) to give **32** (782 mg, 85%) as a white amorphous powder contaminated with PTSA. Physical and analytical data of **32⁵⁴** were in agreement with those published in the literature. HR-ESI-TOF-MS *m*/*z* 350.0190 [M + Na]⁺ (calcd for C₁₀H₁₈BrNO₆, 350.0215).

### 2-Bromoethyl 2-acetamido-3-O-allyl-2-deoxy-β-D-glucofuranoside (36).

To a solution of **33** (5.00 g, 17.6 mmol) in anhydrous 2-bromoethanol (29 mL) was added Yb(OTf)₃ (5.47 g, 8.82 mmol, 0.5 equiv). The mixture was stirred overnight at room temperature under an argon atmosphere, then the solvents were evaporated under reduced pressure. The resulting residue was purified by flash chromatography (CH₂Cl₂/MeOH 95:5 to 85:15) to afford **36** (6.47 g, 99%) as a brownish foam. *R_{f}* 0.12 (CH₂Cl₂/MeOH 95:5); [a]²⁵_{D} -71° (c 1.0, MeOH); ¹H NMR (CDCl₃, 400 MHz) δ: 6.43 (d, *J*_{NH,2} = 7.7 Hz, 1H, N*H*), 5.89 (high-order m, 1H, H-2_{All}), 5.32 (br d, *J* = 17.3 Hz, 1H, H-3a_{All}), 5.19 (br d, *J* = 10.5 Hz, 1H, H-3b_{All}), 4.95 (s, 1H, H-1), 4.41 (d, *J*_{1,2} = 7.6 Hz, 1H, H-2), 4.32 (br dd, *J*= 13.0, 4.9 Hz, 1H, H-1a_{All}), 4.26 (dd, *J*_{4,5} = 8.7 Hz, *J*_{3,4} = 6.1 Hz, 1H, H-4), 4.09-4.02 (m, 3H, H-1b_{All}, H-5, H-3), 3.93 (dt, *J =* 11.1, 6.0 Hz, 1H, OC*H*₂a), 3.82 (dd, *J=* 11.5, 3.1 Hz, 1H, H-6a), 3.76-3.67 (m, 2H, H-6b, OC*H*₂b), 3.44 (br t, *J* = 6.1 Hz, 2H, C*H*₂Br), 2.98 (br s, 2H, 2×O*H*), 1.98 (s, 3H, C(O)C*H*₃). ¹³C NMR (CDCl₃, 100 MHz) δ: 170.2 (*C*=O), 133.8 (C-2_{All}), 118.1 (C-3_{All}), 107.0 (C-1), 83.0 (C-3), 80.1 (C-4), 70.9 (C-1_{All}), 70.8 (C-5), 68.2 (O*C*H₂), 64.0 (C-6), 59.7 (C-2), 30.2 (*C*H₂Br), 23.2 (C(O)*C*H₃). HR-ESI-TOF-MS *m*/*z* 390.0522 [M + Na]⁺ (calcd for C₁₃H₂₂BrNO₆Na, 390.0528).

### 2-Bromoethyl 2-acetamido-3-O-allyl-2-deoxy-β-D-glucopyranoside (34).

To a solution of **33** (100 mg, 0.353 mmol) in anhydrous 2-bromoethanol (0.71 mL) was added CSA (20 mg, 0.086 mmol, 0.25 equiv). The mixture was stirred 4 d at room temperature under an argon atmosphere. The reaction was neutralized by the addition of NaHCO₃, filtered and the solvents were evaporated under reduced pressure. The resulting residue was purified by flash chromatography (CH₂Cl₂/MeOH 95:5 to 9:1) to give **34** (70 mg, 55%) as a white amorphous powder. *R_{f}* 0.13 (CH₂Cl₂/MeOH 95:5); [α]²⁵_{D} -19° (c 1.0, MeOH); ¹H NMR (DMSO-d₆, 400 MHz) δ: 7.78 (d, *J_{NH,2} =* 9.1 Hz, 1H, N*H*), 5.84 (high-order m, 1H, H-2_{All}), 5.18 (ddd, *J*= 17.2, 3.8, 1.8 Hz, 1H, H-3a_{All}), 5.04 (ddd, *J*= 10.4, 3.5, 1.5 Hz, 1H, H-3b_{All}), 4.41 (d, *J*_{1,2} = 8.5 Hz, 1H, H-1), 4.21 (ddt, *J =* 13.1, 5.3, 1.5 Hz, 1H, H-1a_{All}), 4.05 (ddt, *J =* 13.1, 5.3, 1.5 Hz, 1H, H-1b_{All}), 3.95 (dt, *J =* 11.6, 5.8 Hz, 1H, OC*H*₂a), 3.76 *(dt, J =* 11.7, 6.0 Hz, 1H, OC*H*₂b), 3.68 (br d, *J*_{6a,6b} = 10.7 Hz, 1H, H-6a), 3.60-3.43 (m, 4H, C*H*₂Br, H-6b, H-2), 3.32-3.26 (m, 1H, H-3), 3.20 (high-order m, 1H, H-5), 3.13 (ddd, *J* = 9.5, 5.7, 2.0 Hz, 1H, H-4), 1.80 (s, 3H, C(O)C*H*₃). ¹³C NMR (DMSO-d₆, 100 MHz) δ: 168.9 (*C*=O), 136.0 (C-2_{All}), 115.4 (C-3_{All}), 100.7 (C-1), 82.3 (C-3), 77.0 (C-4), 72.3 (C-1_{All}), 69.9 (C-5), 68.3 (O*C*H₂), 60.8 (C-6), 54.0 (C-2), 31.9 (*C*H₂Br), 23.0 (C(O)CH₃). HR-ESI-TOF-MS *m*/*z* 390.0540 [M + Na]⁺ (calcd for C₁₃H₂₂BrNO₆Na, 390.0528).

### 2-Azidoethyl 2-acetamido-3-O-allyl-2-deoxy-β-D-glucopyranoside (37).

**To a solution of 34 (3.19 g, 8.67 mmol) in anhydrous DMF (43.4** mL) was added NaI (6.50 g, 43.4 mmol, 5.0 equiv) and NaN₃ (2.82 g, 43.4 mmol, 5.0 equiv). The mixture was stirred overnight at 80 °C, then the solvents were removed under reduced pressure. The resulting residue was adsorbed on silica gel (22 g) and purified by flash chromatography (CH₂Cl₂/MeOH 95:5 to 9: 1) to give **37** (6.78 g) contaminated with sodium salts. *R_{f}* 0.11 (CH₂Cl₂/MeOH 95:5); [α]²⁵_{D} -35° (c 1.0, MeOH); ¹H NMR (Pyr-d₅, 400 MHz) δ: 9.56 (br s, 1H, N*H*), 6.58 (br s, 2H, 2×OH), 6.06 (high-order m, 1H, H-2_{All}), 5.36-5.25 (m, 2H, H-3a_{All}, H-1), 5.02 (br d, *J* = 10.4 Hz, 1H, H-3b_{All}), 4.70 (ddd, *J =* 28.4, 13.0, 5.3 Hz, 2H, H-1a_{All}, H-1b_{All}), 4.52 (dd, *J*_{1,2} = 18.8 Hz, *J*_{2,3} = 9.6 Hz, 1H, H-2), 4.42-4.31 (m, 2H, H-3, H-6a), 4.26 (dd, *J*_{6a,bb} = 11.9 Hz, *J*_{5,6b} = 4.5 Hz, 1H, H-6b), 4.11-4.01 (m, 2H, C*H*₂N₃a, H-4), 3.96-3.82 (m, 2H, C*H*₂N₃b, H-5), 3.59-3.46 (m, 1H, OC*H*₂a), 3.45-3.36 (m, 1H, OC*H*₂b), 2.23 (s, 3H, C(O)C*H*₃). ¹³C NMR (Pyr-d₅, 100 MHz) δ: 172.5 (C=O), 136.4 (C-2_{All}), 116.6 (C-3_{All}), 102.0 (C-1), 83.7 (C-3), 78.0 (C-5), 74.5 (C-1_{All}), 71.9 (C-4), 68.8 (CH₂N₃), 62.2 (C-6), 56.1 (C-2), 51.3 (O*C*H₂), 23.9 (C(O)*C*H₃). HR-ESI-TOF-MS *m*/*z* 353.1406 [M + Na]⁺ (calcd for C₁₃H₂₂N₄O₆Na, 353.1437).

### 2-Azidoethyl 2-acetamido-3-O-allyl-4,6-di-O-benzyl-2-deoxy-β-D-glucopyranoside (38).

To a solution of **37** (2.72 g, 8.24 mmol) in anhydrous DMF (165 mL) was slowly added BnBr (3.91 mL, 32.9 mmol, 4.0 equiv) at 0 °C. NaH (858 mg, 25.5 mmol, 3.0 equiv) was then added portionwise and the mixture was stirred for 2 h at 0 °C. Few drops of MeOH were added to quench the reaction, the mixture was diluted with EtOAc (250 mL), H₂O was added carefully (250 mL) and the aqueous phase was extracted with EtOAc (3x250 mL). The organic layer was washed with H₂O (1×250 mL), dried (anhydrous Na₂SO₄), filtered and the solvents were removed under reduced pressure. The resulting residue was purified by flash chromatography (Chex/EtOAc 6:4 to 100% EtOAc) to furnish **38** (2.39 g, 57%, 2 steps from **34)** as a white amorphous powder. *R_{f}* 0.06 (Chex/EtOAc 7:3); [a]²⁵_{D} +1° (c 1.0, CHCl₃); ¹H NMR (CDCl₃, 400 MHz) δ: 7.35-7.21 (m, 10H, C*H*_{Ph}), 5.89 (high-order m, 1H, H-2_{All}), 5.73 (d, *J_{NH,2}* = 7.3 Hz, 1H, N*H*), 5.24 (ddd, *J =* 17.3, 3.2, 1.6 Hz, 1H, H-3a_{All}), 5.15 (br d, *J*= 10.4 Hz, 1H, H-3b_{All}), 4.96 (d, *J*_{1,2} = 7.8 Hz, 1H, H-1), 4.78 (d, *J* = 11.1 Hz, 1H, C*H*₂Ph), 4.60 (d, *J* = 12.2 Hz, 1H, C*H*₂Ph), 4.57 (d, *J* = 11.3 Hz, 1H, C*H*₂Ph), 4.54 (d, *J* = 12.2 Hz, 1H, C*H*₂Ph), 4.27 (br dd, *J* = 12.6, 5.5 Hz, 1H, H-1a_{All}), 4.15 (br dd, *J* = 12.6, 5.8 Hz, 1H, H-1b_{All}), 4.11-4.00 (m, 2H, H-3, OC*H*₂a), 3.76-3.66 (m, 3H, OC*H*₂b, H-6a, H-6b), 3.60-3.52 (m, 2H, H-4, H-5), 3.48 (ddd, *J* = 13.2, 8.0, 3.4 Hz, 1H, C*H*₂N₃a), 3.32-3.22 (m, 2H, H-2, C*H*₂N₃b), 1.98 (s, 3H, C(O)C*H*₃). ¹³C NMR (CDCl₃, 100 MHz) δ: 170.8 (*C*=O), 138.1 (2×C_{Ph}), 134.9 (C-2_{All}), 128.4-127.7 (*C*H_{Ph}), 117.0 (C-3_{All}), 99.7 (C-1), 80.3 (C-3), 78.4 (C-4) 74.8 (C-5), 74.7 (*C*H₂Ph), 73.8 (C-1_{All}), 73.5 (CH₂Ph), 68.9 (C-6), 68.3 (OCH₂), 57.4 (C-2), 50.7 (*C*H₂N₃), 23.7 (C(O)*C*H₃). HR-ESI-TOF-MS *m*/*z* 511.2566 [M + H]⁺ (calcd for C₂₇H₃₅N₄O₆, 511.2556).

### 2-Azidoethyl 2-acetamido-4,6-di-O-benzyl-2-deoxy-β-D-glucopyranoside (6).

1,5-Cyclooctadiene-bis(methyldiphenylphosphine)-iridium hexafluorophosphate (190 mg, 0.22 mmol, 0.05 equiv) was dissolved in anhydrous THF (22 mL) and the resulting red solution was degassed under an argon atmosphere. Hydrogen was bubbled through the solution for 5 min, then the resulting yellow solution was once again degassed under an argon atmosphere. A solution of **38** (2.29 g, 4.49 mmol) in anhydrous THF (22 mL) was then added. The mixture was stirred for 16 h at room temperature under an argon atmosphere. A solution of iodine (2.28 g, 8.97 mmol, 2.0 equiv) in THF/H₂O (27 mL, 4:1 v/v) was added to the mixture, which was stirred for 1 h at room temperature. The reaction was then quenched by the addition of a freshly prepared solution of 10% aqueous sodium bisulfite. The mixture was diluted with CH₂Cl₂ (250 mL), water was added (100 mL) and the aqueous phase was extracted with CH₂Cl₂ (3x250 mL). The organic layer was dried over anhydrous Na₂SO₄, filtered and evaporated to dryness. The resulting residue was purified by flash chromatography (Chex/EtOAc 2:8 to 100% EtOAc) to afford **6** (1.72 g, 82%, 2 steps) as a yellow amorphous powder. [a]²⁵D -37° (c 1.0, CHCl₃); ¹H NMR (CDCl₃, 400 MHz) δ: 7.35-7.24 (m, 10H, C*H*_{Ph}), 5.88 (d, *J*_{NH,2} = 4.2 Hz, 1H, N*H*), 4.94 (d, *J* = 10.2 Hz, 1H, C*H*₂Ph), 4.61 (d, *J* = 11.2 Hz, 1H, C*H₂*Ph)*,* 4.60 (d, *J* = 12.2 Hz, 1H, C*H*₂Ph), 4.54 (d, *J* = 12.2 Hz, 1H, C*H*₂Ph), 4.53 (d, *J*_{1,2} = 8.2 Hz, 1H, H-1), 4.09 (ddd, *J =* 10.8, 4.5, 3.2 Hz, 1H, OC*H*₂a), 3.93 (dt, *J =* 9.5, 4.0 Hz, 1H, H-3), 3.78-3.65 (m, 3H, OC*H*₂b, H-6a, H-6b), 3.61-3.49 (m, 4H, H-2, C*H*₂N₃a, H-4, H-5), 3.29 (ddd, *J* = 13.2, 4.0, 2.9, 1H, C*H*₂N₃b), 2.05 (s, 3H, C(O)C*H*₃). ¹³C NMR (CDCl₃, 100 MHz) δ: 173.0 (*C*=O), 138.4 (C_{Ph}), 138.1 (C_{Ph}), 128.7-127.8 (CH_{Ph}), 100.5 (C-1), 78.4 (C-4), 76.3 (C-3), 75.2 (C-5), 74.9 (CH₂Ph), 73.6 (*C*H₂Ph), 69.0 (C-6), 68.6 (OCH₂), 58.6 (C-2), 50.8 (CH₂N₃), 23.6 (C(O)CH₃). HR-ESI-TOF-MS *m*/*z* 471.2222 [M + H]⁺ (calcd for C₂₄H₃₁N₄O₆, 471.2244).

### Allyl (2,3,4,6-tetra-O-benzyl-α-D-glucopyranosyl)-(1→4)-2-O-acetyl-α-L-rhamnopyranoside (39).

To a solution of diol **10**⁶³ (2.96 g, 4.07 mmol) in anhydrous CH₃CN (8.3 mL) was added MeC(OMe)₃ (1.04 mL, 8.25 mmol, 2.0 equiv) and PTSA (14 mg, 0.081 mmol, 0.02 equiv). The mixture was stirred for 1 h at room temperature, then 80% aqueous HOAc (8.3 mL) was added at 0 °C. The mixture was stirred for another 1 h at room temperature, then cold H₂O (50 mL) was added dropwise to quench the reaction. The aqueous phase was extracted with CH₂Cl₂ (3×100 mL), then the organic layer was washed with brine (1 ×50 mL), dried over anhydrous Na₂SO₄ and evaporated to dryness. The alcohol **39** obtained as a colorless oil was used directly for the next step without further purification. ¹H NMR (CDCl₃, 400 MHz) δ: 7.38-7.12 (m, 20H, CH_{PH}), 5.90 (high-order m, 1H, H-2_{All}), 5.30 (ddd, *J* = 17.2, 3.2, 1.6 Hz, 1H, H-3a_{All}), 5.21 *(ddd, J =* 10.4, 2.8, 1.3 Hz, 1H, H-3b_{All}), 5.14 (dd, *J*_{2,3} = 3.7 Hz, *J*_{1,2} = 1.7 Hz, 1H, H-2_{C}), 4.98-4.93 (m, 2H, H-1_{C}, C*H*₂Ph), 4.82 (d, *J* = 10.9 Hz, 1H, C*H*₂Ph), 4.82 (d, *J* = 10.9 Hz, 1H, C*H*₂Ph), 4.77 (d, *J*_{1,2} = 1.6 Hz, 1H, H-1_{E}), 4.71 (dd, *J =* 22.9, 11.7 Hz, 2H, C*H*₂Ph), 4.56 (d, *J* = 12.1 Hz, 1H, C*H*₂Ph), 4.47 (d, *J* = 10.9 Hz, 1H, C*H*₂Ph), 4.47 (d, *J =* 12.1 Hz, 1H, C*H*₂Ph), 4.15 (ddt, *J =* 12.6, 5.1, 1.5 Hz, 1H, H-1a_{All}), 4.08 (ddd, *J* = 10.1, 5.1, 2.2 Hz, 1H, H-2_{E}), 4.01-3.93 (m, 3H, H-1b_{All}, H-3_{C}, H-4_{E}), 3.78 (high-order m, 1H, H-5_{C}), 3.66-3.51 (m, 4H, H-6a_{E}, H-6b_{E}, H-5_{E}, H-3_{E}), 3.37 (t, *J*_{3,4} *= J*_{4,5} = 9.2 Hz, 1H, H-4_{C}), 2.11 (s, 3H, C(O)C*H*₃), 1.39 (d, *J*_{5,6} *=* 6.4 Hz, 3H, H-6_{c}). ¹³C NMR (CDCl₃, 100 MHz) δ: 170.6 (*C*=O), 138.8 (C_{Ph}), 138.2 (C_{Ph}), 138.1 (C_{Ph}), 137.8 (C_{Ph}), 133.8 (C-2_{All}), 129.2-127.8 (*C*H_{Ph}), 117.7 (C-3_{All}), 98.8 (C-1_{C}), 96.7 (C-1_{E}), 85.4 (C-4_{C}), 81.9 (C-3_{C}), 80.2 (C-5_{E}), 78.1 (C-3_{E}), 75.8 (CH₂Ph), 75.3 (*C*H₂Ph), 73.8 (2×CH₂Ph), 72.4 (C-2_{C}), 71.4 (C-2_{E}), 68.9 (C-6_{E}), 68.4 (C-1_{All}), 68.4 (C-4_{E}), 66.8 (C-5_{C}), 21.2 (C(O)*C*H₃), 17.9 (C-6_{C}). HR-ESI-TOF-MS *m*/*z* 769.3573 [M + H]⁺ (calcd for C₄₅H₅₃O₁₁, 769.3588), *m*/*z* 791.3387 [M + Na]⁺ (calcd for C₄₅H₅₂O₁₁Na, 791.3408.

### Allyl (2-O-2-(azidomethyl)benzoyl-3,4-di-O-benzyl-α-L-rhamnopyranosyl)-(1→3)-[2,3,4,6-tetra-C-benzyl-α-D-glucopyranosyl-(1→4)]-2-O-acetyl-α-L-rhamnopyranoside (40).

The crude acceptor **39** (3.13 g, 4.08 mmol) and the donor **11** (3.17 g, 4.89 mmol, 1.2 equiv) were dissolved in anhydrous Et₂O (61 mL) with 4 Å AW molecular sieves (1.0 g). The temperature was lowered to -10 °C (acetone/ice water) and the mixture was stirred for 15 min under an argon atmosphere. Then, TMSOTf (74 µL, 0.41 mmol, 0.1 equiv) was added keeping rigorously anhydrous conditions. The mixture was stirred for 1 h at -10 °C to room temperature and the reaction was quenched by the addition of Et₃N (300 µL). The solution was filtered and the solvents were evaporated under reduced pressure and the resulting residue was purified by flash chromatography (Chex/EtOAc 95:5 to 8:2) to afford **40** (4.42 g, 88%, 3 steps) as a white foam. *R_{f}* 0.74 (Chex/EtOAc 7:3); [α]²⁵_{D} +18° (c 1.0, CHCl₃); ¹H NMR (CDCl₃, 400 MHz) δ: 8.07-8.03 (m, 1H, C*H*_{Ph}), 7.62-7.52 (m, 2H, C*H*_{Ph}), 7.46-7.41 (m, 1H, C*H*_{PH}), 7.36-7.10 (m, 30H, C*H*_{PH}), 5.96 (br s, 1H, H-2_{B}), 5.88 (high-order m, 1H, H-2_{All}), 5.29 (br d, *J* = 17.4 Hz, 1H, H-3a_{All}), 5.20 (br d, *J* = 10.5 Hz, 1H, H-3b_{All}), 5.13 (br s, 1H, H-2_{C}), 5.09 (br s, 1H, H-1_{B}), 4.99 (d, *J*_{1,2} = 3.1 Hz, 1H, H-1_{C}), 4.95 (d, *J* = 10.9 Hz, 1H, C*H*₂Ph), 4.93 (d, *J* = 10.5 Hz, 1H, C*H*₂Ph), 4.88-4.73 (m, 6H, C*H*₂Ph (3H), H-1_{E}, C*H*₂N₃), 4.71-4.50 (m, 6H, C*H*₂Ph), 4.37 (d, *J*= 11.9 Hz, 1H, C*H*₂Ph), 4.13 (br dd, *J* = 12.9, 5.3 Hz, 1H, H-1a_{All}), 4.09-4.04 (m, 2H, H-2_{E}, H-3_{C}), 4.03-3.87 (m, 4H, H-5_{E}, H-1b_{All}, H-4_{E}, H-3_{B}), 3.84-3.70 (m, 4H, H-6a_{E}, H-6b_{E}, H-5_{B}, H-5_{C}), 3.56-3.45 (m, 3H, H-4_{C}, H-3_{E}, H-4_{B}), 2.12 (s, 3H, C(O)C*H*₃), 1.34 (d, *J*_{5,6} = 6.1 Hz, 3H, H-6_{C}), 1.27 (d, *J_{5,6}* = 6.1 Hz, 3H, H-6_{B}). ¹³C NMR (CDCl₃, 100 MHz) δ: 170.4 (*C*=O), 165.7 (*C*=O), 139.0 (C_{Ph}), 138.6 (C_{Ph}), 138.6 (C_{Ph}), 138.5 (C_{Ph}), 138.5 (C_{Ph}), 138.4 (C_{Ph}), 137.5 (C_{Ph}), 133.7 (C-2_{All}), 133.0 (*C*H_{Ph}), 131.6 (*C*H_{Ph}), 129.6 (CH_{Ph}), 129.0 (CH_{Ph}), 128.6-127.5 (*C*H_{Ph}), 117.9 (C-3_{All}), 99.7 (C-1_{B}), 98.2 (C-1_{C}), 96.0 (C-1_{E}), 81.9 (C-5_{E}), 81.7 (C-3_{E}), 80.2 (C-3_{C}, C-4_{B}), 78.7 (C-4_{C}), 78.0 (C-5_{C}), 77.5 (C-3_{B}), 75.7 (*C*H₂Ph), 75.7 (*C*H₂Ph), 75.3 (*C*H₂Ph), 74.0 (*C*H₂Ph), 73.0 (*C*H₂Ph), 72.0 (C-2_{C}), 71.8 (C-2_{E}), 70.5 (CH₂Ph), 69.5 (C-2_{B}), 68.8 (C-4_{E}), 68.8 (C-6_{E}), 68.7 (C-1_{All}), 67.4 (C-5_{B}), 53.1 (*C*H₂N₃), 21.3 (C(O)CH₃), 18.6 (C-6_{C}), 18.3 (C-6_{B}). HR-ESI-TOF-MS *m*/*z* 1276.5396 [M + Na]⁺ (calcd for C₇₃H₇₉N₃O₁₆Na, 1276.5358).

### Allyl (2-O-2-(azidomethyl)benzoyl-3,4-di-O-benzyl-α-L-rhamnopyranosyl)-(1→3)-[2,3,4,6-tetra-O-benzyl-α-D-glucopyranosyl-(1→4)]-α-L-rhamnopyranoside (41).

To a solution of **40** (3.00 g, 2.39 mmol) in anhydrous MeOH/CH₂Cl₂ (180 mL, 2:1 v/v) at 0 °C was slowly added AcCl (8.50 mL, 120 mmol, 50 equiv). The mixture was stirred for 6 d at room temperature under an argon atmosphere, then the temperature was lowered to 0 °C and the reaction was quenched by the dropwise addition of Et₃N (16.7 mL). The solvents were then evaporated under reduced pressure and the resulting residue was purified by flash chromatography (Tol/EtOAc 95:5 to 9:1) to give **41** (1.89g, 66%) as a colorless oil along with unreacted **40** (649 mg, 22%) as a yellow oil. *R*_{f} 0.55 (Chex/EtOAc 7:3); [α]²⁵_{D} +26° (c 1.0, CHCl₃); ¹H NMR (CDCl₃, 400 MHz) δ: 8.07-8.04 (m, 1H, C*H*_{Ph}), 7.63-7.58 (m, 1H, C*H*_{Ph}), 7.57-7.53 (m, 1H, C*H*_{Ph}), 7.47-7.41 (m, 1H, C*H*_{Ph}), 7.37-7.10 (m, 30H, C*H*_{Ph}), 5.93 (m, 1H, H-2_{B}), 5.90 (high-order m, 1H, H-2_{All}), 5.29 (br dd, *J =* 17.2, 1.5 Hz, 1H, H-3a_{All}), 5.21 (br d, *J* = 10.3 Hz, 1H, H-3b_{All}), 5.12 (d, *J*_{1,2} = 1.6 Hz, 1H, H-1_{B}), 4.96 (d, *J* = 11.0 Hz, 1H, C*H*₂Ph), 4.95 (d, *J* = 10.8 Hz, 1H, C*H*₂Ph), 4.91 (d, *J*_{1,2} = 3.3 Hz, 1H, H-1_{C}), 4.85 (d, *J* = 11.0 Hz, 1H, C*H*₂Ph), 4.84 (d, *J* = 10.8 Hz, 1H, C*H*₂Ph), 4.80 (d, *J*_{1,2} = 1.7 Hz, 1H, H-1_{E}), 4.77-4.73 (m, 3H, C*H*₂Ph (1H), C*H*₂N₃), 4.68 (d, *J* = 12.7 Hz, 1H, C*H*₂Ph), 4.64 (d, *J* = 11.0 Hz, 1H, C*H*₂Ph), 4.57-4.45 (m, 4H, C*H*₂Ph), 4.33 (d, *J* = 11.9 Hz, 1H, C*H*₂Ph), 4.16 (br dd, *J* = 12.9, 5.3 Hz, 1H, H-1a_{All}), 4.09-3.89 (m, 7H, H-4_{E}, H-2_{E}, H-3_{C}, H-1b_{All}, H-5_{C}, H-5_{E}, H-3_{B}), 3.84 (d, *J* = 9.8 Hz, 1H, H-6a_{E}), 3.79-3.72 (m, 2H, H-6b_{E}, H-5_{B}), 3.68 (t, *J*_{3,4} = *J*_{4,5} = 9.5 Hz, 1H, H-4_{B}), 1.58 (br s, 1H, O*H*), 1.36 (d, *J*₅,₆ = 6.2 Hz, 6H, H-6_{B}, H-6_{C}). ¹³C NMR (CDCl₃, 100 MHz) δ: 165.6 (*C*=O), 139.0-137.6 (C_{Ph}), 133.9 (C-2_{All}), 133.0 (*C*H_{Ph}), 131.6 (*C*H_{Ph}), 129.6 (*C*H_{Ph}), 129.2 (C_{Ph}), 129.0 (C_{Ph}), 128.7-127.5 (*C*H_{Ph}), 117.8 (C-3_{All}), 99.2 (C-1_{B}), 98.3 (C-1_{E}), 98.0 (C-1_{C}), 82.1 (C-5_{E}), 81.8 (C-3_{C}), 81.5 (C-2_{C}), 79.8 (C-3_{E}), 78.4 (C-4_{B}), 77.8 (C-4_{C}), 77.5 (C-3_{B}), 75.7 (*C*H₂Ph), 75.6 (*C*H₂Ph), 75.2 (*C*H₂Ph), 74.0 (*C*H₂Ph), 72.8 (*C*H₂Ph), 71.5 (C-4_{E}), 70.5 (*C*H₂Ph), 70.0 (C-2_{E}), 69.4 (C-2_{B}), 69.0 (C-5_{C}), 68.7 (C-6_{E}), 68.2 (C-1_{All}), 67.1 (C-5_{B}), 53.1 (*C*H₂N₃), 18.6 (C-6_{C}), 18.4 (C-6_{B}). HR-ESI-TOF-MS *m*/*z* 1212.5446 [M + H]⁺ (calcd for C₇₁H₇₈N₃O₁₅, 1212.5433), *m*/*z* 1234.5249 [M + Na]⁺ (calcd for C₇₁H₇₇N₃O₁₅Na, 1234.5253).

### Allyl (2-O-2-(azidomethyl)benzoyl-3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (42).

To a solution of **41** (7.38 g, 6.09 mmol) in anhydrous Py (40.2 mL) was added DMAP (3.72 g, 30.4 mmol, 5.0 equiv) and the temperature was raised to 50 °C. Levulinic anhydride⁷⁵ (13.0 g, 60.9 mmol, 10 equiv) in anhydrous Py (53.6 mL) was then slowly added over 10 min and the mixture was stirred for 1 h at 50 °C under an argon atmosphere. The solvents were evaporated under reduced pressure and the resulting residue was purified by flash chromatography (Tol/EtOAc 95:5 to 9:1) to furnish **42** (8.43 g, 100%) as a yellow oil. *R*_{f} 0.51 (Chex/EtOAc 7:3); [α]²⁵_{D} +31° (c 1.0, CHCl₃); ¹H NMR (CDCl₃, 400 MHz) δ: 7.97-7.93 (m, 1H, C*H*_{Ph}), 7.52-7.47 (m, 1H, C*H*_{Ph}), 7.45-7.42 (m, 1H, C*H*_{Ph}), 7.35-7.30 (m, 1H, C*H*_{Ph}), 7.25-6.99 (m, 30H, C*H*_{PH}), 5.84 (t, *J*_{1,2} = *J*_{2,3} = 2.4 Hz, 1H, H-2_{B}), 5.78 (high-order m, 1H, H-2_{All}), 5.18 (ddd, *J*= 17.2, 3.0, 1.5 Hz, 1H, H-3a_{All}), 5.09 (ddd, *J* = 10.4, 2.6, 1.2 Hz, 1H, H-3b_{All}), 5.04-5.01 (m, 2H, H-1_{B}, H-2_{C}), 4.92 (d, *J*_{1,2} = 3.2 Hz, 1H, H-1_{C}), 4.86 (d, *J* = 10.9 Hz, 1H, C*H*₂Ph), 4.81 (d, *J =* 11.0 Hz, 1H, C*H*₂Ph), 4.76 (d, *J =* 11.2 Hz, 1H, C*H*₂Ph), 4.73 (d, *J* = 11.2 Hz, 1H, C*H*₂Ph), 4.70-4.50 (m, 7H, C*H*₂Ph (5H), C*H*₂N₃, H-1_{E}), 4.43 (d, *J* = 12.1 Hz, 1H, C*H*₂Ph), 4.40 (d, *J* = 10.0 Hz, 1H, C*H*₂Ph), 4.37 (d, *J* = 11.7 Hz, 1H, C*H*₂Ph), 4.23 (d, *J =* 11.9 Hz, 1H, C*H*₂Ph), 4.02 (ddt, *J =* 12.9, 5.4, 1.4Hz, 1H, H-la_{All}), 3.99-3.93 (m, 2H, H-2_{E}, H-3_{C}), 3.92-3.83 (m, 2H, H-5_{E}, H-1b_{All}), 3.80 (dd, *J*_{3,4} = 9.3 Hz, *J*_{2,3} = 3.0 Hz, 1H, H-3_{B}), 3.75 (dd, *J* = 10.7, 1.5 Hz, 1H, H-4_{E}), 3.69-3.56 (m, 4H, H-6a_{E}, H-5_{B}, H-6b_{E}, H-5_{C}), 3.48-3.41 (m, 2H, H-4_{C}, H-3_{E}), 3.37 (t, *J*_{3,4} = *J*_{4,5} = 9.4 Hz, 1H, H-4_{B}), 2.68-2.46 (high-order m, 4H, 2×CH_{2Lev}), 1.98 (s, 3H, C(O)*C*H₃), 1.24 (d, *J*_{5,6} = 6.2 Hz, 3H, H-6_{C}), 1.16 (d, *J*_{5,6} = 6.1Hz, 3H, H-6_{B}). ¹³C NMR (CDCl₃, 100 MHz) δ: 206.0 (C(O)CH_{2Lev}), 172.3 (*C*=O), 165.6 (C=O), 139.0 (C_{Ph}), 138.7 (C_{Ph}), 138.6 (C_{Ph}), 138.5 (C_{Ph}), 138.4 (C_{Ph}), 138.3 (C_{Ph}), 137.6 (C_{Ph}), 133.7 (C-2_{All}), 132.9 (CH_{Ph}), 131.6 (*C*H_{Ph}), 129.6-127.5 (*C*H_{Ph}), 117.8 (C-3_{All}), 100.0 (C-1_{B}), 98.2 (C-1_{C}), 96.0 (C-1_{E}), 81.9 (C-5_{E}), 81.5 (C-3_{E}), 80.2 (C-4_{B}), 80.1 (C-3_{C}), 78.7 (C-4_{C}), 78.0 (C-5_{C}), 77.7 (C-3_{B}), 75.7 (*C*H₂Ph), 75.5 (*C*H₂Ph), 75.2 (*C*H₂Ph), 74.0 (*C*H₂Ph), 72.9 (*C*H₂Ph), 72.4 (C-2_{C}), 71.8 (C-2_{E}), 70.7 (*C*H₂Ph), 69.5 (C-2_{B}), 68.8 (C-6_{E}), 68.7 (C-4_{E}), 68.6 (C-1_{All}), 67.5 (C-5_{B}), 53.1 (*C*H₂N₃), 38.1 (*C*H_{2Lev}), 29.8 (C(O)CH₃), 28.4 (CH_{2Lev)}, 18.7 (C-6_{C}), 18.3 (C-6_{B}). HR-ESI-TOF-MS *m*/*z* 1310.5774 [M + H]⁺ (calcd for C₇₆H₈₄N₃O₁₇, 1310.5801), *m*/*z* 1332.5585 [M + Na]⁺ (calcd for C₇₆H₈₃N₃O₁₇Na, 1332.5620).

### Allyl (3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside(43).

To a solution of **42** (1.72 g, 1.31 mmol) in THF (13.1 mL) was added H₂O (118 µL, 6.56 mmol, 5.0 equiv) and PBu₃ (983 µL, 3.94 mmol, 3.0 equiv). The mixture was stirred for 15 min at room temperature, then the solvents were evaporated under reduced pressure. The resulting residue was purified by flash chromatography (Tol/EtOAc 95:5 to 85:15) to afford **43** (1.37 g, 91%) as a yellow oil. *R_{f}* 0.39 (Chex/EtOAc 7:3); [α]²⁵_{D} +13° (c 1.0, CHCl₃); ¹H NMR (CDCl₃, 400 MHz) δ: 7.39-7.09 (m, 30H, C*H*_{Ph}), 5.90 (high-order m, 1H, H-2_{All}), 5.29 (ddd, *J* = 17.3, 3.1, 1.5 Hz, 1H, H-3a_{All}), 5.21 (ddd, *J*= 10.4, 2.6, 1.3 Hz, 1H, H-3b_{All}), 5.07 (dd, *J*_{1,2} = 3.3, *J*_{2,3} = 1.8 Hz, 1H, H-2_{C}), 5.05 (d, *J*_{1,2} = 3.1 Hz, 1H, H-1_{C}), 5.01 (d, *J*_{1,2} = 1.5 Hz, 1H, H-1_{B}), 4.95 (d, *J* = 11.0 Hz, 1H, C*H*₂Ph), 4.85-4.81 (m, 3H, C*H*₂Ph), 4.78 (d, *J* = 11.5 Hz, 1H, C*H*₂Ph), 4.72 (d, *J*_{1,2} = 1.6 Hz, 1H, H-1_{E}), 4.71-4.58 (m, 5H, C*H*₂Ph), 4.48 (d, *J* = 10.8 Hz, 1H, C*H*₂Ph), 4.47 (d, *J* = 12.4 Hz, 1H, C*H*₂Ph), 4.28 (dd, *J*_{2,3} = 3.3 Hz, *J*_{1,2} = 1.9 Hz, 1H, H-2_{B}), 4.14 (ddt, *J* = 12.9, 5.3, 1.5 Hz, 1H, H-1a_{All}), 4.03-3.95 (m, 4H, H-2_{E}, H-3_{C}, H-1b_{All}, H-5_{E}), 3.79-3.67 (m, 4H, H-5_{C}, H-6a_{E}, H-6b_{E}, H-3_{B}), 3.62 (t, *J*_{3,4} = *J*_{4,5} = 9.4 Hz, 1H, H-4_{E}), 3.61-3.51 (m, 3H, H-5_{B}, H-4_{C}, H-3_{E}), 3.45 (t, J_{3,4} = *J*_{4,5} = 8.9 Hz, 1H, H-4_{B}), 2.72-2.60 (high-order m, 4H, 2×C*H*_{2Lev}), 2.05 (C(O)C*H*₃), 1.34 (d, *J*_{5,6} = 6.3 Hz, 3H, C-6_{C}), 1.26 (d, *J*_{5,6} = 6.2 Hz, 3H, C-6_{B}). ¹³C NMR (CDCl₃, 100 MHz) δ: 206.1 (C(O)CH_{2Lev}), 172.3 (*C*=O), 138.9 (C_{Ph}), 138.8 (C_{Ph}), 138.5 (C_{Ph}), 138.4 (P_{Ph}), 138.3 (P_{Ph}), 138.0 (P_{Ph}), 133.8 (C-2_{All}), 129.2 (*C*H_{Ph}), 128.6-127.7 (*C*H_{Ph}), 125.4 (*C*H_{Ph}), 117.7 (C-3_{All}), 103.1 (C-1_{B}), 98.4 (C-1_{C}), 96.2 (C-1_{E}), 81.7 (C-5_{E}), 81.3 (C-3_{E}), 80.2 (C-4_{B}), 79.7 (C-4_{C}), 79.5 (C-3_{B}), 78.8 (C-3_{C}), 77.9 (C-4_{E}), 75.8 (*C*H₂Ph), 75.3 (*C*H₂Ph), 75.0 (*C*H₂Ph), 73.8 (*C*H₂Ph), 73.4 (*C*H₂Ph), 73.1 (C-2_{C}), 71.8 (*C*H₂Ph), 71.5 (C-2_{E}), 68.7 (C-6_{E}), 68.5 (C-1_{All}), 68.4 (C-5_{B}), 68.4 (C-2_{B}), 67.6 (C-5_{C}), 38.2 (*C*H_{2Lev}), 29.8 (C(O)*C*H₃), 28.4 (*C*H_{2Lev}), 18.7 (C-6_{C}), 18.0 (C-6_{B}). HR-ESI-TOF-MS *m*/*z* 1173.5118 [M + Na]⁺ (calcd for C₆₈H₇₈O₁₆Na, 1173.5188).

### Allyl (3,4-di-O-benzyl-α-L,-rhamnopyranosyl)-(1→3)-[2,3,4,6-tetra-O-benzyl-α-D-glucopyranosyl-(1→4)]-2-O-acetyl-α-L-rhamnopyranoside(47).

To a solution of **40** (500 mg, 0.40 mmol) in anhydrous THF (4.0 mL) was added dry PBu₃ (299 µL, 1.20 mmol, 3.0 equiv). The mixture was stirred for 30 min at room temperature, then H₂O (36 µL, 2.0 mmol, 5.0 equiv) was added and the mixture was stirred for 1 h at room temperature. The solvents were evaporated under reduced pressure and the resulting residue was purified by flash chromatography (Tol/EtOAc 95:5 to 8:2) to afford **47** (386 mg, 88%) as a colorless oil. *R_{f}* 0.52 (Chex/EtOAc 7:3); [α]²⁵_{D} +13° (c 1.0, CHCl₃); ¹H NMR (CDCl₃, 400 MHz) δ: 7.28-7.02 (m, 30H, C*H*_{Ph}), 5.82 (high-order m, 1H, H-2_{All}), 5.20 (ddd, *J* = 17.2, 3.1, 1.5 Hz, 1H, H-3a_{All}), 5.12 (ddd, *J*= 10.3, 2.6, 1.3 Hz, H-3b_{All}), 4.98 (dd, *J*_{2,3} = 3.4 Hz, *J*_{1,2} = 1.9 Hz, 1H, H-2_{C}), 4.95 (d, *J*_{1,2} = 3.1 Hz, 1H, H-1_{E}), 4.91 (d, *J*_{1,2} = 1.7 Hz, 1H, H-1_{B}), 4.86 (d, *J* = 11.1Hz, 1H, C*H*₂Ph), 4.77-4.72 (m, 3H, C*H*₂Ph), 4.68 (d, *J*= 11.7 Hz, 1H, C*H*₂Ph), 4.68 (d, *J*_{1,2} = 1.7 Hz, 1H, H-1_{C}), 4.60-4.49 (m, 5H, C*H*₂Ph), 4.40 (d, *J* = 10.8 Hz, 1H, C*H*₂Ph), 4.38 (d, *J* = 12.2 Hz, 1H, C*H*₂Ph), 4.21 (dd, *J*_{2,3} = 2.8 Hz, *J*_{1,2} = 2.1 Hz, 1H, H-2_{B}), 4.05 (ddt, *J* = 12.9, 5.3, 1.4 Hz, 1H, H-1a_{All}), 3.95-3.86 (m, 4H, H-2_{E}, H-3_{C}, H-1b_{All}, H-5_{E}), 3.71-3.46 (m, 7H, H-5_{C}, H-6a_{E}, H-6b_{E}, H-3_{B}, H-5_{B}, H-4_{E}, H-4_{C}), 3.43 (dd, *J* = 9.9, 3.2 Hz, 1H, H-3_{E}), 3.36 (t, *J*₃,₄ *= J*_{4,5} = 9.0 Hz, 1H, H-4_{B}), 2.62 (br s, 1H, O*H*), 2.00 (s, 3H, C(O)C*H*₃), 1.26 (d, *J*_{5,6} = 6.2 Hz, 3H, H-6_{C}), 1.16 (d, *J*_{5,6} = 6.2 Hz, 3H, H-6_{B}). ¹³C NMR (CDCl₃, 100 MHz) δ: 170.4 (*C*=O_{Ac}), 138.9-138.1 (P_{Ph}), 133.5 (C-2_{All}), 128.5-127.3 (*C*H_{Ph}), 117.7 (C-3_{All}), 102.9 (C-1_{B}), 98.1 (C-1_{E}), 96.0 (C-1_{C}), 81.5 (C-5_{E}), 81.1 (C-3_{E}), 80.0 (C-4_{B}), 79.3 (C-4_{C}), 79.2 (C-3_{B}), 78.7 (C-3_{C}), 77.6 (C-4_{E}), 75.6 (CH₂Ph), 75.1 (CH₂Ph), 74.9 (CH₂Ph), 73.6 (*C*H₂Ph), 73.1 (*C*H₂Ph), 72.7 (C-2_{C}), 71.5 (*C*H₂Ph), 71.2 (C-2_{E}), 68.4 (C-6_{E}), 68.3 (C-1_{All}), 68.1 (C-5_{B}, C-2_{B}), 67.3 (C-5_{C}), 21.1 (C(O)CH₃), 18.5 (C-6_{C}), 17.8 (C-6_{B}). HR-ESI-TOF-MS *m*/*z* 1095.5004 [M + H]⁺ (calcd for C₆₅H₇₅O₁₅, 1095.5106), *m*/*z* 1112.5289 [M + NH₄]⁺ (calcd for C₆₅H₇₄O₁₅NH₄, 1112.5371), *m*/*z* 1117.4888 [M + Na]⁺ (calcd for C₆₅H₇₄O₁₅Na, 1117.4926).

### Allyl (2-O-2-(azidomethyl)benzoyl-4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (44).

The acceptor **43** (5.36 g, 4.66 mmol) and the donor **12** (4.74 g, 6.99 mmol, 1.5 equiv) were dissolved in anhydrous Et₂O (70 mL) with 4 Å AW molecular sieves (7.0 g). The temperature was lowered to -10 °C (acetone/ice water) and the mixture was stirred for 30 min under an argon atmosphere. Then, TMSOTf (84 µL, 0.47 mmol, 0.1 equiv) was added keeping rigorously anhydrous conditions. The mixture was stirred for 1h at -10 °C to room temperature and the reaction was quenched by the addition of Et₃N (300 µL). The solution was filtered, the solvents were evaporated under reduced pressure and the resulting residue was purified by flash chromatography (Tol/EtOAc 95:5 to 9:1) to afford **44** (6.18 g, 80%) as a white sticky solid slightly contaminated with trichloroacetamide along with unreacted **43** (1.35 g, 20%). *R_{f}* 0.45 (Chex/EtOAc 7:3); [α]²⁵_{D} +10° (c 1.0, CHCl₃); ¹H NMR (CDCl₃, 400 MHz) δ: 7.94-7.91 (m, 1H, C*H*_{Ph}), 7.55-7.47 (m, 2H, C*H*_{Ph}), 7.36-7.02 (m, 38H, C*H*_{Ph}), 6.80-6.76 (m, 2H, C*H*_{Ph}), 5.88 (high-order m, 1H, H-2_{All}), 5.74 (dd, *J*_{2,3} = 3.2 Hz, *J*_{1,2} = 2.0 Hz, 1H, H-2_{A}), 5.27 (ddd, *J* = 17.3, 3.2, 1.5 Hz, 1H, H-3a_{All}), 5.19 (ddd, *J* = 10.4, 2.6, 1.2 Hz, 1H, H-3b_{All}), 5.19 (d, *J*_{1,2} = 1.7 Hz, 1H, H-1_{A}), 5.11 (dd, *J*_{2,3} = 3.5 Hz, *J*_{1,2} = 1.9 Hz, 1H, H-2_{C}), 4.98 (d, *J*_{1,2} = 3.2 Hz, 1H, H-1_{E}), 4.96 (d, J_{1,2} = 1.6 Hz, 1H, H-1_{B}), 4.93-4.89 (m, 3H, CH₂Ph), 4.81 (d, *J*= 10.9 Hz, 1H, C*H*₂Ph), 4.76-4.69 (m, 5H, C*H*₂N₃a, H-1_{C}, C*H*₂Ph (3H)), 4.67-4.44 (m, 8H, C*H*₂Ph (7H), C*H*₂N₃b), 4.44 (m, 1H, H-2_{B}), 4.38 (d, *J* = 10.8 Hz, 1H, C*H*₂Ph), 4.28 (d, *J* = 12.1 Hz, 1H, C*H*₂Ph), 4.11 (ddt, *J*= 13.0, 5.3, 1.5 Hz, 1H, H-1a_{All}), 4.05 (dd, *J*_{3,4} = 9.4 Hz, *J*_{2,3} = 3.1 Hz, 1H, H-3_{A}), 4.01-3.90 (m, 5H, H-1b_{All,} H-5_{A}, H-2_{E}, H-3_{C}, H-5_{E}), 3.79-3.67 (m, 7H, OC*H*₃, H-5_{C}, H-6a_{E}, H-6b_{E}, H-3_{B}), 3.64 (t, *J*_{3,4} = *J*_{4,5} = 9.6 Hz, 1H, H-4_{E}), 3.60-3.49 (m, 3H, H-4_{A}, H-5_{B}, H-4_{C}), 3.47-3.38 (m, 2H, H-3_{E}, H-4_{B}), 2.74-2.54 (high-order m, 4H, 2×C*H*_{2Lev}), 2.08 (s, 3H, C(O)C*H*₃), 1.36 (d, *J*_{5,6} = 6.2 Hz, 3H, H-6_{A}), 1.30 (d, *J*_{5,6} = 6.3 Hz, 3H, H-6_{C}), 1.20 (d, *J*_{5,6} = 6.2 Hz, 3H, H-6_{B}). ¹³C NMR (CDCl₃, 100 MHz) δ: 206.3 (*C*(O)CH_{2Lev}), 172.3 (*C*=O), 165.5 (*C*=O), 159.3 (C_{Ph}), 138.8 (C_{Ph}), 138.8 (C_{Ph}), 138.7 (C_{Ph}), 138.6 (C_{Ph}), 138.3 (C_{Ph}), 138.3 (C_{Ph}), 138.1 (C_{Ph}), 137.7 (*C*_{Ph}), 133.7 (C-2_{All}), 132.9 (*C*H_{Ph}), 131.4 (*C*H_{Ph}), 130.2 (*C*H_{Ph}), 129.8 (*C*H_{Ph}), 129.5 (*C*H_{Ph}), 128.7-127.4 (*C*H_{Ph}), 117.7 (C-3_{All}), 113.8 (*C*H_{Ph}), 101.4 (C-1_{B}), 99.3 (C-1_{A}), 97.8 (C-1_{E}), 95.9 (C-1_{C}), 81.8 (C-5_{E}), 81.3 (C-3_{E}), 80.3 (C-4_{C}), 80.1 (C-4_{B}), 79.9 (C-3_{C}), 79.1 (C-3_{B}), 77.7 (C-4_{A}), 77.4 (C-3_{A}, C-4_{E}), 75.7 (CH₂Ph), 75.4 (CH₂Ph), 75.1 (*C*H₂Ph), 74.8 (C-2_{B}), 73.9 (*C*H₂Ph), 73.0 (*C*H₂Ph), 72.5 (C-2_{C}), 71.4 (*C*H₂Ph), 71.4 (C-5_{A}), 71.0 (*C*H₂Ph), 70.1 (C-2_{A}), 68.9 (C-5_{B}), 68.5 (C-1_{All}), 68.4 (C-2_{E}), 68.3 (C-6_{E}), 67.6 (C-5_{C}), 55.3 (OCH₃), 53.1 (*C*H₂N₃), 38.1 (*C*H_{2Lev}), 31.0 (C(O)CH₃), 28.3 (*C*H_{2Lev}), 18.7 (C-6_{C}), 18.6 (C-6_{A}), 18.2 (C-6_{B}). HR-ESI-TOF-MS *m*/*z* 1688.7185 [M + Na]⁺ (calcd for C₉₇H₁₀₇N₃O₂₂Na, 1688.7244).

### Allyl (4-O-benzyl-2-O-chloroacetyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (52).

The acceptor **43** (769 mg, 668 µmol) and the donor **13** (791 mg, 1.34 mmol, 2.0 equiv) were dissolved in anhydrous Et₂O (10 mL) in the presence of 4 Å AW molecular sieves. The temperature was lowered to -10 °C (acetone/ice water) and the mixture was stirred for 30 min under an argon atmosphere. Then, TMSOTf (12 µL, 67 µmol, 0.1 equiv) was added keeping rigorously anhydrous conditions. The mixture was stirred for 1h at -10 °C to room temperature and the reaction was quenched by the addition of Et₃N (100 µL). The solution was filtered, the solvents were evaporated under reduced pressure and the resulting residue was purified by flash chromatography (Tol/EtOAc 95:5 to 8:2) to afford **52** (1.03 g, 98%) as a white sticky solid slightly contaminated with trichloroacetamide. *R*_{f} 0.46 (Chex/EtOAc 7:3); [α]²⁵_{D} -2° (c 1.0, CHCl₃); ¹H NMR (CDCl₃, 400 MHz) δ: 7.38-7.09 (m, 37H, C*H*_{Ph}), 6.84-6.80 (m, 2H, C*H*_{PH}), 5.89 (high-order m, 1H, H-2_{All}), 5.57 (dd, *J*_{2,3} = 2.9 Hz, *J*_{1,2} = 1.8 Hz, 1H, H-2_{A}), 5.29 (ddd, *J =* 17.3, 3.1, 1.5 Hz, 1H, H-3a_{All}), 5.20 (ddd, *J =* 10.4, 2.6, 1.3 Hz, 1H, H-3b_{All}), 5.11 (dd, *J*_{2,3} = 3.0 Hz, *J*_{1,2} = 2.1Hz, 1H, H-2_{C}), 5.06 (d, *J*_{1,2} = 1.3 Hz, 1H, H-1_{A}), 5.01 (d, *J*_{1,2} = 3.1Hz, 1H, H-1_{E}), 4.95 (br s, 1H, H-1_{B}), 4.94 (d, *J* = 11.1 Hz, 1H, C*H*₂Ph), 4.89 (d, *J* = 11.0 Hz, 1H, C*H*₂Ph), 4.88 (d, *J* = 10.9 Hz, C*H*₂Ph), 4.85-4.74 (m, 4H, C*H*₂Ph (3H), H-1_{C}), 4.67-4.52 (m, 7H, C*H*₂Ph), 4.47 (d, *J* = 11.0 Hz, 1H, C*H*₂Ph), 4.45 (d, *J* = 11.0 Hz, 1H, C*H*₂Ph), 4.40 (t, *J*_{2,3} = *J*_{1,2} = 2.0 Hz, 1H, H-2_{B}), 4.36 (d, *J* = 12.0 Hz, 1H, C*H*₂Ph), 4.13 (ddt, *J* = 12.8, 5.3, 1.6 Hz, 1H, H-1a_{All}), 4.04-3.90 (m, 7H, C*H*₂Cla, H-5_{A}, H-1b_{All}, H-5_{E}, H-3_{C}, H-3_{A}, H-2_{E}), 3.84 (d, *J* = 15.0 Hz, 1H, C*H*₂Clb), 3.81-3.66 (m, 8H, H-6a_{E}, H-6b_{E}, H-4_{E}, H-3_{B}, H-5_{C}, OC*H*₃), 3.62-3.53 (m, 2H, H-4_{A}, H-5_{B}), 3.49 (dd, *J* = 9.7, 3.3 Hz, 1H, H-3_{E}), 3.37 (t, *J*_{3,4} = *J*_{4,5} = 9.5 Hz, 2H, H-4_{B}, H-4_{C}), 2.77-2.53 (high-order m, 4H, 2×C*H*_{2Lev}), 2.08 (s, 3H, C(O)C*H*₃), 1.33 (d, *J*_{5,6} = 6.1 Hz, 6H, H-6_{A}, H-6_{C}), 1.20 (d, *J*_{5,6} = 6.1 Hz, 3H, H-6_{B}). ¹³C NMR (CDCl₃, 100 MHz) δ: 206.2 (*C*(O)CH_{2Lev}), 172.3 (*C*=O_{Lev}), 166.5 (*C*=O_{AcCl}), 159.3 (C_{Ph}), 138.8-137.9 (C_{Ph}), 133.7 (C-2_{All}), 130.1-125.4 (*C*H_{Ph}), 117.8 (C-3_{All}), 113.9 (*C*H_{Ph}), 101.4 (C-1_{B}), 99.0 (C-1_{A}), 97.7 (C-1_{E}), 95.9 (C-1_{C}), 81.8 (C-5_{E}), 81.1 (C-3_{E}), 80.0 (C-4_{B}*), 79.9 (C-4_{C}*), 79.7 (C-3_{C}), 79.0 (C-3_{B}), 77.6 (C-4_{A}), 77.4 (C-4_{E}), 77.2 (C-3_{A}), 75.7 (*C*H₂Ph), 75.4 (*C*H₂Ph), 75.3 (*C*H₂Ph), 75.1 (*C*H₂Ph), 74.8 (C-2_{B}), 73.9 (*C*H₂Ph), 72.9 (*C*H₂Ph), 72.5 (C-2_{C}), 71.7 (*C*H₂Ph), 71.3 (C-5_{A}), 71.1 (C-2_{A}), 70.9 (CH₂Ph), 68.8 (C-5_{B}), 68.5 (C-1_{All}), 68.4 (C-2_{E}), 68.2 (C-6_{E}), 67.5 (C-5_{C}), 55.3 (OCH₃), 41.0 (CH₂Cl), 38.0 (CH_{2Lev}), 29.9 (C(O)CH₃), 28.2 (CH_{2Lev}), 18.7 (C-6_{A}*), 18.4 (C-6_{C}*), 18.1 (C-6_{B}). HR-ESI-TOF-MS *m*/*z* 1583.6742 [M + H]⁺ (calcd for C₉₁H₁₀₄ClO₂₂, 1583.6708), *m*/*z* 1605.6541 [M + Na]⁺ (calcd for C₉₁H₁₀₃ClO₂₂Na, 1605.6527).

### Allyl (4-O-benzyl-2-O-levulinoyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-acetyl-α-L-rhamnopyranoside (48).

The acceptor **47** (912 mg, 833 µmol) and the donor **15** (1.03 g, 1.67 mmol, 2.0 equiv) were dissolved in anhydrous DCE (12 mL) in the presence of 4 Å powdered molecular sieves. The temperature was lowered to -10 °C (acetone/ice water) and the mixture was stirred for 30 min under an argon atmosphere. Then, cold TMSOTf (15 µL, 83 µmol, 0.1 equiv) was added keeping rigorously anhydrous conditions. The mixture was stirred for 2 h at -10 °C to room temperature and the reaction was quenched by the addition of Et₃N (100 µL). The solution was filtered, the solvents were evaporated under reduced pressure and the resulting residue was purified by flash chromatography (Chex/EtOAc 95:5 to 8:2) to afford **48** (983 mg, 76%) as a white sticky solid slightly contaminated with trichloroacetamide. *R_{f}* 0.47 (Chex/EtOAc 7:3); [α]²⁵_{D}-4° (c 0.5, CHCl₃); ¹H NMR (CDCl₃, 400 MHz) δ: 7.25-6.94 (m, 37H, C*H*_{PH}), 6.70-6.66 (m, 2H, C*H*_{Ph}), 5.74 (high-order m, 1H, H-2_{All}), 5.38 (dd, *J*_{2,3} = 3.0 Hz, *J*_{1,2} = 1.9 Hz, 1H, H-2_{A}), 5.14 *(ddd, J =* 17.2, 3.0, 1.4 Hz, 1H, H-3a_{All}), 5.05 (ddd, *J=* 10.3, 2.5, 1.3 Hz, 1H, H-3b_{All}), 4.95 (dd, *J*_{2,3} = 3.1 Hz, *J*_{1,2} = 2.0 Hz, 1H, H-2_{C}), 4.90 (d, *J*_{1,2} = 1.4 Hz, 1H, H-1_{A}), 4.80-4.74 (m, 5H, H-1_{E}, C*H*₂Ph (3H), H-1_{B}), 4.68 (d, *J* = 11.0 Hz, 1H, C*H*₂Ph), 4.66 (d, *J* = 10.8 Hz, 1H, C*H*₂Ph), 4.63 (d, *J*_{1,2} = 1.6 Hz, 1H, H-1_{C}), 4.57 *(d, J=* 11.6 Hz, 1H, C*H*₂Ph), 4.51-4.37 (m, 7H, C*H*₂Ph), 4.35-4.28 (m, 3H, C*H*₂Ph, H-2_{B}), 4.20 (d, *J =* 12.0 Hz, 1H, C*H*₂Ph), 3.97 (ddt, *J =* 12.8, 5.2, 1.3 Hz, 1H, H-1a_{All}), 3.85-3.75 (m, 6H, H-1b_{All}, H-5_{A}, H-5_{E}, H-3_{A}, H-3_{C}, H-2_{E}), 3.69-3.52 (m, 8H, H-6a_{E}, H-6b_{E}, OC*H*₃, H-5_{C}, H-3_{B}, H-4_{E}), 3.51-3.42 (high-order m, 1H, H-5_{B}), 3.37 (t, *J*_{3,4} = *J*_{4,5} = 9.0 Hz, 1H, H-4_{A}), 3.29-3.24 (m, 3H, H-3_{E}, H-4_{B}, H-4_{C}), 2.52-2.30 (high-order m, 4H, 2×C*H*_{2Lev}), 1.94 (s, 3H, C(O)C*H*_{3Lev}*), 1.93 (s, 3H, C(O)C*H*_{3Ac}*), 1.19 (d, *J*_{5,6} = 6.1 Hz, 3H, H-6_{A}*), 1.16 (d, *J*_{5,6} = 6.3 Hz, 3H, H-6_{C}*), 1.05 (d, *J*_{5,6} = 6.3 Hz, 3H, H-6_{B}). ¹³C NMR (CDCl₃, 100 MHz) δ: 206.3 (*C*(O)CH_{2Lev}), 171.8 (*C*=O), 163.6 (*C*=O), 159.2 (C_{Ph}), 138.8-138.3 (C_{Ph}), 133.7 (C-2_{All}), 130.0-127.3 (*C*H_{Ph}), 117.8 (C-3_{All}), 113.8 (*C*H_{Ph}), 101.1 (C-1_{B}), 99.3 (C-1_{A}), 97.9 (C-1_{E}), 95.9 (C-1_{C}), 81.8 (C-5_{E}), 81.4 (C-3_{E}), 80.1 (C-4_{B}, C-4_{B}), 79.9 (C-3_{C}), 78.7 (C-3_{B}), 78.1 (C-4_{A}), 77.5 (C-4_{E}), 77.2 (C-3_{A}), 75.7 (*C*H₂Ph), 75.5 (*C*H₂Ph), 75.4 (*C*H₂Ph), 75.1 (*C*H₂Ph), 74.3 (C-2_{B}), 73.9 (*C*H₂Ph), 72.9 (*C*H₂Ph), 72.0 (C-2_{C}), 71.4 (C-5_{A}), 71.3 (*C*H₂Ph), 70.3 (*C*H₂Ph), 69.5 (C-2_{A}), 68.9 (C-5_{B}), 68.5 (C-1_{All}), 68.3 (C-2_{E}), 68.3 (C-6_{E}), 67.4 (C-5c), 55.3 (OCH₃), 38.2 (*C*H_{2Lev}), 29.9 (C(O)*C*H_{3Lev}), 28.2 (*C*H_{2Lev}), 21.3 (C(O)*C*H_{3Ac}), 18.6 (C-6_{A}*), 18.4 (C-6_{C}*), 18.1 (C-6_{B}). HR-ESI-TOF-MS *mlz* 1549.7200 [M + H]⁺ (calcd for C₉₁H₁₀₅O₂₂, 1549.7097), *mlz* 1571.7025 [M + Na]⁺ (calcd for C₉₁H₁₀₄O₂₂Na, 1571.6917).

### Allyl (4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-acetyl-α-L-rhamnopyranoside (8).

To a solution of **48** (756 mg, 0.488 mmol) in anhydrous Py/HOAc (73 mL, 3:2 *v*/*v*) was added hydrazine hydrate (76 µL, 2.4 mmol, 5.0 equiv). The mixture was stirred for 1 h at room temperature, then the solvents were evaporated under reduced pressure and co-evaporated three times with toluene. The resulting residue was purified by flash chromatography (Chex/EtOAc 9:1 to 7:3) to give 8 (605 mg, 85%) as a colorless sticky solid. *R*_{f} 0.58 (Chex/EtOAc 7:3); [α]²⁵_{D} -4° (c 1.0, CHCl₃); ¹H NMR (CDCl₃, 400 MHz) δ: 7.22-6.98 (m, 37H, C*H*_{Ph}), 6.73-6.68 (m, 2H, C*H*_{Ph}), 5.74 (high-order m, 1H, H-2_{All}), 5.13 (ddd, *J* = 17.2,3.1,1.5 Hz, 1H, H-3a_{All}), 5.05 (ddd, *J* = 10.4, 2.7, 1.3 Hz, 1H, H-3b_{All}), 4.96 (dd, *J*_{2,3} = 3.3 Hz, *J*_{1,2} = 2.0 Hz, 1H, H-2_{C}), 4.94 (d, *J*_{1,2} = 1.1 Hz, 1H, H-1_{A}), 4.82-4.67 (m, 7H, C*H*₂Ph (5H), H-1_{E}, H-1_{B}), 4.63 (d, *J*_{1,2} = 1.7 Hz, 1H, H-1_{C}), 4.56 (d, *J* = 11.7 Hz, 1H, C*H*₂Ph), 4.51-4.24 (m, 11H, C*H*₂Ph (10H), H-2_{B}), 3.98 (ddt, *J* = 12.9,5.3,1.4 Hz, 1H, H-1a_{All}), 3.94 (dd, *J*_{2,3} = 3.0 Hz, *J*_{1,2} = 1.8 Hz, 1H, H-2_{A}), 3.87-3.74 (m, 5H, H-1b_{All} H-5_{A}, H-5_{E}, H-3_{C}, H-2_{E}), 3.73-3.52 (m, 9H, H-3_{A}, H-6a_{E}, H-6b_{E}, H-4_{E}, H-5_{C}, H-3_{B}, OC*H*₃), 3.52-3.44 (high-order m, 1H, H-5_{B}), 3.40 (t, *J*_{3,4} = *J*_{4,5} = 9.0 Hz, 1H, H-4_{A}), 3.35-3.27 (m, 2H, H-4_{C} H-3_{E}), 3.20 (t, *J*_{3,4} = *J*_{4,5} = 9.4 Hz, 1H, H-4_{B}), 2.19 (br s, 1H, O*H*), 1.92 (s, 3H, C(O)C*H*₃), 1.17 (d, *J*_{5,6} = 6.1 Hz, 3H, H-6_{A}*), 1.16 (d, *J*_{5,6} = 6.0 Hz, 3H, H-6_{C}*), 1.07 (d, *J*_{5,6} = 6.2 Hz, 3H, H-6_{B}). ¹³C NMR (CDCl₃, 100 MHz) δ: 163.5 (*C*=O), 159.4 (P_{Ph}), 138.8-138.3 (P_{Ph}), 133.6 (C-2_{All}), 129.8-127.4 (*C*H_{Ph}), 117.7 (C-3_{All}), 114.0 (*C*H_{Ph}), 101.3 (C-1_{B})_{,} 101.0 (C-1_{A}), 97.8 (C-1_{E}), 95.9 (C-1_{C}), 81.8 (C-5_{E}), 81.4 (C-3_{E}), 80.2 (C-4_{A}, C-4_{B}), 79.9 (C-3_{C}), 79.1 (C-3_{A}), 78.7 (C-3_{B}), 77.9 (C-4_{A}), 77.5 (C-4_{E}), 75.7 (*C*H₂Ph), 75.5 (*C*H₂Ph), 75.4 (*C*H₂Ph), 75.2 (*C*H₂Ph), 74.4 (C-2_{B}), 73.9 (*C*H₂Ph), 73.1 (*C*H₂Ph), 72.1 (C-2_{C}), 71.8 (*C*H₂Ph), 71.3 (C-5_{A}), 70.2 (*C*H₂Ph), 68.9 (C-5_{B}), 68.8 (C-2_{A}), 68.5 (C-1_{All}), 68.2 (C-6_{E}), 68.0 (C-2_{E}), 67.4 (C-5_{C}), 55.3 (OCH₃), 21.3 (C(O)CH₃), 18.7 (C-6_{A}*), 18.3 (C-6_{C}*), 18.1 (C-6_{B}). HR-ESI-TOF-MS *m*/*z* 1473.6768 [M + Na]⁺ (calcd for C₈₆H₉₈O₂₀Na, 1473.6549).

### Allyl (4-O-benzyl-3-O-para-methoxybenzyl-α-1,-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (7).

**Route a.** To a solution of **44** (6.15 g, 3.69 mmol) in THF/H₂O (36.9 mL, 9:1 v/v) was added PPh₃ (4.84 g, 18.4 mmol, 5.0 equiv) and SiO₂ (1.54 g). The mixture was stirred for 24 h at room temperature, then additional portions of PPh₃ (4.84 g, 18.4 mmol, 5.0 equiv) and SiO₂ (1.54 g) were added. The mixture was stirred overnight at room temperature, filtered, washed with several portions of CH₂Cl₂ and the solvents were removed under reduced pressure. The resulting residue was purified by flash chromatography (Tol/EtOAc 95:5 to 8:2) to afford 7 (3.39 g, 61 %) as a white foam along with unreacted **44** (476 mg, 8%).

**Route b**. To a solution of **52** (187 mg, 0.118 mmol) in anhydrous MeOH/Py (11.8 mL, 1:1 v/v) was added thiourea (90 mg, 1.2 mmol, 10 equiv). The mixture was stirred for 2 h at 60 °C, then the solvents were evaporated under reduced pressure. The residue was taken off in CH₂Cl₂/MeOH (50 mL, 2:1 *v*/*v*) and washed with 10% aqueous HCl (1×25 mL). The aqueous phase was extracted with CH₂Cl₂ (2x25 mL), then the pooled organic phases were washed with saturated NaHCO₃ (1×25 mL) and brine (1×25 mL). The solvents of the dried (anhydrous Na₂SO₄) solution were evaporated under reduced pressure and the resulting residue was purified by flash chromatography (Tol/EtOAc 9:1 to 7:3) to give 7 (149 mg, 84%) as a colorless oil. *R*_{f} 0.40 (Chex/EtOAc 7:3); [α]²⁵_{D} +6° (c 1.0, CHCl₃); ¹H NMR (CDCl₃, 400 MHz) δ: 7.28-7.03 (m, 37H, C*H*_{Ph}), 6.79-6.74 (m, 2H, C*H*_{Ph}), 5.80 (high-order m, 1H, H-2_{All}), 5.19 (ddd, *J* = 17.2, 2.9, 1.5 Hz, 1H, H-3a_{All}), 5.11 (br dd, *J* = 10.3, 1.2 Hz, 1H, H-3b_{All}), 5.03-4.99 (m, 2H, H-2_{C}, H-1_{A}), 4.90 (m, 2H, H-1_{E}, H-1_{B}), 4.87-4.73 (m, 5H, C*H*₂Ph), 4.65 (d, *J* = 11.6 Hz, 1H, C*H*₂Ph), 4.65 (d, *J*_{1,2} = 1.7 Hz, 1H, H-1_{C}), 4.57-4.48 (m, 6H, C*H*₂Ph), 4.42-4.38 (m, 3H, C*H*₂Ph), 4.33 (d, *J* = 11.9 Hz, 1H, C*H*₂Ph), 4.30 (br s, 1H, H-2_{B}), 4.04 (ddt, *J* = 12.8, 5.2, 1.4 Hz, 1H, H-1a_{All}), 3.98 (m, 1H, H-2_{A}), 3.96-3.80 (m, 5H, H-5_{A}, H-1b_{All,} H-5_{E}, H-3_{C}, H-2_{E}), 3.78-3.69 (m, 3H, H-3_{A}, H-6a_{E}, H-6b_{E}), 3.68-3.58 (m, 6H, OC*H*₃, H-5_{C}, H-4_{E}, H-3_{B}), 3.52-3.43 (m, 2H, H-4_{A}, H-5_{B}), 3.41-3.35 (m, 2H, H-4_{C}, H-5_{E}), 3.25 (t, *J*_{3,4} = *J*_{4,5} = 9.4 Hz, 1H, H-4_{B}), 2.64-2.45 (m, 4H, 2×C*H*_{2Lev}), 1.97 (s, 3H, C(O)C*H*₃), 1.23 (d, *J*_{5,6} = 6.1 Hz, 3H, H-6_{A}), 1.22 (d, *J*_{5,6} = 6.1 Hz, 3H, H-6_{C}), 1.12 (d, *J*_{5,6} = 6.2 Hz, 3H, C-6_{B}). ¹³C NMR (CDCl₃, 100 MHz) δ: 206.0 (*C*(O)CH_{2Lev}), 172.2 (*C*=O), 159.5 (C_{Ph}), 138.9-138.0 (C_{Ph}), 133.8 (C-2_{All}), 131.0-125.4 (*C*H_{Ph}), 117.6 (C-3_{All}), 114.0 (*C*H_{Ph}), 101.6 (C-1_{B}), 101.1 (C-1_{A}), 97.7 (C-1_{E}), 96.0 (C-1_{C}), 81.9 (C-5_{E}), 81.3 (C-3_{E}), 80.3 (C-4_{C}, C-4_{B}), 79.7 (C-3_{C}), 79.3 (C-3_{A}), 78.9 (C-3_{B}), 75.7 (*C*H₂Ph), 75.4 (*C*H₂Ph), 75.3 (*C*H₂Ph), 75.3 (*C*H₂Ph), 74.9 (C-2_{B}), 73.9 (*C*H₂Ph), 73.2 (*C*H₂Ph), 72.6 (C-2_{C}), 71.8 (*C*H₂Ph), 71.4 (C-5_{A}), 70.7 (*C*H₂Ph), 69.0 (C-2_{A}), 68.8 (C-5_{B}), 68.5 (C-1_{All}), 68.4 (C-6_{E}), 68.1 (C-2_{E}), 67.6 (C-5_{C}), 55.3 (OCH₃), 38.1 (CH_{2Lev}), 29.8 (C(O)CH₃), 28.3 (*C*H_{2Lev}), 18.8 (C-6_{C}), 18.4 (C-6_{A}), 18.2 (C-6_{B}). HR-ESI-TOF-MS *m*/*z* 1507.6835 [M + H]⁺ (calcd for C₈₉H₁₀₂O₂₁, 1507.6992).

### Allyl (4-O-benzyl-3-O-para-methoxybenzyl-2-O-(1-O-(triphenylphosphonium)isoindol-1-yl)-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (45).

Eluting the chromatographic column of the precedent reaction (route a) using CH₂Cl₂/MeOH 95:5 to 100% MeOH afforded **45** (632 mg, 9%) as a white foam. *R*_{f} 0.16 (CH₂Cl₂/MeOH 95:5); [α]²⁵_{D} +15° (c 1.0, CHCl₃); ¹H NMR (CDCl₃, 400 MHz) δ: 9.51 (dd, *J* = 17.7, 7.4 Hz, 1H, N*H*), 8.16-8.12 (m, 1H, C*H*_{Ph}), 7.82-7.03 (m, 55H, C*H*_{Ph}), 6.79-6.75 (m, 2H, C*H*_{Ph}), 5.89 (high-order m, 1H, H-2_{All}), 5.54 (dd, *J*_{2,3} = 3.3 Hz, *J*_{1,2} = 2.0 Hz, 1H, H-2_{A}), 5.29 (ddd, *J =* 17.2, 3.0, 1.5 Hz, 1H, H-3a_{All}), 5.20 (ddd, *J* = 10.4, 2.6, 1.3 Hz, 1H, H-3b_{All}), 5.13-5.10 (m, 2H, H-2_{C}, H-1_{A}), 5.01-4.72 (m, 11H, C*H*₂NH, H-1_{E}, H-1_{B}, H-1_{C}, C*H*₂Ph (6H)), 4.66-4.37 (m, 10H, C*H*₂Ph (9H), H-2_{B}), 4.29 (d, *J* = 11.8 Hz, 1H, C*H*₂Ph), 4.13 (ddt, *J* = 12.9, 5.3, 1.4 Hz, 1H, H-1aₐₗₗ), 4.02-3.91 (m, 6H, H-5_{A}, H-1b_{All}, H-3_{A}, H-3_{C}, H-5_{E}, H-2_{E}), 3.81-3.69 (m, 7H, OC*H*₃, H-5_{C}, H-6a_{E}, H-6b_{E}, H-3_{B}), 3.68-3.52 (m, 3H, H-4_{E}, H-4_{A}, H-5_{B}), 3.50-3.42 (m, 2H, H-4_{C}, H-3_{E}), 3.67 (t, *J*_{3,4} = *J*₄,₅ = 9.3 Hz, 1H, H-4_{B}), 2.76-2.54 (high-order m, 4H, 2×C*H*_{2Lev}), 2.08 (s, 3H, C(O)C*H*₃), 1.35 (d, *J_{5,6}=* 6.2 Hz, 3H, C-6_{A}), 1.33 (d, *J*_{5,6} = 6.2 Hz, 3H, C-6_{C}), 1.20 (d, *J*_{5,6} = 6.2 Hz, 3H, C-6_{B}). ¹³C NMR (CDCl₃, 100 MHz) δ: 205.9 (*C*(O)CH_{2Lev}), 172.2 (*C*=O), 165.5 (*C*OPPh₃⁺), 159.3 (C_{Ph}), 141.4 (C_{Ph}), 141.3 (C_{Ph}), 138.8 (C_{Ph}), 138.8 (C_{Ph}), 138.6 (C_{Ph}), 138.6 (C_{Ph}), 138.4 (C_{Ph}), 138.3 (C_{Ph}), 138.0 (C_{Ph}), 134.6 (C-2_{All}), 133.7-126.9 (CH_{Ph}), 117.6 (C-3_{All}), 113.9 (*C*H_{Ph}), 101.3 (C-1_{B}), 99.1 (C-1_{A}), 97.8 (C-1_{E}), 96.0 (C-1_{C}), 81.8 (C-5_{E}), 81.3 (C-3_{E}), 80.3 (C-4_{C}), 80.1 (C-4_{B}), 79.8 (C-3_{C}), 79.1 (C-3_{B}), 78.0 (C-4_{A}), 77.8 (C-4_{E}), 77.0 (C-3_{A}), 75.7 (*C*H₂Ph), 75.2 (2×*C*H₂Ph), 75.1 (*C*H₂Ph), 74.4 (C-2_{B}), 73.9 (*C*H₂Ph), 73.0 (*C*H₂Ph), 72.5 (C-2_{C}), 71.4 (C-5_{A}), 71.2 (CH₂Ph), 70.9 (*C*H₂Ph), 69.9 (C-2_{A}), 68.9 (C-5_{B}), 68.5 (C-1_{All}), 68.5 (C-2_{E}), 68.3 (C-6_{E}), 67.6 (C-5_{C}), 55.4 (OCH₃), 43.2 (*C*H₂NH), 38.1 (*C*H_{2Lev}), 29.8 (C(O)CH₃), 28.3 (*C*H_{2Lev}), 18.7 (C-6_{A}), 18.6 (C-6_{C}), 18.1 (C-6_{B}). HR-ESI-TOF-MS *m*/*z* 1900.8340 [M]⁺ (calcd for C₁₁₅H₁₂₃NO₂₂P, 1900.8274).

### Allyl (4-O-benzyl-3-O-para-methoxybenzyl-2-O-(1-O-(tributylphosphonium)isoindol-1-yl)-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (46).

To a solution of **44** (500 mg, 0.30 mmol) in anhydrous THF (10 mL) was added dry PBu₃ (225 µL, 0.90 mmol, 3.0 equiv). The mixture was stirred for 2 h at room temperature, then H₂O (27 µL, 1.5 mmol, 5.0 equiv) was added. The mixture was stirred overnight at room temperature and the solvents were removed under reduced pressure. The resulting residue was purified by flash chromatography (CH₂Cl₂/MeOH 95:5 to 9:1) to furnish **46** (245 mg, 49%) as a white foam. *R*_{f} 0.15 (CH₂Cl₂/MeOH 95:5); [α]²⁵_{D} +13° (c 1.0, CHCl₃); ¹H NMR (Pyr-d₅, 400 MHz) δ: 9.41 (dd, *J* = 15.2, 7.6 Hz, 1H, N*H*), 8.34-8.30 (m, 1H, C*H*_{Ph}), 7.63-7.20 (m, 40H, C*H*_{Ph}), 7.04-6.99 (m, 2H, C*H*_{Ph}), 6.28 (br s, 1H, H-2_{A}), 5.85 (high-order m, 1H, H-2_{All}), 5.81 (br s, 1H, H-1_{A}), 5.71 (br s, 1H, H-2_{C}), 5.64 (br s, 1H, H-1_{B}), 5.49 (d, *J*_{1,2} = 2.9 Hz, 1H, H-1_{E}), 5.28-4.63 (m, 22H, 8×C*H*₂Ph, H-3a_{All}, H-1_{C}, H-3b_{All}, H-2_{B}, C*H*₂NHa, C*H*₂NHb), 4.57 (dd, *J*_{3,4} = 8.9 Hz, *J*_{2,3} = 2.4 Hz, 1H, H-3_{C}), 4.49 (dd, *J*_{3,4} *=* 9.3 Hz, *J*_{2,3} = 2.9 Hz, 1H, H-3_{A}), 4.46-4.38 (m, 2H, H-5_{A}, H-5_{C}), 4.36-4.27 (m, 2H, H-3_{E}, H-3_{B}), 4.22-4.04 (m, 6H, H-1a_{All}, H-6a_{E}, H-4_{C}, H-5_{B}, H-5_{E}, H-6b_{E}), 4.03-3.90 (m, 4H, H-1b_{All} H-4_{E}, H-4_{A}, H-4_{B}), 3.82 (dd, *J*_{2,3} = 9.8 Hz, *J*_{1,2} = 3.0 Hz, 1H, H-2_{E}), 3.66 (s, 3H, OC*H*₃), 2.82-2.69 (high-order m, 4H, 2×C*H*_{2Lev}), 2.41 (dd, *J* = 16.7, 12.6 Hz, 6H, 3×C*H*_{2Bu}), 2.03 (s, 3H, C(O)C*H*₃), 1.67-1.47 (m, 15H, 3×C*H*_{3Rha}, 3×C*H*₂ᵤ), 1.37-1.26 (high-order m, 6H, 3×C*H*_{2Bu}), 0.80 (t, *J*= 7.2 Hz, 9H, 3×C*H*_{3Bu}). ¹³C NMR (Pyr-d₅, 100 MHz) δ: 206.1 (C(O)CH_{2Lev}), 173.1 (*C*=O), 167.1 (*C*OPBu₃⁺), 160.2 (P_{Ph}), 143.0-139.4 (C_{Ph}), 134.7 (C-2_{All}), 133.9-128.2 (*C*H_{Ph}), 117.7 (C-3_{All}), 114.8 (*C*H_{Ph}), 102.4 (C-1_{B}), 100.1 (C-1_{A}), 98.6 (C-1_{E}), 96.8 (C-1_{C}), 82.6 (C-3_{E}), 82.3 (C-2_{E}), 81.2 (C-4_{A}), 80.9 (C-4_{B}), 80.7 (C-3_{C}), 80.2 (C-3_{B}), 79.0 (C-4_{C}), 78.8 (C-4_{E}), 78.3 (C-3_{A}), 76.1 (*C*H₂Ph), 75.8 (*C*H₂Ph), 75.6 (*C*H₂Ph), 75.5 (*C*H₂Ph), 75.5 (C-2_{B}), 74.3 (*C*H₂Ph), 73.7 (*C*H₂Ph), 73.4 (C-2_{C}), 72.4 (C-5_{A}), 72.0 (*C*H₂Ph), 71.8 (*C*H₂Ph), 71.1 (C-2_{A}), 69.8 (C-5_{B}), 69.7 (C-6_{E}), 69.4 (C-5_{C}), 68.8 (C-1_{All}), 68.6 (C-5_{E}), 55.7 (OCH₃), 42.4 (CH₂NH), 38.5 (*C*H_{2Lev}), 29.9 (C(O)CH₃), 29.1 (*C*H_{2Lev}), 24.5 (*C*H_{2Bu}), 24.4 (*C*H_{2Bu}), 23.8 (*C*H_{2Bu}), 22.6 (*C*H_{2Bu}), 22.0 (*C*H_{2Bu}), 19.7 (*C*H_{3Rha}), 19.0 (*C*H_{3Rha}), 19.0 (*C*H_{3Rha}), 13.9 (*C*H_{3Bu}). HR-ESI-TOF-MS *m*/*z* 1840.9080 [M]⁺ (calcd for C₁₀₉H₁₃₅NO₂₂P, 1841.9247).

### Allyl (3-O-tert-butyldimethylsilyl-2-deoxy-4,6-O-isopropylidene-2-trichloroacetamido-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside (53).

The acceptor 7 (2.59 g, 1.72 mmol) and the donor **9** (1.61 g, 2.58 mmol, 1.5 equiv) were dissolved in anhydrous CH₂Cl₂ (52 mL) with 4 Å AW molecular sieves (3.0 g). The temperature was lowered to -78 °C (acetone/CO₂) and the mixture was stirred for 30 min at this temperature under an argon atmosphere. Then, TMSOTf (93 µL, 0.52 mmol, 0.3 equiv) was added keeping rigorously anhydrous conditions. The mixture was stirred for 30 min at -78 °C and quenched by the addition of Et₃N (300 µL). The solvents were evaporated under reduced pressure and the resulting residue was purified by flash chromatography (Chex/EtOAc 9:1 to 6:4 + 1% Et₃N) to afford **53** (3.00 g, 89%) as a white foam. *R*_{f} 0.47 (Chex/EtOAc 7:3); [α]²⁵ -4° (c 0.5, CHCl₃); ¹H NMR (Pyr-d₅, 400 MHz) δ: 10.17 (br s, 1H, N*H*), 7.67-7.28 (m, 37H, C*H*_{Ph}), 7.03-6.99 (m, 2H, C*H*_{Ph}), 5.89 (high-order m, 1H, H-2_{All}), 5.73-5.68 (m, 2H, H-1_{D'}, H-2_{C}), 5.65 (br s, 1H, H-1_{A}), 5.54 (br s, 1H, H-1_{B}), 5.44 (d, *J*_{1,2} = 3.1 Hz, 1H, H-1_{E}), 5.31-5.19 (m, 4H, H-3a_{All}, C*H*₂Ph (3H)), 5.15 (d, *J*_{1,2} = 1.6 Hz, 1H, H-1_{C}), 5.11-4.97 (m, 6H, H-3b_{All}, C*H*₂Ph (4H), H-2_{B}), 4.95-4.77 (m, 7H, C*H*₂Ph), 4.74-4.63 (m, 4H, C*H*₂Ph, H-3_{D'}, H-2_{A}), 4.49 (high-order m, 1H, H-3_{C}), 4.39-4.19 (m, 7H, H-5_{A}, H-3_{E}, H-6a_{E}, H-3_{A}, H-3_{B}, H-1a_{All}, H-6b_{E}), 4.12-3.79 (m, 11H, H-5_{E}, H-4_{C}, H-5_{B}, H-5_{C}, H-4_{E}, H-4_{A}, H-1b_{All}, H-2_{D'}, H-4_{B}, H-6a_{D'}, H-2_{E}), 3.64 (s, 3H, OC*H*₃), 3.56-3.40 (m, 2H, H-6b_{D'}, H-5_{D'}), 3.39 (t, *J*_{3,4} = *J*_{4,5} = 8.7 Hz, 1H, H-4_{D'}), 2.82-2.69 (high-order m, 4H, 2×C*H*_{2Lev}), 2.03 (s, 3H, C(O)C*H*₃), 1.65 (d, *J*_{5,6} = 6.3 Hz, 3H, H-6_{B}), 1.52 (d, *J*_{5,6} = 6.1 Hz, 3H, H-6_{C}), 1.48 (d, *J*_{5,6} = 6.1 Hz, 3H, H-6_{A}), 1.46 (s, 3H, C*H*_{3Iso}), 1.29 (s, 3H, C*H*_{3Iso}), 1.07 (s, 9H, C(C*H*₃)₃), 0.32 (s, 3H, Si(C*H*₃)₂), 0.24 (s, 3H, Si(C*H*₃)₂). ¹³C NMR (Pyr-d₅, 100 MHz) δ: 206.1 (*C*(O)CH_{2Lev}), 173.1 (*C*=O), 164.5 (*C*=O), 160.2 (C_{Ph}), 140.1-139.4 (C_{Ph}), 134.6 (C-2_{All}), 131.5-128.2 (*C*H_{Ph}), 117.7 (C-3_{All}), 114.6 (CH_{Ph}), 102.8 (*C*-1_{B}), 102.5 (C-1_{A}), 100.8 (C-1_{D'}), 99.9 (*C*(CH₃)₂), 99.1 (C-1_{E}), 96.7 (C-1_{C}), 94.4 (*C*Cl₃), 82.5 (C-3_{A}, C-2_{E}), 81.5 (C-4_{A}), 81.3 (C-3_{C}), 81.1 (C-4_{B}), 79.8 (C-3_{E}), 79.4 (C-3_{B}, C-4_{C}), 78.4 (C-4_{E}), 76.1 (*C*H₂Ph), 76.1 (*C*H₂Ph), 75.9 (*C*H₂Ph), 75.7 (C-4_{D'}), 75.4 (*C*H₂Ph), 75.3 (C-2_{B}), 75.0 (C-2_{A}), 74.3 (*C*H₂Ph), 73.8 (*C*H₂Ph), 73.2 (C-2_{C}), 73.0 (*C*H₂Ph), 72.5 (C-5_{A}), 71.9 (C-3_{D'}), 70.7 (*C*H₂Ph), 69.9 (C-5_{B}), 69.4 (C-5_{C}), 69.3 (C-6_{E}), 68.7 (C-1_{All}), 68.3 (C-5_{E}), 67.2 (C-5_{D'}), 62.6 (C-6_{D'}), 61.8 (C-2_{D'}), 55.4 (O*C*H₃), 38.4 (*C*H_{2Lev}), 29.8 (C(O)CH₃), 29.5 (*C*H_{3Iso}), 29.0 (*C*H_{2Lev}), 26.7 (C(CH₃)₃), 19.6 (C-6_{B}), 19.4 (*C*H_{3Iso}), 18.9 (C-6_{C}, *C*(CH₃)₃), 18.8 (C-6_{A}), -3.0 (Si(*C*H₃)₂), -4.0 (Si(*C*H₃)₂). HR-ESI-TOF-MS *m*/*z* 1966.7288 [M + H]⁺ (calcd for C₁₀₆H₁₃₁Cl₃NO₂₆Si, 1966.7794), *m*/*z* 1988.7192 [M + Na]⁺ (calcd for C₁₀₆H₁₃₀Cl₃NO₂₆SiNa, 1988.7614).

### Allyl (3-O-tert-butyldimethylsilyl-2-deoxy-4,6-O-isopropylidene-2-trichloroacetamido-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-acetyl-α-L-rhamnopyranoside (49).

The acceptor **8** (309 mg, 0.213 mmol) and the donor **9** (199 mg, 0.319 mmol, 1.5 equiv) were dissolved in anhydrous CH₂Cl₂ (8.5 mL) with 4 Å powdered molecular sieves (426 mg). The temperature was lowered to -45 °C (CH₃CN/CO₂) and the mixture was stirred for 30 min at this temperature under an argon atmosphere. Then, TMSOTf (2 µL, 11 µmol, 0.05 equiv) was added keeping rigorously anhydrous conditions. Additional TMSOTf (12 µL, 64 µmol, 0.3 equiv) was added at 60, 90, 120 and 150 min. During this time the mixture was stirred at room temperature under an argon atmosphere. After completion of the reaction (3 h), the mixture was quenched by the addition of Et₃N (300 µL). The solvents were evaporated under reduced pressure and the resulting residue was purified by flash chromatography (isocratic Tol/EtOAc 95:5 + 0.5% Et₃N) to afford **49** (304 mg, 75%) as a white foam. *R*_{f} 0.73 (Chex/EtOAc 7:3); [α]²⁵_{D} -7° (c 1.0, CHCl₃); ¹H NMR (Pyr-d₅, 400 MHz) δ: 10.5 (br s, 1H, N*H*), 7.70-7.25 (m, 37H, C*H*_{Ph}), 7.03-6.98 (m, 2H, C*H*_{Ph}), 5.89 (high-order m, 1H, H-2_{All}), 5.74 (d, *J*_{1,2} = 8.4 Hz, 1H, H-1_{D'}), 5.68 (dd, *J*_{2,3} = 3.2 Hz, *J*_{1,2} = 1.9 Hz, 1H, H-2_{C}), 5.62 (br s, 1H, H-1_{A}), 5.51 (br s, 1H, H-1_{B}), 5.35 (d, *J*_{1,2} = 3.2 Hz, 1H, H-1_{E}), 5.32-5.03 (m, 10H, C*H*₂Ph (6H), H-3a_{All}, H-1_{C}, H-3b_{All} H-2_{B}), 4.99-4.59 (m, 12H, C*H*₂Ph (10H), H-3_{D'}, H-2_{A}), 4.47 (dd, *J*_{3,4} = 8.4 Hz, *J*_{2,3} = 3.2 Hz, 1H, H-3_{C}), 4.36-4.17 (m, 7H, H-5_{A}, H-3_{E}, H-6a_{E}, H-3_{A}, H-1a_{All}, H-3_{B}, H-6b_{E}), 4.13-3.96 (m, 7H, H-5_{B}, H-5_{E}, H-5_{C}, H-1b_{All}, H-4_{C}, H-4_{E}, H-4_{A}), 3.95-3.77 (m, 4H, H-2_{D'}, H-4_{B}, H-6a_{D'}, H-2_{E}), 3.61 (s, 3H, OC*H*₃), 3.52-3.43 (m, 2H, H-6b_{D'}, H-5_{D'}), 3.35 (t, *J*_{3,4} = *J*_{4,5} = 8.5 Hz, 1H, H-4_{D'}), 2.01 (s, 3H, C(O)C*H*₃), 1.67 (d, *J*_{5,6} = 5.6 Hz, 3H, H-6_{B}), 1.48 (d, *J*_{5,6} = 6.1 Hz, 3H, H-6_{C}*), 1.46-1.43 (m, 6H, H-6_{A}*, C(C*H*₃)₂), 1.24 (s, 3H, C(C*H*₃)₂), 1.06 (s, 9H, C(C*H*₃)₃), 0.31 (s, 3H, Si(C*H*₃)₂), 0.22 (s, 3H, Si(C*H*₃)₂). ¹³C NMR (Pyr-d₅, 100 MHz) δ: 170.9 (*C*=O), 163.0 (*C*=O), 160.3 (C_{Ph}), 140.2-139.5 (C_{Ph}), 134.7 (C-2_{All}), 131.6-128.1 (*C*H_{Ph}), 117.7 (C-3_{All}), 114.8 (*C*H_{Ph}), 102.5 (C-1_{A}, C-1_{B}), 100.9 (C-1_{D'}), 99.9 (*C*(CH₃)₂), 99.2 (C-1_{E}), 96.7 (C-1_{C}), 94.5 (*C*Cl₃), 82.6 (C-3_{A}, C-2_{E}), 81.6 (C-3_{C}, C-4_{A}), 81.2 (C-4_{B}), 80.0 (C-3_{E}), 79.9 (C-4_{C}), 79.5 (C-3_{B}), 78.6 (C-4_{E}), 76.2 (*C*H₂Ph), 76.1 (*C*H₂Ph), 76.0 (*C*H₂Ph), 75.8 (C-4_{D'}), 75.5 (*C*H₂Ph), 75.4 (C-2_{B}), 75.1 (C-2_{A}), 74.4 (*C*H₂Ph), 73.9 (*C*H₂Ph), 73.1 (*C*H₂Ph), 73.1 (C-2_{C}), 72.6 (C-5_{A}), 72.1 (C-3_{D'}), 70.8 (*C*H₂Ph), 69.9 (C-5_{B}), 69.6 (C-5_{C}), 69.4 (C-6_{E}), 68.9 (C-1_{All}), 68.3 (C-5_{E}), 67.4 (C-5_{D'}), 62.7 (C-6_{D},), 61.9 (C-2_{D'}), 55.6 (OCH₃), 29.6 (C(CH₃)₂), 26.8 (C(CH₃)₃), 21.3 (C(O)CH₃), 19.5 (C-6_{B}, C(*C*E₃)₂)), 18.9 (*C*(CH₃)₃), 18.9 (C-6_{C}, C-6_{A}), -3.02 (Si(*C*H₃)₂), -3.91 (Si(*C*H₃)₂)- HR-ESI-TOF-MS *m*/*z* 1932.7523 [M + Na]⁺ (calcd for C₁₀₃H₁₂₆Cl₃NO₂₅SiNa, 1932.7351).

### (2-Acetamido-3-O-tert-butyldimethylsilyl-2-deoxy-4,6-O-isopropylidene-β-D-glucopyranosyl)-(1→2)-(4-O-benzyl-3-O-para-methoxybenzyl-α-L-rhamnopyranosyl)-(1→2)-(3,4-di-0-benzyl-α-L- rhamnopyranosyl)-(1→3)-[2,3,4,6-tetra-0-benzyl-α-D-glucopyranosyl-(1→4)]-2-O-levulinoyl-α-L-rhamnopyranose (54).

To a solution of **53** (1.50 g, 0.762 mmol) in anhydrous toluene (30.5 mL) was added Bu₃SnH (2.05 mL, 7.62 mmol, 10.0 equiv) followed by AIBN (125 mg, 0.762 mmol, 1.0 equiv). The mixture was stirred for 2 h at reflux under an argon atmosphere, then the solvents were removed under reduced pressure. The resulting residue was purified by flash chromatography (Tol/EtOAc 95:5 to 9:1 + 1% Et₃N) to give a crude white foam (1.42 g). 1,5-Cyclooctadiene-bis(methyldiphenylphosphine)-iridium hexafluorophosphate (64 mg, 0.076 mmol, 0.10 equiv) was dissolved in anhydrous THF (3.8 mL) and the resulting red solution was degassed under an argon atmosphere. Hydrogen was bubbled through the solution for 15 min, then the resulting yellow solution was once again degassed under an argon atmosphere. The previous crude white foam (1.42 g, 0.762 mmol) was dissolved in anhydrous THF (3.8 mL). The mixture was stirred for 16 h at room temperature under an argon atmosphere. A solution of iodine (387 mg, 1.52 mmol, 2.0 equiv) in THF/H₂O (4.6 mL, 4:1 v/v) was added to the mixture, which was stirred for 1 h at room temperature. The reaction was then quenched by the addition of a freshly prepared solution of 10% aqueous sodium bisulfite. The mixture was diluted with CH₂Cl₂ (100 mL), water was added (50 mL) and the aqueous phase was extracted with CH₂Cl₂ (3×100 mL). The organic layer was dried over anhydrous Na₂SO₄, filtered and evaporated to dryness. The resulting residue was purified by flash chromatography (Tol/EtOAc 9:1 to 6:4 + 1% Et₃N) to afford **54** (810 mg, 58%, 3 steps from **53**) as a white foam. *R*_{f} 0.15(Chex/EtOAc 7:3); [CC]²⁵_{D} +12° (c 0.5, CHCl₃); ¹H NMR (Pyr-d₅, 400 MHz) δ: 9.27 (br s, 1H, N*H*), 7.68-7.26 (m, 37H, C*H*_{Ph}), 7.01-6.96 (m, 2H, C*H*_{Ph}), 5.85 (br s, 1H, H-2_{C}), 5.76-5.72 (m, 2H, H-1_{A}, H-1_{C}), 5.65 (d, *J*_{1,2} = 7.2 Hz, 1H, H-1_{D'}), 5.58 (br s, 1H, H-1_{B}), 5.48 (d, *J*_{1,2} = 2.7 Hz, 1H, H-1_{E}), 5.29-4.97 (m, 8H, C*H*₂Ph (7H), H-2_{B}), 4.96-4.62 (m, 12H, C*H*₂Ph (9H), H-3_{C}, H-2_{A}, H-3_{D'}), 4.55 (high-order m, 1H, H-5_{C}), 4.43 (br d, *J* = 9.9 Hz, 1H, H-5_{E}), 4.39-4.02 (m, 9H, H-3_{E}, H-3_{A}, H-3_{B}, H-5_{A}, H-6a_{E}, H-4_{C}, H-5_{B}, H-6b_{E}, H-4_{E}), 3.98 (t, *J₃,₄ = J*_{4,5} = 9.4 Hz, 1H, H-4_{A}), 3.88 (t, *J*_{3,4} = *J*₄,₅ = 9.4 Hz, 1H, H-4_{B}), 3.85-3.73 (m, 3H, H-2_{E}, H-2_{D'}, H-6a_{D'}), 3.58 (s, 3H, OC*H*₃), 3.56-3.42 (m, 3H, H-6b_{D'}, H-5_{D'}, H-4_{D'}), 2.83-2.65 (high-order m, 4H, 2×C*H*_{2Lev}), 2.28 (s, 3H, C(O)C*H*_{3Ac}), 1.99 (s, 3H, C(O)C*H*_{3Lev}), 1.68 (d, *J*_{5,6} = 6.1 Hz, 3H, H-6_{C}), 1.52 (d, *J*_{5,6} = 6.0 Hz, 3H, H-6_{B}), 1.46 (s, 3H, C*H*_{3Iso}), 1.39 (d, *J*_{5,6} = 6.0 Hz, 3H, H-6_{A}), 1.29 (s, 3H, C*H*_{3Iso}), 1.02 (s, 9H, C(C*H*₃)₃), 0.24 (s, 3H, Si(C*H*₃)₂), 0.21 (s, 3H, Si(C*H*₃)₂). ¹³C NMR (Pyr-d₅, 100 MHz) δ: 206.0 (*C*(O)CH_{2Lev}), 173.2 (*C*=O), 170.7 (*C*(O)CH_{3Ac}), 160.3 (C_{Ph}), 140.3-139.7 (C_{Ph}), 131.6-128.2 (*C*H_{Ph}), 114.7 (*C*H_{Ph}), 102.9 (C-1_{B}), 102.8 (C-1_{D'}), 102.5 (C-1_{A}), 99.8 (*C*(CH₃)₂), 98.6 (C-1_{E}), 92.1 (C-1_{C}), 82.6 (C-3_{E}), 82.6 (C-2_{E}), 81.7 (C-4_{A}), 81.3 (C-3_{C}, C-4_{B}), 80.2 (C-3_{A}), 79.7 (C-4_{C}), 79.6 (C-4_{B}), 78.4 (C-4_{E}), 76.7 (C-2_{A}), 76.1 (2×CH₂Ph), 75.8 (C-4_{D}), 75.8 (*C*H₂Ph), 75.5 (C-2_{B}), 75.3 (*C*H₂Ph), 75.0 (C-2_{C}), 74.2 (*C*H₂Ph), 74.0 (*C*H₂Ph), 72.8 (*C*H₂Ph), 72.7 (C-3_{D'}), 72.5 (C-5_{E}), 71.8 (*C*H₂Ph), 69.8 (C-5_{B}), 69.7 (C-5_{A}), 69.6 (C-6_{E}), 67.9 (C-5_{C}), 67.5 (C-5_{D'}), 62.9 (C-6_{D'}), 60.4 (C-2_{D'}), 55.5 (O*C*H₃), 38.6 (*C*H_{2Lev}), 29.9 (C(O)*C*H_{3Lev}), 29.7 (*C*H_{3Iso}), 29.2 (*C*H_{2Lev}), 26.5 (C(*C*H₃)₃), 24.5 (C(O)*C*H_{3Ac}), 20.1 (C-6_{C}), 19.6 (*C*H_{3Iso}), 19.0 (C-6_{B}), 18.9 (*C*(CH₃)₃), 18.8 (C-6_{A}), -3.4 (Si(*C*H₃)₂), -4.2 (Si(*C*H₃)₂). HR-ESI-TOF-MS *m*/*z* 1824.8639 [M + H]⁺ (calcd for C₁₀₃H₁₃₀NO₂₆Si, 1824.8650), *m*/*z* 1846.8492 [M + Na]⁺ (calcd for C₁₀₃H₁₂₉NO₂₆SiNa, 1846.8469).

### (2-Acetamido-3-O-tert-butyldimethylsilyl-2-deoxy-4,6-O-isopropylidene-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-0-benzyl-α-D-glucopyranosyl-(1→4)]-2-0-levulinoyl-α-L-rhamnopyranosyl N-(phenyl)trifluoroacetimidate(5).

To a solution of **54** (777 mg, 0.426 mmol) in acetone (2.1 mL) was added *N*-(phenyl)trifluoroacetimidoyl chloride⁹⁷ (177 mg, 0.851 mmol, 2.0 equiv) followed by Cs₂CO₃ (153 mg, 0.468 mmol, 1.1 equiv). The mixture was stirred for 2 h at room temperature, then filtered and evaporated to dryness. The resulting residue was purified by flash chromatography (Tol/EtOAc 95:5 to 9:1 + 1% Et₃N) to furnish **5** (753 mg, 89%) as a white foam. *R_{f}* 0.48 (Chex/EtOAc 7:3); [α]²⁵D+4° (c 0.5, CHCl₃); ¹H NMR (Pyr-d₅, 400 MHz) δ: 7.67-7.09 (m, 42H, C*H*_{Ph}), 7.03-6.97 (m, 3H, C*H*_{Ph}, H-1_{C}), 5.86 (br s, 1H, H-2_{C}), 5.73 (br s, 1H, H-1_{A}), 5.65-5.59 (m, 2H, H-1_{D'}, H-1_{B}), 5.46 (br s, 1H, H-1_{E}), 5.23-4.57 (m, 19H, C*H*₂Ph (16H), H-2_{B}, H-2_{A}, H-3_{C}), 4.38 (br d, *J*= 10.0 Hz, 1H, H-5_{E}), 4.35-4.13 (m, 7H, H-3_{A}, H-5_{A}, H-3_{E}, H-5_{C}, H-3_{B}, H-6a_{E}, H-4c), 4.13-3.95 (m, 4H, H-5_{B}, H-6b_{E}, H-4_{E}, H-4_{B}), 3.88 (t, *J*_{3,4} *= J*_{4,5} = 9.1 Hz, 1H, H-4_{A}), 3.85-3.77 (m, 3H, H-2_{E}, H-2_{D'}, H-6a_{D'}), 3.63 (s, 3H, OC*H*₃), 3.61-3.44 (m, 3H, H-6b_{D'}, H-5_{D'}) H-4_{D'}), 2.75 (br s, 4H, 2×C*H*_{2Lev}), 2.26 (s, 3H, C(O)C*H*_{3Ac}), 2.00 (s, 3H, C(O)C*H*_{3Lev}), 1.63 (d, *J*_{5,6} = 5.6 Hz, 3H, H-6_{C}), 1.53 (d, *J*_{5,6} = 6.2 Hz, 3H, H-6_{B}), 1.49 (d, *J*_{5,6} = 6.1 Hz, 3H, H-6_{A}), 1.48 (s, 3H, C*H*_{3Iso}), 1.34 (s, 3H, C*H*_{3Iso}), 1.04 (s, 9H, C(C*H*₃)₃), 0.25 (s, 3H, Si(C*H*₃)₂), 0.23 (s, 3H, Si(C*H*₃)₂). ¹³C NMR (Pyr-d₅, 100 MHz) δ: 205.9 (*C*(O)CH_{2Lev}), 172.9 (*C*=O), 170.7 (*C*(O)CH_{3Ac}), 160.3 (C_{Ph}), 144.3 (*C*=NPh), 140.2-138.4 (C_{Ph}), 131.6-126.2 (*C*H_{Ph}), 102.9 (C-1_{D}, C-1_{B}), 102.7 (C-1_{A}), 99.8 (*C*(CH₃)₂), 99.3 (C-1_{E}), 82.6 (C-3_{E}), 82.4 (C-2_{E}), 81.7 (C-4_{B}), 81.2 (C-4_{A}), 80.3 (C-3_{C}), 80.2 (C-3_{A}), 79.6 (C-3_{B}), 79.0 (C-4_{C}), 78.5 (C-4_{E}), 76.7 (C-2_{A}), 76.2 (*C*H₂Ph), 76.1 (*C*H₂Ph), 75.9 (C-4_{D'}), 75.9 (*C*H₂Ph), 75.8 (C-2_{B}), 75.5 (*C*H₂Ph), 74.5 (*C*H₂Ph), 74.1 (*C*H₂Ph), 72.8 (*C*H₂Ph), 72.8 (C-5_{E}), 71.8 (C-2_{C}), 71.4 (C-5_{C}), 71.3 (*C*H₂Ph), 70.1 (C-5_{B}), 69.8 (C-5_{A}), 69.5 (C-6_{E}), 67.6 (C-5_{D'}), 63.0 (C-6_{D'}), 60.4 (C-2_{D'}), 55.6 (O*C*H₃), 38.4 (*C*H_{2Lev}), 29.8 (C(O)*C*H_{3Lev}), 29.7 (*C*H_{3Iso}), 28.8 (*C*H_{2Lev}), 26.5 (C(*C*H₃)₃), 24.5 (C(O)*C*H_{3Ac}), 19.6 (C-6_{C}), 19.5 (*C*H_{3Iso}), 19.0 (C-6_{B}), 18.9 (*C*(CH₃)₃), 18.7 (C-6_{A}), -3.4 (Si(*C*H₃)₂), -4.2 (Si(*C*H₃)₂).

### 2-Azidoethyl (2-acetamido-3-O-tert-butyldimethylsilyl-2-deoxy-4,6-O-isopropylidene-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-(2,3,4,6-tetra-O-benzyl-α-D-glucopyranosyl-(1→4)]-(2-O-levulinoyl-α-L-rhamnopyranosyl)-(1→3)-2-acetamido-4,6-di-O-benzyl-2-deoxy-β-D-glucopyranoside (55).

The acceptor **6** (266 mg, 0.566 mmol, 1.5 equiv) was dissolved in anhydrous toluene (15.1 mL) and the mixture was stirred for 1 h at room temperature in the presence of powdered 4 Å molecular sieves (2.11 g). TBSOTf (26 µL, 0.11 mmol, 0.3 equiv) was added keeping rigorous anhydrous conditions and the mixture was heated to 75 °C. A solution of donor **5** (753 mg, 0.377 mmol, 1.0 equiv) in anhydrous toluene (15.1 mL) was slowly added to the mixture, then an additional portion of TBSOTf (26 µL, 0.11 mmol, 0.3 equiv) was injected. The reaction was stirred for 1 h at 75 °C under an argon atmosphere. The temperature was lowered to room temperature, then the mixture was quenched with Et₃N (300 µL), filtered and the solvents were removed under reduced pressure. The resulting residue was purified by flash chromatography (Chex/EtOAc 6:4 to 4:6 + 1% Et₃N) to give **55** (719 mg, 84%) as a white foam. [a]²⁵_{D}-8° (*c* 0.5, CHCl₃); ¹H NMR (Pyr-d₅, 400 MHz) δ: 9.53 (br s, 1H, N*H*), 8.94 (br s, 1H, N*H*), 7.70-7.25 (m, 47H, C*H*_{Ph}), 7.01-6.96 (m, 2H, C*H*_{Ph}), 5.76 (br s, 1H, H-2_{C}), 5.72 (br s, 1H, H-1_{A}), 5.69-5.62 (m, 2H, H-1_{D'}, H-1_{C}), 5.60 (br s, 1H, H-1_{B}), 5.37 (d, *J*_{1,2} = 2.5 Hz, 1H, H-1_{E}), 5.28 (m, 1H, H-1_{D}), 5.25-4.54 (m, 25H, 10×C*H*_{2Ph}, H-2_{B}, H-3_{D}, H-2_{A}, H-3_{D'}, H-3_{C}), 4.44-4.04 (m, 11H, H-5_{E}, H-5_{C}, H-5_{A}, H-6a_{E}, H-3_{A}, H-3_{B}, H-3_{E}, H-6b_{E}, H-6a_{D}, H-5_{B}, H-4_{E}), 4.03-3.92 (m, 2H, H-4_{C}, H-4_{A}), 3.91-3.68 (m, 9H, H-2_{D}, OC*H*₂a, OC*H*₂b, H-4_{B}, H-6b_{D}, H-6a_{D'}, H-2_{D'}, H-2_{E}, H-5_{D}), 3.63-3.44 (m, 8H, OC*H*₃, H-4_{D}, H-6b_{D'}, H-5_{D'}, H-4_{D'}, C*H*₂N₃a), 3.41-3.33 (m, 1H, C*H*₂N₃b), 2.75-2.61 (m, 4H, 2×C*H*_{2Lev}), 2.37 (s, 3H, C(O)C*H*_{3Ac}), 2.28 (s, 3H, C(O)C*H*_{3Ac}), 1.99 (s, 3H, C(O)C*H*_{3Lev}), 1.53 (d, *J*_{5,6} = 6.1 Hz, 3H, C*H*_{3Rha}), 1.47 (s, 3H, C*H*_{3Iso}), 1.39 (d, *J*_{5,6} = 6.0 Hz, 3H, C*H*_{3Rha}), 1.36-1.31(m, 6H, C*H*_{3Iso}, C*H*_{3Rha}), 1.02 (s, 9H, C(C*H*₃)₃), 0.25 (s, 3H, Si(C*H*₃)₂), 0.22 (s, 3H, Si(C*H*₃)₂). ¹³C NMR (Pyr-d₅, 100 MHz) δ: 206.0 (*C*(O)CH_{2Lev}), 173.0 (*C*=O_{Lev}), 171.9 (*C*(O)CH_{3Ac}), 170.7 (*C*(O)CH_{3Ac}), 160.2 (C_{Ph}), 140.3-139.4 (C_{Ph}), 131.6-127.9 (*C*H_{Ph}), 114.7 (*C*H_{Ph}), 102.8 (C-1_{D'}*), 102.4 (C-1_{B}*), 102.3 (C-1_{A}), 101.1 (C-1_{D}), 99.7 (*C*(CH₃)₂), 99.2 (C-1_{E}), 98.4 (C-1_{C}) 82.6 (C-3_{E}, C-2_{E}), 81.7 (C-4_{A}), 81.4 (C-3_{C}), 81.1 (C-4_{B}), 79.8 (C-3_{A}, C-3_{B}, C-4_{C}), 79.1 (C-3_{D}), 78.7 (C-4_{E}), 78.6 (C-4_{D}), 76.6 (C-2_{A}), 76.1 (*C*H₂Ph), 76.0 (C-5_{D}), 76.0 (*C*H₂Ph), 75.9 (C-4_{D},), 75.8 (*C*H₂Ph), 75.6 (C-2_{B}), 75.5 (*C*H₂Ph), 75.0 (*C*H₂Ph), 74.3 (*C*H₂Ph), 74.0 (2×*C*H₂Ph), 73.6 (C-2_{C}), 72.8 (C-5_{E}), 72.7 (C-3_{D'}), 72.6 (*C*H₂Ph), 70.9 (*C*H₂Ph), 69.9 (O*C*H₂), 69.8 (C-5_{B}), 69.6 (C-5_{A}), 69.4 (C-6_{E}), 68.6 (C-5_{C}), 68.5 (C-6_{D}), 67.5 (C-5_{D'}), 62.9 (C-6_{D},), 60.3 (C-2_{D'}), 58.2 (C-2_{D}), 55.5 (OCH₃), 51.6 (CH₂N₃), 38.4 (*C*H_{2Lev}), 29.8 (C(O)*C*H_{3Lev}), 29.7 (*C*H_{3Iso}), 29.1 (*C*H_{2Lev}), 26.5 (C(*C*H₃)₃), 24.5 (C(O)*C*H_{3Ac}), 24.2 (C(O)*C*H_{3Ac}), 19.5 (C-6_{C}, *C*H_{3Iso}), 19.0 (C-6_{B}*), 18.9 (C-6_{A}*), 18.9 (C(CH₃)₃), -3.4 (Si(CH₃)₂), -4.2 (Si(CH₃)₂). HR-ESI-TOF-MS *m*/*z* 2277.0205 [M + H]⁺ (calcd for C₁₂₇H₁₅₈N₅O₃₁Si, 2277.0710), *m*/*z* 2299.0171 [M + Na]⁺ (calcd for C₁₂₇H₁₅₇N₅O₃₁SiNa, 2299.0530).

### 2-Azidoethyl (2-acetamido-2-deoxy-4,6-O-isopropylidene-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-2-acetamido-4,6-di-O-benzyl-2-deoxy-β-D-glucopyranoside (56).

To a solution of **55** (712 mg, 0.313 mmol) in anhydrous THF (12.5 mL) was added HOAc (179 µL, 3.13 mmol, 10.0 equiv) followed by 1 M TBAF in THF (6.24 mL, 6.25 mmol, 20.0 equiv). The mixture was stirred for 5 d at room temperature under an argon atmosphere. CH₂Cl₂ (100 mL) was added and the organic layer was washed with H₂O (1×50 mL). Then, the solvents of the dried solution (anhydrous Na₂SO₄) were removed under reduced pressure. The resulting residue was purified by flash chromatography (Chex/EtOAc 5:5 to 100% EtOAc + 1% Et₃N) to afford **56** (576 mg, 85%) along with unreacted **55** (51 mg, 7%) both as white foam. *R_{f}* 0.69 (CH₂Ch/MeOH 95:5); [a]²⁵_{D}-8° (*c* 1.0, CHCl₃); ¹H NMR (Pyr-d₅, 400 MHz) δ: 9_{.}36 (d, *J =* 6.2 Hz, 1H, N*H*), 8.61 (d, *J =* 7.6 Hz, 1H, O*H*), 7.74-7.26 (m, 47H, C*H*_{Ph}), 7.02-6.97 (m, 2H, C*H*_{Ph}), 5.79 (br s, 1H, H-1_{A}), 5.76 (br s, 1H, H-2_{C}), 5.65 (br s, 1H, H-1_{C}), 5.61 (br s, 1H, H-1_{B}), 5.45 (d, *J*_{1,2} = 8.3 Hz, 1H, H-1_{D'}), 5.40 (d, *J*_{1,2} = 2.8 Hz, 1H, H-1_{E}), 5.28-4.55 (m, 25H, 10×C*H*₂Ph, H-1_{D}, H-2_{B}, H-2_{A}, H-3_{D}, H-3c), 4.52 (t, *J*_{2,3} = *J*_{3,4} = 9.3 Hz, 1H, H-3_{D'}), 4.43-3.98 (m, 14H, H-5_{E}, H-3_{A}, H-5_{C}, H-6a_{E}, H-5_{A}, H-2_{D'}, H-3_{B}, H-3_{E}, H-6b_{E}, H-6a_{D}, H-5_{B}, H-4_{E}, H-4_{A}, H-4_{C}), 3.92-3.45 (m, 16H, H-2_{D}, H-4_{B}, OC*H*₂a, OC*H*₂b, H-6a_{D'}, H-6b_{D}, H-5_{D}, H-2_{E}, H-4_{D'}, H-6b_{D'}, H-4_{D}, OC*H*₃, H-5_{D'}, C*H*₂N₃a), 3.41-3.34 (m, 1H, C*H*₂N₃b), 2.77-2.64 (m, 4H, 2×C*H*_{2Lev}), 2.36 (s, 3H, C(O)C*H*_{3Ac}), 2.12 (s, 3H, C(O)C*H*_{3Ac}), 2.01 (s, 3H, C(O)C*H*_{3Lev}), 1.54 (d, *J*_{5,6} = 6.0 Hz, 3H, C*H*_{3Rha}), 1.47 (s, 3H, C*H*_{3Iso}), 1.42-1.38 (m, 9H, 2×C*H*_{3Rha}, C*H*_{3Iso}). ¹³C NMR (Pyr-d₅, 100 MHz) δ: 206.0 (C(O)CH_{2Lev}), 173.0 (*C*=O_{Lev}), 171.9 (*C*(O)CH_{3Ac}), 171.3 (C(O)CH_{3Ac}), 160.3 (C_{Ph}), 140.3-139.4 (C_{Ph}), 131.5-128.0 (*C*H_{Ph}), 114.8 (CH_{Ph}), 104.2 (C-1_{D'}), 102.5 (C-1_{B}), 102.2 (C-1_{A}), 101.1(C-1_{D}), 100.0 (C(C*H*₃)₂), 99.3 (C-1_{E}), 98.4 (C-1_{C}), 82.7 (C-2_{E}, C-3_{E}), 81.8 (C-4_{A}), 81.4 (C-3_{C}), 81.2 (C-4_{B}), 80.1 (C-3_{A}, C-4_{C}), 79.8 (C-3_{B}), 79.1 (C-3_{D}), 78.7 (C-4_{E}), 78.6 (C-4_{D}), 77.3 (C-2_{A}), 76.1 (*C*H₂Ph), 76.0 (*C*H₂Ph), 76.0 (C-5_{D}, C-4_{D'}), 75.8 (CH₂Ph), 75.6 (*C*H₂Ph), 75.0 (*C*H₂Ph), 74.8 (C-2_{B}), 74.3 (CH₂Ph), 74.0 (2×*C*H₂Ph), 73.6 (C-2_{C}), 72.9 (C-5_{E}), 72.8 (CH₂Ph), 72.5 (C-3_{D'},), 71.0 (CH₂Ph), 69.9 (OC*H₂*), 69.7 (C-5_{A}, C-5_{B}), 69.4 (C-6_{E}), 68.6 (C-5_{C}), 68.5 (C-6_{D}), 68.3 (C-5_{D'}), 63.0 (C-6_{D'},), 59.8 (C-2_{D'}), 58.2 (C-2_{D}), 55.6 (OCH₃), 51.6 (CH₂N₃), 38.4 (*C*H_{2Lev}), 29.9 (C(O)*C*H_{3Lev}, CH_{3Iso}), 29.1 (CH_{2Lev}), 24.2 (2×C(O)*C*H_{3Ac}), 19.7, 19.5, 19.0, 18.9 (C-6_{A}, C-6_{B}, C-6c, *C*H_{3Iso}). HR-ESI-TOF-MS *m*/*z* 2163.0098 [M + H]⁺ (calcd for C₁₂₁H₁₄₄N₅O₃₁, 2162.9846), *m*/*z* 2184.9822 [M + Na]⁺ (calcd for C₁₂₁H₁₄₃N₅O₃₁Na, 2184.9666).

### Allyl (3,4-di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranoside(57).

The acceptor **43** (1.56 g, 1.36 mmol) and the donor **14** (1.20 g, 2.04 mmol, 1.5 equiv) were dissolved in anhydrous Et₂O (20 mL) with 4 Å AW molecular sieves (2.7 g). The temperature was lowered to -10 °C (acetone/ice water) and the mixture was stirred for 30 min under an argon atmosphere. Then, TMSOTf (24 µL, 0.13 mmol, 0.1 equiv) was added keeping rigorously anhydrous conditions. The mixture was stirred for 1 h at -10 °C to room temperature and the reaction was quenched by the addition of Et₃N (300 µL). The solvents were evaporated under reduced pressure and the resulting residue was purified by flash chromatography (Tol/EtOAc 95:5 to 9:1) to afford **57** (1.95 g, 92%) as a colorless oil. *R_{f}* 0.41 (Chex/E_{tOA}c 7:3); [α]²⁵_{D} +11° (c 1.0, CHCl₃); ¹H NMR (CDCl₃, 400 MHz) δ: 7.40-7.11 (m, 40H, C*H*_{Ph}), 5.91 (high-order m, 1H, H-2_{All}), 5.57 (d, *J*_{2,3} = 3.4 Hz, *J*_{1,2} = 1.8 Hz, 1H, H-2_{A}), 5.30 (ddd, *J* = 17.2, 3.2, 1.6 Hz, 1H, H-3a_{All}), 5.21 (ddd, *J* = 10.4, 2.7, 1.3 Hz, 1H, H-3b_{All}), 5.13 (dd, *J*_{2,3} = 3.4 Hz, *J*_{1,2} = 2.0 Hz, 1H, H-2c), 5.10 (d, *J*_{1,2} = 1.5 Hz, 1H, H-1_{A}), 5.01 (d, *J*_{1,2} = 3.1 Hz, 1H, H-1_{E}), 4.98 (d, *J*_{1,2} = 1.6 Hz, 1H, H-1_{B}), 4.96 (d, *J* = 10.9 Hz, 1H, C*H*₂Ph), 4.94 (d, *J* = 10.9 Hz, 1H, C*H*₂Ph), 4.92 (d, *J* = 10.8 Hz, 1H, C*H*₂Ph), 4.86 (d, *J* = 10.9 Hz, 1H, C*H*₂Ph), 4.82 (d, *J* = 10.9 Hz, 1H, C*H*₂Ph), 4.76 (d, *J* = 11.5 Hz, 1H, C*H*₂Ph), 4.76 (d, *J*_{1,2} = 1.6 Hz, 1H, H-1_{C}), 4.72 (d, *J* = 11.2 Hz, 1H, C*H*₂Ph), 4.67-4.52 (m, 7H, C*H*₂Ph), 4.48 (d, *J* = 11.0 Hz, 1H, C*H*₂Ph), 4.42 (br s, 1H, H-2_{B}), 4.39 (d, *J =* 11.7 Hz, 1H, C*H*₂Ph), 4.14 (ddt, *J =* 13.0, 5.3, 1.4 Hz, 1H, H-1aAₗₗ)_{,} 4.05-3.93 (m, 6H, H-5_{A}, H-1b_{All}, H-3_{A}, H-3_{C}, H-5_{E}, H-2_{E}), 3.82-3.68 (m, 5H, H-6a_{E}, H-6b_{E}, H-5_{C}, H-3_{B}, H-4_{E}), 3.61-3.53 (m, 2H, H-4_{A}, H-5_{B}), 3.48 (dd, *J =* 9.8, 3.3 Hz, 1H, H-3_{E}), 3.44 (t, *J*_{3,4} = *J*_{4,5} = 3.4 Hz, 1H, H-4_{C}), 3.40 (t, *J*_{3,4} = *J*_{4,5} = 3.4 Hz, 1H, H-4_{B}), 2.78-2.48 (high-order m, 8H, 4×C*H*_{2Lev}), 2.09 (s, 3H, C(O)C*H*₃), 2.08 (s, 3H, C(O)C*H*₃), 1.36 (d, *J*_{5,6} = 6.2 Hz, 3H, H-6_{A}), 1.34 (d, *J*_{5,6} = 6.2 Hz, 3H, H-6_{C}), 1.31 (d, *J*_{5,6} = 6.2 Hz, 3H, H-6_{B}). ¹³C NMR (CDCl₃, 100 MHz) δ: 206.0 (C(O)CH_{2Lev}), 205.9 (*C*(O)CH_{2Lev}), 172.2 (*C*=O), 171.8 (*C*=O), 138.9-138.3 (P_{Ph}), 133.8 (C-2_{All}), 129.2-125.4 (*C*H_{Ph}), 117.6 (C-3_{All}), 101.3 (C-1_{B}), 99.4 (C-1_{A}), 97.8 (C-1_{E}), 96.0 (C-1_{C}), 81.8 (C-5_{E}), 81.4 (C-3_{E}), 80.2 (C-4_{C}), 80.0 (C-4_{B}), 79.8 (C-3_{C}), 79.0 (C-3_{B}), 78.0 (C-4_{A}), 77.7 (C-3_{A}, C-4_{E}), 75.7 (CH₂Ph), 75.4 (*C*H₂PH), 75.3 (*C*H₂Ph), 75.1 (CH₂Ph), 74.8 (C-2_{B}), 73.9 (CH₂Ph), 73.0 *C*H₂Ph), 72.5 (C-2_{C}), 71.7 (CH₂Ph), 71.4 (C-5_{A}), 70.8 (CH₂Ph), 69.5 (C-2_{A}), 68.9 (C-5_{B}), 68.5 (C-1_{All}, C-6_{E}), 68.3 (C-2_{E}), 67.5 (C-5_{C}), 38.2 (*C*H_{2Lev}), 38.1 (*C*H_{2Lev}), 29.8 (C(O)*C*H₃), 29.8 (C(O)CH₃), 28.3 (CH_{2Lev}), 28.3 (CH_{2Lev}), 18.8 (C-6_{A}), 18.4 (C-6_{C}), 18.2 (C-6_{B}). HR-ESI-TOF-MS *m*/*z* 1575.7284 [M + H]⁺ (calcd for C₉₃H₁₀₇O₂₂, 1575.7253), *m*/*z* 1597.7047 [M + Na]⁺ (calcd for C₉₃H₁₀₆O₂₂Na, 1597.7074).

### (3,4-Di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranose(58).

1,5-Cyclooctadiene-bis(methyldiphenylphosphine)-iridium hexafluorophosphate (91 mg, 0.11 mmol, 0.10 equiv) was dissolved in anhydrous THF (5.4 mL) and the resulting red solution was degassed under an argon atmosphere. Hydrogen was bubbled through the solution for 15 min, then the resulting yellow solution was once again degassed under an argon atmosphere. A solution of crude **57** (1.69g, 1.07 mmol) in anhydrous THF (5.4 mL) was then added. The mixture was stirred for 20 h at room temperature under an argon atmosphere. A solution of iodine (544 mg, 2.14 mmol, 2.0 equiv) in THF/H₂O (6.4 mL, 4:1 v/v) was added to the mixture, which was stirred for 1 h at room temperature. The reaction was then quenched by the addition of a freshly prepared solution of 10% aqueous sodium bisulfite. The mixture was diluted with CH₂Cl₂ (250 mL), water was added (100 mL) and the aqueous phase was extracted with CH₂Cl₂ (3x250 mL). The organic layer was dried over anhydrous Na₂SO₄, filtered and evaporated to dryness. The resulting residue was purified by flash chromatography (Tol/EtOAc 8:2 to 7:3) to afford **58** (1.52g, 92%, 2 steps, ratio α/β ≈ 70:1) as a light yellow foam. *R_{f}* 0.19 (Chex/EtOAc 7:3); [α]²⁵_{D}+22° (c 1.0, CHCl₃); ¹H NMR (CDCl₃, 400 MHz) δ (α): 7.37-7.11 (m, 40H, C*H*_{Ph}), 5.56 (dd, *J*_{2,3} = 3.3 Hz, *J*_{1,2} = 1.8 Hz, 1H, H-2_{A}), 5.12 (pt, *J* = 2.7 Hz, 1H, H-2_{C}), 5.10-5.06 (m, 2H, H-1_{C}, H-1_{A}), 5.00-4.90 (m, 5H, H-1_{E}, H-1_{B}, C*H*₂Ph (3H)), 4.85 (d, *J* = 11.0 Hz, 1H, C*H*₂Ph), 4.8 (d, *J* = 11.0 Hz, 1H, CH₂Ph), 4.75 (d, *J* = 11.7 Hz, 1H, C*H*₂Ph), 4.72 (d, *J* = 11.4 Hz, 1H, C*H*₂Ph), 4.67-4.51 (m, 7H, C*H*₂Ph), 4.48 (d, *J* = 10.8 Hz, 1H, C*H*₂Ph), 4.41-4.36 (m, 2H, H-2_{B}, C*H*₂Ph), 4.05-3.91 (m, 6H, H-3_{C}, H-5_{A}, H-5_{E}, H-3_{A}, H-2_{E}, H-5_{C}), 3.82-3.68 (m, 4H, H-6a_{E}, H-6b_{E}, H-3_{B}, H-4_{E}), 3.62-3.53 (m, 2H, H-5_{B}, H-4_{A}), 3.48 (dd, *J* = 9.7, 3.1Hz, 1H, H-3_{E}), 3.43 (t, *J*_{3,4} = *J*_{4,5} = 9.3 Hz, 1H, H-4_{C}), 3.40 (t, *J*_{3,4} = *J*_{4,5} = 9.3 Hz, 1H, H-4_{B}), 3.06 (d, *J*_{OH,1} = 4.2 Hz, 1H, O*H*), 2.76-2.47 (high-order m, 8H, 4×C*H*_{2Lev}), 2.08 (s, 6H, 2×C(O)C*H*₃), 1.35 (d, *J*_{5,6} = 6.2 Hz, 3H, H-6_{A}), 1.32 (d, *J*_{5,6} = 6.3 Hz, 3H, H-6_{C}), 1.21 (d, *J*_{5,6} = 6.2 Hz, 3H, H-6_{B}). ¹³C NMR (CDCl₃, 100 MHz) δ (α): 206.0 (2×*C*(O)CH_{2Lev}), 172.2 (*C*=O), 171.8 (*C*=O), 138.9-138.3 (P_{Ph}), 128.7-127.4 (*C*H_{Ph}), 101.1 (C-1_{B}), 99.4 (C-1_{A}), 97.8 (C-1_{E}), 91.3 (C-1_{C}), 81.9 (C-5_{E}), 81.4 (C-3_{E}), 80.2 (C-4_{C}), 80.1 (C-4_{B}), 79.0 (C-3_{C}, C-3_{B}), 78.2 (C-4_{A}), 77.8 (C-3_{A}, C-4_{E}), 75.7 (*C*E₂Ph), 75.4 (*C*H₂Ph), 75.4 (*C*H₂Ph), 75.2 (*C*H₂Ph), 74.9 (C-2_{B}), 73.9 (*C*H₂Ph), 73.1 (*C*H₂Ph), 72.8 (C-2_{C}), 71.7 (*C*H₂Ph), 71.5 (C-5_{A}), 70.9 (*C*H₂Ph), 69.5 (C-2_{A}), 68.9 (C-5_{B}), 68.5 (C-6_{E}), 68.4 (C-2_{E}), 67.6 (C-5_{C}), 38.2 (*C*H_{2Lev}), 38.1 (*C*H_{2Lev}), 29.8 (C(O)*C*H₃), 29.8 (C(O)*C*H₃), 28.3 (*C*H_{2Lev}), 28.2 (*C*H_{2Lev}), 18.9 (C-6_{A}), 18.5 (C-6_{C}), 18.1 (C-6_{B}). HR-ESI-TOF-MS *m*/*z* 1535.6907 [M + H]⁺ (calcd for C₉₀H₁₀₃O₂₂, 1535.6941), *m*/*z* 1557.6749 [M + Na]⁺ (calcd for C₉₀H₁₀₂O₂₂Na, 1557.6760).

### (3,4-Di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-2-O-levulinoyl-α-L-rhamnopyranosyl N-(phenyl)trifluoroacetimidate (4).

To a solution of **58** (940 mg, 0.612 mmol) in acetone (6.1 mL) was added *N*-(phenyl)trifluoroacetimidoyl chloride⁹⁷ (254 mg, 1.22 mmol, 2.0 equiv) followed by Cs₂CO₃ (219 mg, 0.673 mmol, 1.1 equiv). The mixture was stirred for 3 h at room temperature, then filtered and evaporated to dryness. The resulting residue was purified by flash chromatography (Tol/EtOAc 95:5 to 9:1 + 1% Et₃N) to furnish **4** (971 mg, 93%) as a white foam. *R_{f}* 0.47 (Chex/EtOAc 7:3); [α]²⁵_{D} +11° (c 1.0, CHCl₃); ¹H NMR (Pyr-d₅, 400 MHz) δ: 7.67-7.10 (m, 45H, C*H*_{PH}), 7.00 (s, *J*_{1,2} = 7.1 Hz, 1H, H-1_{C}), 6.09 (br s, 1H, H-2_{A}), 5.89 (br s, 1H, H-2_{C}), 5.72-5.68 (m, 2H, H-1_{A}, H-1_{B}), 5.25-5.07 (m, 5H, C*H*₂Ph), 5.02-4.77 (m, 11H, C*H*₂Ph (10H), H-2_{B}), 4.73 (d, *J* = 12.0 Hz, 1H, C*H*₂Ph), 4.70-4.65 (m, 1H, H-3_{C}), 4.61 (d, *J* = 11.2 Hz, 1H, C*H*₂Ph), 4.45-4.08 (m, 10H, H-5_{E}, H-5_{A}, H-3_{A}, H-3_{E}, H-3_{B}, H-4_{C}, H-5_{C}, H-5_{B}, H-6a_{E}, H-6b_{E}), 4.01 (t, *J*_{3,4} = *J*_{4,5} = 9.5 Hz, 1H, H-4_{E}), 3.95 (t, *J*_{3,4} = *J*_{4,5} = 9.3 Hz, 1H, H-4_{B}), 3.90-3.83 (m, 2H, H-4_{A}, H-2_{E}), 2.90-2.70 (m, 8H, 4×C*H*_{2Lev}), 2.03 (s, 3H, C(O)C*H*₃), 2.02 (s, 3H, C(O)C*H*₃), 1.64 (d, *J*_{5,6} = 5.5 Hz, 3H, H-6_{C}), 1.53 (d, *J*_{5,6} = 6.1Hz, 3H, H-6_{B}), 1.52 (d, *J*_{5,6} = 6.0 Hz, 3H, H-6_{A}). ¹³C NMR (Pyr-d₅, 100 MHz) δ: 206.3 (*C*(O)CH_{2Lev}), 206.0 (*C*(O)CH_{2Lev}), 172.9 (*C*=O), 172.8 (*C*=O), 144.3 (*C*=NPh), 140.2-139.2 (P_{Ph}), 129.7-128.2 (*C*H_{Ph}), 120.4 (C-1_{c}), 102.4 (C-1_{B}), 100.5 (C-1_{A}), 99.0 (C-1_{E}), 82.5 (C-3_{E}), 82.1 (C-2_{E}), 81.0 (C-4_{A}), 80.6 (C-4_{B}), 79.9 (C-3_{C}), 79.8 (C-3_{B}), 78.8 (C-3_{A}), 78.5 (C-4_{E}), 77.9 (C-4_{C}), 76.0 (*C*H₂Ph), 75.9 (*C*H₂Ph), 75.8 (C-2_{B}), 75.6 (*C*H₂Ph), 75.5 (*C*H₂Ph), 74.3 (*C*H₂Ph), 73.8 (CH₂Ph), 72.5 (C-5_{E}), 72.0 (CH₂Ph), 71.6 (C-2_{C}), 71.6 (*C*H₂Ph), 71.4 (C-5_{C}), 70.1 (C-2_{A}), 70.0 (C-5_{B}), 69.5 (C-6_{E}), 69.3 (C-5_{A}), 38.4 (*C*H_{2Lev}), 38.3 (*C*H_{2Lev}), 29.9 (C(O)*C*H₃), 29.8 (C(O)CH₃), 28.9 (*C*H_{2Lev}), 28.8 (*C*H_{2Lev}), 19.6 (C-6_{C}), 18.9 (C-6_{B}*), 18.7 (C-6_{A}*).

### 2-Azidoethyl (3,4-di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-(2-acetamido-2-deoxy-4,6-O-isopropylidene-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-2-acetamido-4,6-di-O-benzyl-2-deoxy-β-D-glucopyranoside(59).

The acceptor **56** (330 mg, 0.153 mmol) and the donor **4** (430 mg, 0.252 mmol, 1.65 equiv) were dissolved in anhydrous DCE (9.9 mL). The mixture was stirred for 1h at room temperature under an argon atmosphere in the presence of powdered 4 Å molecular sieves (854 mg). The temperature was lowered to -10 °C (acetone/ice water), then TBSOTf (21 µL, 0.092 mmol, 0.6 equiv) was injected keeping rigorous anhydrous conditions. The mixture was stirred for 3 h at -10 °C to room temperature under an argon atmosphere, then quenched with Et₃N (100µL). The solution was filtered and the solvents were evaporated under reduced pressure. The resulting residue was purified by flash chromatography (Chex/EtOAc 6:4 to 3:7 + 1% Et₃N) to give **59** (412 mg, 73%) as a white foam. [α]²⁵_{D} -18° (c 0.34, CHCl₃); ¹H NMR (Pyr-d₅, 400 MHz) δ: 9.56 (m, 2H, 2×N*H*), 7.69-7.25 (m, 87H, C*H*_{PH}), 7.00-6.96 (m, 2H, C*H*_{Ph}), 6.11 (br s, 1H, H-2_{A'}), [5.77-5.38 (11H, H-1_{A}, H-1_{A'}, H-1_{B,} H-1_{B'}, H-1_{C}, H-1_{C'}, H-1_{D'}, H-1_{E}, H-1_{E'}, H-2_{C}, H-2_{C'}) 5.77 (br s, 1H), 5.74-5.68 (m, 4H), 5.65-5.60 (m, 2H), 5.57 (d, *J*_{1,2} = 5.6 Hz, 1H), 5.54 (br s, 1H), 5.49 (br s, 1H), 5.3 8 (br s, 1H)], 5.32-3.74 (m, 78H, H-2_{A}, H-3_{A}, H-3_{A'}, H-4_{A}, H-4_{A'}, H-5_{A}, H-5_{A'}, H-2_{B}, H-2_{B'}, H-3_{B}, H-3_{B'}, H-4_{B}, H-4_{B'}, H-5_{B}, H-5_{B'}, H-3_{C}, H-3_{C}, H-4_{C}, H-4_{C'}, H-5_{C}, H-5_{C'}, H-1_{D}, H-2_{D}, H-3_{D}, H-3_{D'}, H-6a_{D}, H-6b_{D}, H-6a_{D'}, H-2_{E}, H-2_{E'}, H-3_{E}, H-3_{E'}, H-4_{E}, H-4_{E'}, H-5_{E}, H-5_{E'}, H-6a_{E}, H-6b_{E}, H-6a_{E'}, H-6b_{E'}, OC*H*₂a, OC*H*₂b, 18×C*H*₂Ph), 3.74-3.43 (m, 10H, H-2_{D'}, H-4_{D}, H-4_{D'}, H-5_{D}, H-5_{D'}, H-6b_{D'}, OC*H*₃, C*H*₂N₃a), 3.41-3.33 (m, 1H, C*H*₂N₃b), 2.87-2.62 (m, 12H, 6×C*H*_{2Lev}), 2.44 (s, 3H, C(O)C*H*_{3Ac}), 2.38 (s, 3H, C(O)C*H*_{3Ac}), 2.03 (s, 3H, C(O)C*H*_{3Lev}), 2.01 (s, 3H, C(O)C*H*_{3Lev}), 2.00 (s, 3H, C(O)C*H*_{3Lev}), 1.68 (d, *J*_{5,6} = 6.0 Hz, 3H, C*H*_{3Rha}), 1.55 (d, *J*_{5,6} = 5.8 Hz, 3H, C*H*_{3Rha}), 1.50 (d, *J*_{5,6} = 6.1 Hz, 3H, C*H*_{3Rha}), 1.46 (s, 3H, C*H*_{3Iso}), 1.44-1.36 (m, 9H, 3×C*H*_{3Rha}), 1.23 (s, 3H, C*H*_{3Iso}). ¹³C NMR (Pyr-d₅, 100 MHz) δ (partial): 206.1 (*C*(O)CH_{2Lev}), 206.0 (2×*C*(O)CH_{2Lev}), 173.1 (*C*=O_{Lev}), 173.0 (*C*=O_{Lev}), 172.7 (*C*=O_{Lev}), 171.9 (*C*(O)CH_{3Ac}), 171.8 (*C*(O)CH_{3Ac}), 160.2 (C_{Ph}), 140.3-139.4 (C_{Ph}), 131.1-128.1 (C*H*_{Ph}), 114.7 (C*H*_{Ph}), 102.6, 102.4, 102.2, 101.1, 100.4, 100.4, 99.3, 98.5, 98.1, 98.0 (C-1_{A}, C-1_{A'}, C-1B, C-1_{B'}, C-1_{C}, C-1_{C'}, C-1_{D}, C-1_{D'}, C-1_{E}, C-1_{E'}) 99.9 (*C*(CH₃)₂), 82.7, 82.6, 82.3, 81.8, 81.2, 80.9, 80.3, 79.9, 79.7, 79.4, 78.8, 78.6, 76.9, 76.3, 76.2, 76.0, 76.0, 75.8, 75.6, 75.4, 75.0, 74.3, 74.1, 74.0, 73.9, 73.8, 73.6, 72.9, 72.7, 72.4, 72.0, 71.9, 71.0, 70.3, 69.9, 69.8, 69.7, 69.6, 69.3, 68.6, 68.5, 68.2, 67.9 (C-2_{A}, C-2_{A'}, C-3_{A}, C-3_{A'}, C-4_{A}, C-4_{A'}, C-5_{A}, C-5_{A'}, C-2_{B}, C-2_{B'}, C-3_{B}, C-3_{B'}, C-4_{B}, C-4_{B'}, C-5_{B}, C-5_{B'}, C-2_{C}, C-2_{C'}, C-3_{C}, C-3_{C'}, C-4_{C}, C-4_{C'}, C-5_{C}, C-5_{C'}, C-3_{D}, C-3_{D'}, C-4_{D}, C-4_{D'}, C-5_{D}, C-5_{D'}, C-6_{D}, C-2_{E}, C-2_{E'}, C-3_{E}, C-3_{E'}, C-4_{E}, C-4_{E'}, C-5_{E}, C-5_{E'}, C-6_{E}, C-6_{E'}, 18×CH₂Ph), 63.0 (C-6_{D'}), 59.6 (C-2_{D'}), 58.3 (C-2_{D}), 55.5 (OCH₃), 51.6 (CH₂N₃), 38.6, 38.5, 38.5 (3×*C*H_{2Lev}), 29.9 (3C, 3×C(O)*C*H_{3Lev}), 29.2, 29.1, 29.0 (3×*C*H_{2Lev}), 24.4, 24.2 (2×C(O)*C*H_{3Ac}), 19.8, 19.8, 19.5, 19.5, 19.1 (2C), 19.0, 18.9 (6x*C*H_{3Rha}, 2×*C*H_{3Iso}). HR-ESI-TOF-MS *m*/*z* 1840.38 [M + 2H]²⁺ (calcd for C₂₁₁H₂₄₅N₅O₅₂, 1840.33).

### 2-Azidoethyl (3,4-di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-(2-acetamido-2-deoxy-4,6-O-isopropylidene-β-D-glucopyranosyl)-(1→2)-(4-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-2-acetamido-4,6-di-O-benzyl-2-deoxy-β-D-glucopyranoside (60).

To a solution of **59** (313 mg, 85.0 µmol) in anhydrous CH₂Cl₂ (4.3 mL) was added H₂O (425 µL) followed by DDQ (39 mg, 170 µmol, 2.0 equiv). The mixture was stirred for 1.5 h at room temperature under an argon atmosphere. Then, the reaction was quenched with saturated aqueous NaHCO₃ (25 mL) and the aqueous phase was extracted with CH₂Cl₂ (3x25 mL). The solvents of the dried (anhydrous Na₂SO₄) solution were removed under reduced pressure and the resulting residue was purified by flash chromatography (CH₂Cl₂/MeOH 99:1 to 98:2 + 0.5% Et₃N) to furnish **60** (211 mg, 70%) as a white foam along with unreacted **59** (40 mg, 13%). *R*_{f} 0.61 (CH₂Cl₂/MeOH 95:5); [α]²⁵_{D} -22° (c 0.32, CHCl₃); ¹H NMR (Pyr-d₅, 400 MHz) δ: 9.44 (d, *J* = 7.6 Hz, 1H, N*H*), 9.35 (br s, 1H, N*H*), 7.70-7.25 (m, 85H, C*H*_{Ph}), 6.63 (br s, 1H, O*H*), 6.09 (dd, *J*_{2,3} = 2.7 Hz, *J*_{1,2} = 1.8 Hz, 1H, H-2_{A'}), 5.78-5.40 (11H, H-1_{A}, H-1_{A'}, H-1_{B}, H-1_{B'}, H-1_{C}, H-1_{C'}, H-1_{D'}, H-1_{E}, H-1_{E'}, H-2_{C}, H-2_{C'}), 5.36-3.63 (m, 79H, H-2_{A}, H-3_{A}, H-3_{A'}, H-4_{A}, H-4_{A'}, H-5_{A}, H-5_{A'}, H-2_{B}, H-2_{B'}, H-3_{B}, H-3_{B'}, H-4_{B}, H-4_{B'}, H-5_{B}, H-5_{B'}, H-3_{C}, H-3_{C'}, H-4_{C}, H-4_{C'}, H-5_{C}, H-5_{C'}, H-1_{D}, H-2_{D}, H-2D', H-3_{D}, H-3_{D'}, H-4_{D}, H-5_{D}, H-6a_{D}, H-6b_{D}, H-6a_{D'}, H-2_{E}, H-2_{E'}, H-3_{E}, H-3_{E'}, H-4_{E}, H-4_{E'}, H-5_{E}, H-5_{E'}, H-6a_{E}, H-6b_{E}, H-6a_{E'}, H-6b_{E'}, OC*H*₂a, OC*H*₂b, 17×C*H*₂Ph), 3.54-3.32 (m, 5H, H-4_{D'}, H-5_{D'}, H-6b_{D'}, C*H*₂N₃a, C*H*₂N₃b), 2.87-2.62 (m, 12H, 6×C*H*_{2Lev}), 2.50 (s, 3H, C(O)C*H*_{3Ac}), 2.36 (s, 3H, C(O)C*H*_{3Ac}), 2.02 (s, 3H, C(O)C*H*_{3Lev}), 2.02 (s, 3H, C(O)C*H*_{3Lev}), 2.00 (s, 3H, C(O)C*H*_{3Lev}), 1.68 (d, *J*_{5,6} = 6.1 Hz, 3H, C*H*_{3Rha}), 1.53 (d, *J*_{5,6} = 6.2 Hz, 3H, C*H*_{3Rha}), 1.51-1.43 (m, 12H, 3×C*H*_{3Rha}, C*H*_{3Iso}), 1.41 (d, *J*_{5,6} = 6.1 Hz, 3H, C*H*_{3Rha}), 1.26 (s, 3H, C*H*_{3Iso}). ¹³C NMR (Pyr-d₅, 100 MHz) δ (partial): 206.3, 206.1, 206.0 (3×*C*(O)CH_{2Lev}), 173.1, 173.0, 172.7 (3×*C*=O_{Lev}), 171.9 (2C, 2×*C*(O)CH_{3Ac}), 140.2-139.3 (C_{Ph}), 129.3-128.0 (*C*H_{Ph}), 103.4, 102.5 (2C), 102.4, 101.0, 100.3, 99.2, 98.4, 98.1, 97.8 (C-1_{A}, C-1_{A'}, C-1_{B}, C-1_{B'}, C-1_{C}, C-1_{C'}, C-1_{D}, C-1_{D'}, C-1_{E}, C-1_{E'}), 83.2, 82.7, 82.6, 82.5, 82.2, 81.4, 81.0, 80.7, 80.4, 80.2, 79.9, 79.6, 78.7, 78.6, 78.5, 78.4, 77.0, 76.1, 75.9, 75.8, 75.7, 75.5, 75.4, 75.0, 74.2, 74.0, 73.9, 73.7, 73.6, 72.8, 72.4, 72.2, 71.9, 71.6, 70.7, 70.0, 69.7, 69.4, 69.2, 68.6, 68.5, 68.1, 67.8 (C-2_{A}, C-2_{A'}, C-3_{A}, C-3_{A'}, C-4_{A}, C-4_{A'}, C-5_{A}, C-5_{A'}, C-2_{B}, C-2_{B'}, C-3_{B}, C-3_{B'}, C-4_{B}, C-4_{B'}, C-5_{B}, C-5_{B'}, C-2_{C}, C-2_{C'}, C-3_{C}, C-3_{C'}, C-4_{C}, C-4_{C'}, C-5_{C}, C-5_{C'}, C-3_{D}, C-3_{D'}, C-4_{D}, C-4_{D'}, C-5_{D}, C-5_{D'}, C-6_{D}, C-2_{E}, C-2_{E'}, C-3_{E}, C-3_{E'}, C-4_{E}, C-4_{E'}, C-5_{E}, C-5_{E'}, C-6_{E}, C-6_{E'}, 17×CHPh₂), 62.9 (C-6_{D'}), 58.9 (C-2_{D'}), 58.2 (C-2_{D}), 51.5 (CH₂N₃), 38.4, 38.4, 38.3 (3×*C*H_{2Lev}), 29.9, 29.8, 29.8 (3×C(O)*C*H_{3Lev}), 29.0, 28.9, 28.9 (3×*C*H_{2Lev}), 24.4, 24.2 (2×C(O)*C*H_{3Ac}), 19.8, 19.6, 19.5, 19.0, 18.9 (3C), 18.8 (6×*C*H_{3Rha}, 2×*C*H_{3Iso}). HR-ESI-TOF-MS *m*/*z* 1780.38 [M + 2H]²⁺ (calcd for C₂₀₃H₂₃₇N₅O₅₁, 1780.31 ).

### 2-Azidoethyl (3,4-di-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-(2-acetamido-2-deoxy-β-D-glucopyranosyl)-(1→2)-(3-O-acetyl-4-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-2-acetamido-4,6-di-O-benzyl-2-deoxy-β-D-glucopyranoside (61).

To a solution of **60** (200 mg, 56.2 µmol) in anhydrous Py (5.6 mL) was added Ac₂O (531 µL, 5.62 mmol, 100 equiv) followed by DMAP (7.0 mg, 56 µmol, 1.0 equiv). The mixture was stirred for 1 h at room temperature under an argon atmosphere, then the solvents were removed under reduced pressure and co-evaporated three times with toluene. The resulting residue (202 mg, 56.2 µmol) was dissolved in CH₂Cl₂ (8.4 mL), the temperature was lowered to 0 °C, then a solution of 50% aqueous TFA (1.8 mL) was added dropwise. The mixture was stirred for 2 h at 0 °C to room temperature. The solution was diluted with CH₂Cl₂ (10 mL) and the excess of TFA was carefully neutralized with saturated aqueous NaHCO₃ (25 mL). The aqueous phase was extracted with CH₂Cl₂ (3x25 mL), the pooled organic layers were washed with brine (25 mL), dried over anhydrous Na₂SO₄, filtered and the solvents were removed under reduced pressure. The resulting residue (200 mg, 56.2 µmol) was dissolved in anhydrous Py/HOAc (8.5 mL, 3:2 v/v), then hydrazine hydrate (35 µL, 1.12 mmol, 20 equiv) was added. The mixture was stirred for 2 h at room temperature under an argon atmosphere. The solution was diluted with CH₂Cl₂ (25 mL), the organic layer was washed with H₂O (25 mL), the aqueous phase was extracted with CH₂Cl₂ (3x25 mL), then the solvents were removed under reduced pressure and co-evaporated three times with toluene. The resulting residue was purified by flash chromatography (CH₂Cl₂/MeOH 99:1 to 98:2) to afford **61** (145 mg, 79%, 3 steps) as a white crystalline powder. *R*_{f} 0.54 (CH₂Cl₂/MeOH 95:5); [a]²⁵D -55° (*c* 0.20, CHCl₃); ¹H NMR (Pyr-d₅, 400 MHz) δ: 9.53 (d, *J_{NH,2} =* 7.2 Hz, 1H, N*H*Ac), 9.40 (d, *J_{NH,2} =* 8.5 Hz, 1H, N*H*Ac), 7.70-7.14 (m, 85H, C*H*_{Ph}), 6.01 (dd, *J*_{3,4} = 9.8 Hz, *J*_{2,3} = 2.4 Hz, 1H, H-3_{A}), 5.95-5.40 (9H, H-₁A, H-1_{A'}, H-1_{B}, H-1_{B'}, H-1_{C,} H-1_{C'}, H-1_{D'}, H-1_{E}, H-1_{E'}), 5.34-3.62 (m, 84H, H-2_{A}, H-2_{A'}, H-3_{A'}, H-4_{A}, H-4A', H-5_{A}, H-5_{A'}, H-2_{B}, H-2_{B'}, H-3_{B}, H-3_{B'}, H-4_{B}, H-4_{B'}, H-5_{B}, H-5_{B'}, H-2_{C}, H-2_{C'}, H-3_{C}, H-3_{C'}, H-4_{C}, H-4_{C'}, H-5_{C}, H-5_{C'}, H-1_{D}, H-2_{D}, H-2_{D'}, H-3_{D}, H-3_{D'}, H-4_{D}, H-4_{D'}, H-5_{D}, H-5_{D'}, H-6a_{D}, H-6a_{D'}, H-6b_{D}, H-6b_{D'}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₂a, OC*H*₂b, 17×C*H*₂Ph), 3.49-3.41 (high-order m, 1H, C*H*₂N₃a), 3.34-3.27 (high-order m, 1H, C*H*₂N₃b), 2.21 (s, 3H, C(O)C*H*_{3Ac}), 2.15 (s, 3H, C(O)C*H*_{3NHAc}), 2.09 (s, 3H, C(O)C*H*_{3NHAc}), 1.68 (d, *J*₅,₆ = 6.1 Hz, 3H, C*H*_{3Rha}), 1.54-1.42 (m, 15H, 5×C*H*_{3Rha}). ¹³C NMR (Pyr-d₅, 100 MHz) δ (partial): 171.1, 171.0, 170.9 (3×C(O)CH₃), 140.3-139.2 (C_{Ph}), 129.4-127.9 (*C*H_{Ph}), 103.9, 103.1, 102.8, 102.5, 102.3 (2C), 102.2, 101.5, 98.9, 98.4 (C-1_{A}, C-1_{A'}, C-1_{B}, C-1_{B'}, C-1_{C}, C-1_{C'}, C-1_{D}, C-I_{D'}, C-1_{E}, C-1_{E'}), 83.6, 83.2, 82.6, 82.5, 82.3, 82.2, 81.5, 81.4, 81.3, 81.1, 80.5, 80.3, 79.8, 78.9, 78.7, 78.5, 78.0, 77.8, 76.9, 76.0, 75.8, 75.8, 75.7, 75.5, 75.4, 75.3, 73.9, 73.8, 73.8, 73.7, 73.5, 72.7, 72.3, 72.1, 71.8, 71.7, 71.5, 71.0, 70.9, 69.9, 69.6, 69.5, 69.4, 69.2, 69.1, 68.9, 68.2 (C-2_{A}, C-3_{A}, C-4_{A}, C-5_{A}, C-2_{B}, C-3_{B}, C-4_{B}, C-5_{B}, C-2c, C-3_{C}, C-4_{C}, C-5_{C}, C-3_{D}, C-4_{D}, C-5_{D}, C-6_{D}, C-2_{E}, C-3_{E}, C-4_{E}, C-5_{E}, C-6_{E}, C-2_{A'}, C-3_{A'}, C-4_{A'}, C-5_{A'}, C-2_{B'}, C-3_{B'}, C-4_{B'}, C-5_{B'}, C-2_{C'}, C-3_{C'}, C-4_{C'}, C-5_{C'}, C-3_{D'}, C-4_{D'}, C-5_{D'}, C-2_{E'}, C-3_{E'}, C-4_{E'}, C-5_{E'}, C-6_{E'}, O*C*H₂, 17×*C*H_{2Ph}), 63.1 (C-6D'), 59.2 (C-2_{D'}), 57.5 (C-2_{D}), 51.4 (*C*H₂N₃), 24.0 (*C*(O)CH_{3NHAc}), 23.8 (*C*(O)CH_{3NHAc}), 21.4 (*C*(O)CH3Ac), 19.7, 19.6, 19.1, 18.9, 18.8, 18.7 (6×*C*H_{3Rha}). HR-ESI-TOF-MS *m*/*z* 1634.33 [M + 2H]²⁺ (calcd for C₁₈₇H₂₁₇N₅O₄₆, 1634.**24**).

### 2-Azidoethyl (3,4-di-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-(2-acetamido-6-O-acetyl-2-deoxy-β-D-glucopyranosyl)-(1→2)-(3-O-acetyl-4-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-2-acetamido-4,6-di-O-benzyl-2-deoxy-β-D-glucopyranoside (62).

To a solution of **60** (202 mg, 56.7 µmol) in anhydrous Py (2.8 mL) was added Ac₂O (536 µL, 5.67 mmol, 100 equiv) followed by DMAP (7.0 mg, 56 µmol, 1.0 equiv). The mixture was stirred for 1 h at room temperature under an argon atmosphere, then the solvents were removed under reduced pressure and co-evaporated three times with toluene. The resulting residue (204 mg, 56.7 µmol) was dissolved in CH₂Cl₂ (8.5 mL), the temperature was lowered to 0 °C, then a solution of 50% aqueous TFA (1.8 mL) was added dropwise. The mixture was stirred for 2 h at 0 °C to room temperature. The solution was diluted with CH₂Cl₂ (10 mL) and the excess of TFA was carefully neutralized with saturated aqueous NaHCO₃ (25 mL). The aqueous phase was extracted with CH₂Cl₂ (3×25 mL), the pooled organic layers were washed with brine (25 mL), dried over anhydrous Na₂SO₄, filtered and the solvents were removed under reduced pressure. The resulting residue (202 mg, 56.7 µmol) was dissolved in *sym*-collidine (2.2 mL) and the temperature was lowered to -45 °C (CH₃CN/CO₂). Then, chloroacetyl chloride (113 µmol, 100 µL of stock solution, 2.0 equiv) was added from a fresh chloroacetyl chloride stock solution (80 µL of chloroacetyl chloride in 1.0 mL anhydrous CH₂Cl₂). Additional chloroacetyl chloride (57 µmol, 50 µL of stock solution, 1.0 equiv) were added after 60, 120, 180 and 240 min. During this time the reaction was stirred under an argon atmosphere and the temperature was gradually raised from -45 °C to room temperature. After completion of the reaction (6 h), the excess of chloroacetyl chloride was quenched with MeOH, CH₂Cl₂ was added and the organic phase was washed with 5% aqueous HCl (1×), saturated NaHCO₃ (1×) and brine (1×). The solvents of the dried (anhydrous Na₂SO₄) solution were evaporated under reduced pressure and co-evaporated three times with toluene. The resulting residue (204 mg, 56.7 µmol) was dissolved in anhydrous Py/HOAc (8.5 mL, 3:2 v/v), then hydrazine hydrate (35 µL, 1.12 mmol, 20 equiv) was added. The mixture was stirred for 2 h at room temperature under an argon atmosphere. The solution was diluted with CH₂Cl₂ (25 mL), the organic layer was washed with H₂O (25 mL), the aqueous phase was extracted with CH₂Cl₂ (3x25 mL), then the solvents were removed under reduced pressure and co-evaporated three times with toluene. The resulting residue was purified by flash chromatography (CH₂Cl₂/MeOH 99:1 to 98:2) to afford **62** (138 mg, 74%, 4 steps) as a white crystalline powder. *R*_{f} 0.63 (CH₂Cl₂/MeOH 95:5); [α]²⁵D +15° (*c* 0.40, CHCl₃) ¹H NMR (Pyr-d₅, 400 MHz) δ: 9.59 (d, J_{N*H*},₂ = 7.3 Hz, 1H, N*H*Ac), 9.25 (d, *J_{NH},₂ =* 8.5 Hz, 1H, N*H*Ac), 7.69-7.17 (m, 85H, C*H*_{Ph}), 5.98 (dd, *J*_{3,4} = 9.9 Hz, *J*_{2,3} = 2.7 Hz, 1H, H-3_{A}), 5.86-5.47 (m, 9H, H-1_{A}, H-1_{A'} H-1_{B}, H-1_{B'}, H-1_{C}, H-1_{C'}, H-1_{D'}, H-1_{E}, H-1_{E'}), 5.30-3.56 (m, 83H, H-2_{A}, H-2_{A'}, H-3_{A'}, H-4_{A}, H-4_{A'}, H-5_{A}, H-5_{A'}, H-2_{B}, H-2_{B'}, H-3_{B}, H-3_{B'}, H-4_{B}, H-4_{B}', H-5_{B}, H-5_{B'}, H-2_{C}, H-2_{C'}, H-3_{C}, H-3_{C'}, H-4_{C}, H-4_{C'}, H-5_{C}, H-5_{C'}, H-1_{D}, H-2_{D}, H-3_{D}, H-3_{D'}, H-4_{D}, H-4D_{'}, H-5_{D5}, H-5_{D'}, H-6a_{D}, H-6a_{D'}, H-6b_{D}, H-6b_{D'}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₂a, OC*H*₂b, 17×C*H*₂Ph), 3.60 (dd, *J*_{2,3} = 17.0 Hz, *J*_{1,2} = 8.3 Hz, 1H, H-2_{D'}), 3.48-3.41 (high-order m, 1H, C*H*₂N₃a), 3.32 (ddd, *J* = 13.2, 5.3, 3.5 Hz, 1H, C*H*₂N₃b), 2.26 (s, 3H, C(O)C*H*_{3Ac}), 2.18 (s, 3H, C(O)C*H*_{3NHAc}), 2.09 (s, 3H, C(O)C*H*_{3NHAc}), 1.73 (s, 3H, C(O)C*H*_{3Ac}), 1.65 (d, *J*_{5,6 =} 6.1 Hz, 3*H*, C*H*_{3Rha}), 1.54-1.40 (m, 15H, 5×C*H*_{3Rha}). ¹³C NMR (Pyr-d₅, 100 MHz) δ (partial): 171.2 (*C*(O)CH_{3Ac}), 171.0 (3×*C*(O)CH_{3Ac}), 140.4-139.3 (C_{Ph}), 129.4-127.9 (*C*H_{Ph}), 104.0, 103.2, 102.9, 102.6, 102.3, 102.2, 101.6 (2C), 99.0, 98.6 (C-1_{A}, C-1_{A'}, C-1_{B}, C-1_{B'}, C-1_{C}, C-1_{C'}, C-1_{D}, C-1_{D'}, C-1_{E}, C-1_{E'}), 83.4, 82.6, 82.6, 82.3, 81.9, 81.6, 81.6, 81.2, 80.7, 80.5, 80.3, 79.6, 78.9, 78.8, 78.6, 77.2, 77.0, 76.8, 76.1, 76.1, 76.1, 76.0, 75.8, 75.8, 75.7, 75.6, 75.5, 75.1, 74.9, 74.5, 74.4, 74.0, 73.9, 73.9, 73.8, 73.7, 72.9, 72.4, 72.2, 71.9, 71.8, 71.6, 71.0, 70.4, 70.0, 69.6, 69.5, 69.4, 69.3, 69.2, 69.1, 68.3 (C-2_{A}, C-2_{A'}, C-3_{A}, C-3_{A'}, C-4_{A}, C-4_{A'}, C-5_{A}, C-5_{A'}, C-2_{B}, C-2_{B'}, C-3_{B}, C-3_{B'}, C-4_{B}, C-4_{B'}, C-5_{B}, C-5_{B'}, C-2_{C}, C-2_{C'}, C-3_{C}, C-3_{C'}, C-4_{C}, C-4_{C'}, C-5_{C}, C-5_{C'}, C-3_{D}, C-3_{D'}, C-4_{D}, C-4_{D'}, C-5_{D}, C-5_{D'}, C-6_{D}, C-2_{E}, C-2_{E'}, C-3_{E}, C-3_{E'}, C-4_{E}, C-4_{E'}, C-5_{E}, C-5_{E'}, C-6_{E}, C-6_{E'}, O*C*H₂), 59.7 (C-2_{D'}), 57.5 (C-2_{D}), 51.5 (*C*H₂N₃), 24.1 (*C*(O)CH_{3NHAc}), 23.9 (*C*(O)CH_{3NHAc}), 21.5 (*C*(O)CH_{3Ac}), 21.0 (*C*(O)CH_{3Ac}), 19.7, 19.7, 19.2, 18.9 (2C), 18.8 (6×*C*H_{3Rha}). HR-MALDI-TOF-MS *m*/*z* 3331.47489 [M + Na]⁺ (calcd for C₁₈₇H₂₁₇N₅O₄₇Na, 3331.46361).

### 2-Azidoethyl (3,4-di-O-benzyl-2-O-levulinoyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-(2-acetamido-2-deoxy-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(2-O-levulinoyl-α-L-rhamnopyranosyl)-(1→3)-2-acetamido-4,6-di-O-benzyl-2-deoxy-β-D-glucopyranoside (63).

To a solution of **59** (210 mg, 57.0 µmol) in CH₂Cl₂ (8.6 mL) was added 50% aqueous TFA (1.8 mL) at 0 °C. The mixture was stirred for 2 h from 0 °C to room temperature, then diluted with CH₂Cl₂ (10 mL) and washed with saturated NaHCO₃ (1×25 mL). The aqueous phase was extracted with CH₂Cl₂ (3x25 mL), the pooled organic layers were washed with brine (25 mL), dried over anhydrous Na₂SO₄, filtered and the solvents were evaporated to dryness. The crude was purified by flash chromatography (CH₂Cl₂/MeOH 99:1 to 98:2) to give **63** (187 mg, 90%) as a white powder. [α]²⁵D -36° (c 0.22, CHCl₃); ¹H NMR (Pyr-d₅, 400 MHz) δ: 9.54 (br s, 1H, N*H*), 7.77-7.09 (m, 87H, C*H*_{Ph}), 6.98-6.94 (m, 2H, C*H*_{Ph}), 6.12 (br s, 1H, H-2_{A'}), 5.86-5.25 (m, 12H, H-2_{C}, H-2_{C'}, H-1A, H-1A_{'}, H-1_{B}, H-1_{B'}, H-1_{C}, H-1_{C'}, H-1_{D}, H-1_{D'}, H-1E_{,} H-1_{E'}), 5.24-3.53 (m, 86H, H-2_{A}, H-3_{A}, H-3_{A'}, H-4_{A}, H-4_{A'}, H-5_{A}, H-5_{A'}, H-2_{B}, H-2_{B',} H-3_{B}, H-3_{B'}, H-4_{B}, H-4_{B'}, H-5_{B}, H-5_{B'}, H-3_{C}, H-3_{C'}, H-4_{C}, H-4_{C'}, H-5_{C}, H-5_{C'}, H-2_{D}, H-2_{D'}, H-3_{D}, H-3D', H-4_{D}, H-4D_{'}, H-5_{D}, H-5_{D'}, H-6a_{D}, H-6a_{D'}, H-6b_{D}, H-6b_{D'}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₃, OC*H*₂a, OC*H*₂b, 18×C*H*₂Ph), 3.53-3.44 (high-order m, 1H, C*H*₂N₃a), 3.41-3.33 (high-order m, 1H, C*H*₂N₃b), 2.87-2.58 (m, 12H, 6×C*H*_{2Lev}), 2.46 (s, 3H, C(O)C*H*_{3NHAc}), 2.40 (s, 3H, C(O)C*H*_{3NHAc}), 2.04 (s, 3H, C(O)C*H*_{3Lev}), 2.01 (s, 3H, C(O)C*H*_{3Lev}), 1.99 (s, 3H, C(O)C*H*_{3Lev}), 1.71 (d, *J*_{5,6} = 5.9 Hz, 3H, C*H*_{3Rha}), 1.57 (d, *J*_{5,6} = 6.3 Hz, 3H, C*H*_{3Rha}), 1.51 (d, *J*_{5,6} = _{6.0} Hz, 3H, C*H*_{3Rha)}, 1.43-1.36 (m, 6H, 2×C*H*_{3Rha}), 1.30 (d, *J*_{5,6} = 5.8 Hz, 3H, C*H*_{3Rha}). ¹³C NMR (Pyr-d₅, 100 MHz) δ (partial): 206.2 (*C*(O)CH_{2Lev}), 206.0 (2×*C*(O)CH_{2Lev}), 173.0 (2×*C*(O)CH_{3Lev}), 172.7 (*C*(O)CH_{3Lev}), 172.1 (*C*(O)CH_{3NHAc}), 172.0 (*C*(O)CH_{3NHAc}), 160.2 (C_{Ph}), 140.3-139.2 (C_{Ph}), 130.9 (C*H*_{Ph}), 129.4-127.8 (C*H*_{Ph}), 114.6 (C*H*_{Ph}), 102.2 (5C), 101.1, 100.6, 99.2, 98.8, 98.5 (C-1_{A}, C-1_{A'}, C-1_{B}, C-1_{B'}, C-1_{C}, C-1_{C'}, C-1_{D}, C-1_{D'}, C-1_{E}, C-1_{E'}), 82.8, 82.6, 82.5, 81.6, 81.1, 80.9, 80.0, 79.8, 79.6, 79.4, 79.3, 78.7, 78.6, 78.6, 78.5, 78.1, 77.1, 76.4, 76.1, 76.0, 75.9, 75.8, 75.6, 75.0, 74.4, 74.2, 74.0, 73.8, 72.8, 72.5, 72.3, 72.0, 71.5, 71.3, 70.2, 70.0, 69.7, 69.5, 69.5, 69.2, 68.5, 68.4 (C-2_{A}, C-2_{A'}, C-3_{A}, C-3_{A'}, C-4_{A}, C-4_{A'}, C-5_{A}, C-5_{A'}, C-2_{B}, C-2_{B'}, C-3_{B}, C-3_{B'}, C-4_{B}, C-4_{B'}, C-5_{B}, C-5_{B'}, C-2_{C}, C-2_{C'}, C-3_{C}, C-3_{C'}, C-4_{C}, C-4_{C'}, C-5_{C}, C-5_{C'}, C-3_{D}, C-3_{D'}, C-4_{D}, C-4_{D'}, C-5_{D}, C-5_{D'}, C-6_{D}, C-2_{E}, C-2_{E'}, C-3_{E}, C-3_{E'}, C-4_{E}, C-4_{E'}, C-5_{E}, C-5_{E'}, C-6_{E}, C-6_{E'}, OCH₂, 18×CH₂Ph), 63.6 (C-6_{D'}), 59.6 (C-2_{D'}), 58.2 (C-2_{D}), 55.5 (OCH₃), 51.6 (*C*H₂N₃), 38.5, 38.5, 38.4 (3×*C*H_{2Lev}), 29.9, 29.9, 29.8 (3×C(O)*C*H_{3Lev}), 29.1, 29.1, 29.0 (3×*C*H_{2Lev}), 24.5, 24.2 (2×C(O)*C*H_{3NHAc}), 19.5-18.8 (6×*C*H_{3Rha}). HR-MALDI-TOF-MS *m*/*z* 3661.61385 [M + Na]⁺ (calcd for C₂₀₈H₂₃₉N₅O₅₂Na, 3661.61034).

### 2-Azidoethyl (3,4-di-O-benzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-(2-acetamido-6-O-acetyl-2-deoxy-β-D-glucopyranosyl)-(1→2)-(4-O-benzyl-3-O-para-methoxybenzyl-α-L-rhamnopyranosyl)-(1→2)-(3,4-di-O-benzyl-α-L-rhamnopyranosyl)-(1→3)-[2,3,4,6-tetra-O-benzyl-α-D-glucopyranosyl-(1→4)]-(α-L-rhamnopyranosyl)-(1→3)-2-acetamido-4,6-di-O-benzyl-2-deoxy-β-D-glucopyranoside (64).

To a solution of **63** (138 mg, 37.9 µmol) in *sym*-collidine (1.5 mL) at -45 °C (CH₃CN/CO₂) was added chloroacetyl chloride (75.8 µmol, 100 µL of stock solution, 2.0 equiv) from a fresh chloroacetyl chloride stock solution (108 µL of chloroacetyl chloride in 2.0 mL anhydrous CH₂Cl₂). Additional chloroacetyl chloride (190 µmol, 250 µL of stock solution, 5.0 equiv) were added after 60, 120, 180, 240, 300 and 360 min. During this time the reaction was stirred under an argon atmosphere and the temperature was gradually raised from -45 °C to room temperature. After completion of the reaction (8 h), the excess of chloroacetyl chloride was quenched with MeOH, CH₂Cl₂ was added and the organic phase was washed with 10% aqueous HCl (1×). The aqueous layer was extracted with CH₂Cl₂ (3x) and the pooled organic phases were washed with saturated NaHCO₃ (1×) and brine (1×). The solvents of the dried (anhydrous Na₂SO₄) solution were evaporated under reduced pressure and co-evaporated three times with toluene. The resulting residue (140 mg, 37.9 µmol) was dissolved in anhydrous Py/HOAc (5.7 mL, 3:2 v/v), then hydrazine hydrate (24 µL, 0.76 mmol, 20 equiv) was added. The mixture was stirred for 2 h at room temperature under an argon atmosphere. The solution was diluted with CH₂Cl₂ (25 mL), the organic layer was washed with H₂O (25 mL), the aqueous phase was extracted with CH₂Cl₂ (3x25 mL), then the solvents were removed under reduced pressure and co-evaporated three times with toluene. The resulting residue was purified by flash chromatography (CH₂Cl₂/MeOH 99:1 to 98.5:1.5) to afford **64** (94 mg, 81%, 2 steps) as a white amorphous solid. [α]²⁵_{D} -4° (*c* 0.50, CHCl₃); ¹H NMR (Pyr-d₅, 400 MHz) δ: 9.20 (d, J_{NH,2} *=* 8.6 Hz, 1H, N*H*), 9.05 (d, J_{NH,2} = 7.7 Hz, 1H, N*H*), 7.72-7.20 (m, 87H, C*H*_{Ph}), 7.01-6.97 (m, 2H, C*H*_{Ph}), 5.87-5.48 (m, 9H, H-1_{A}, H-1_{A'}, H-1_{B}, H-1_{B'}, H-1_{C}, H-1_{C'}, H-1_{D'}, H-1_{E}, H-1_{E'}), 5.27-3.70 (m, 87H, H-2_{A}, H-2_{A'}, H-3_{A}, H-3_{A'}, H-4_{A}, H-4_{A'}, H-5_{A}, H-5_{A'}, H-2_{B}, H-2_{B'}, H-3_{B}, H-3_{B'}, H-4_{B}, H-4_{B'}, H-5_{B}, H-5_{B'}, H-2_{C}, H-2_{C'}, H-3_{C}, H-3_{C'}, H-4_{C}, H-4_{C'}, H-5_{C}, H-5_{C'}, H-1_{D}, H-2_{D}, H-2_{D'}, H-3_{D}, H-3_{D'}, H-4_{D}, H-4_{D'}, H-5_{D}, H-5_{D'}, H-6a_{D}, H-6a_{D'}, H-6b_{D}, H-6b_{D'}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₂a, OC*H*₂b, 18×C*H*2Ph), 3.65 (s, 3H, OC*H*₃), 3.49-3.41 (high-order m, 1H, C*H*₂N₃a), 3.33 (ddd, *J*= 13.1, 5.1, 3.7 Hz, 1H, C*H*₂N₃b), 2.10 (s, 3H, C(O)C*H*_{3NHAc}), 2.09 (s, 3H, C(O)C*H*_{3NHAc}), 1.79 (s, 3H, C(O)C*H*_{3Ac}), 1.66 (d, *J*_{5,6} = 6.1 Hz, 3H, C*H*_{3Rha}), 1.54-1.39 (m, 15H, 5×C*H*_{3Rha}). ¹³C NMR (Pyr-d₅, 100 MHz) δ (partial): 171.1, 171.0, 170.9 (3×C(O)CH_{3Ac}), 160.2 (C_{Ph}), 140.4-139.2 (C_{Ph}), 130.9 (CH_{Ph}), 129.5-127.8 (CH_{Ph}), 114.7 (CH_{Ph}), 104.0, 103.2, 102.8, 102.7, 102.5, 102.2, 102.0, 101.7, 99.0, 98.5 (C-1_{A}, C-1A_{'}, C-1_{B}, C-1_{B'}, C-1_{C}, C-1_{C'}, C-1_{D}, C-1_{D'}, C-1_{E}, C-1_{E'}), 84.0, 83.2, 82.6, 82.3, 81.9, 81.7, 81.6, 81.3, 80.6, 80.2, 79.5, 78.8, 78.7, 78.6, 77.0, 76.3, 76.1, 76.0, 75.9, 75.8, 75.6, 75.3, 73.9, 73.9, 73.8, 73.7, 72.9, 72.6, 72.3, 72.2, 71.9, 71.7, 70.9, 70.4, 70.1, 69.7, 69.5, 69.5, 69.3, 69.2, 68.3 (C-2_{A}, C-2_{A'}, C-3_{A}, C-3_{A'}, C-4_{A}, C-4_{A'}, C-5_{A}, C-5_{A'}, C-2_{B}, C-2_{B'}, C-3_{B}, C-3_{B'}, C-4_{B}, C-4_{B'}, C-5_{B}, C-5_{B'}, C-2_{C}, C-2_{C'}, C-3_{C}, C-3_{C'}, C-4_{C}, C-4_{C'}, C-5_{C}, C-5_{C'}, C-3_{D}, C-3_{D'}, C-4_{D}, C-4_{D'}, C-5_{D}, C-5_{D'}, C-6_{D}, C-2_{E}, C-2_{E'}, C-3_{E}, C-3_{E'}, C-4_{E}, C-4_{E'}, C-5_{E}, C-5_{E'}, C-6_{E}, C-6_{E'} OCH₂, 18×CH₂Ph), 64.7 (C-6_{D'}), 59.0 (C-2_{D'}), 57.4 (C-2_{D}), 55.6 (OCH₃), 51.5 (CH₂N₃), 24.1, 23.9 (2×C(O)C*H*_{3NHAc}), 21.0 (C(O)*C*H_{3Ac}), 19.9, 19.7, 19.3, 19.0 (2C), 18.9 (6×CH_{3Rha}). HR-MALDI-TOF-MS *m*/*z* 3409.50588 [M + Na]⁺ (calcd for C₁₉₅H₂₂₃N5O₄₇Na, 3409.51056).

### 2-Aminoethyl α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-2-deoxy-β-D-glucopyranosyl-(1→2)-3-O-acetyl-α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-2-deoxy-β-D-glucopyranoside (1).

A solution of **61** (20 mg, 6.1 µmol) in EtOH/HOAc (3.3 mL, 10:1 v/v) was passed through a 10% Pd/C cartridge (CatCart®30) using a H-Cube^{™} continuous flow hydrogenation system in the "Full-H₂" mode. The temperature was set to 60 °C and the flow rate was fixed to 0.5 mL/min. After 8 h of reaction, the cartridge was rinced with EtOH/H₂O (30 mL, 1:1 *v*/*v*) and the solvents were evaporated to dryness. The residue (14 mg) was purified by preparative RP-HPLC to furnish 1 (6.0 mg, 58%) as a white amorphous powder, HPLC (215 nm): *R*ₜ = 8.2 min (Kromasil 5 µm C₁₈ 100 Å 4.6 × 250 mm column; 0-25% linear gradient of CH₃CN in 0.01N aqueous TFA over 20 min at a flow rate of 5.5 mL·min⁻¹). ¹H NMR (D₂O, 400 MHz) δ: 5.20 (br d, *J*_{1,2} = 3.3 Hz, 2H, H-1_{E}, H-1_{E'}), 5.26 (br s, 1H, H-1_{A}), 5.08 (dd, *J*_{2,3} = 3.0 Hz, 1H, H-3_{A}), 5.06 (br s, 2H, H-1_{B}^{*}, H-1_{B'}^{*}), 4.97 (br s, 1H, H-1_{A'}), 4.88 (br s, 1H, H-1_{C}*), 4.82 (br s, 1H, H-1_{C'}*), 4.58 (d, *J*_{1,2} = 8.6 Hz, 1H, H-1_{D'}*), 4.53 (d, *J*_{1,2} = 8.1 Hz, 1H, H-1_{D}*), 4.25 (br s, 1H, H-2_{A}), 4.18-4.13 (m, 2H, H-5_{A}*, H-2_{B'}*), 4.07-4.02 (m, 2H, H-2_{B}*, O*CH*₂), 4.18-3.35 (m, 45H, H-2_{A'}, H-3_{A'}, H-4_{A}, H-4_{A'}, H-5_{A'}*, H-3_{B}, H-3_{B'}, H-4_{B}, H-4_{B'}, H-5_{B}*, H-5_{B}*, H-2_{C}, H-2_{C'}, H-3_{C}, H-3_{C'}, H-4_{C}, H-4_{C'}, H-5_{C}*, H-5_{C'}*, H-2_{D}, H-2_{D'}, H-3_{D}, H-3_{D'}, H-4_{D}, H-4_{D'}, H-5_{D}, H-5_{D'}, H-6a_{D}, H-6a_{D'}, H-6b_{D}, H-6b_{D'}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, *OCH₂*), 3.24-3.16 (m, 2H, C*H*₂NH₂), 2.21 (s, 3H, C(O)C*H*_{3Ac}), 2.11 (s, 3H, C(O)C*H*_{3NHAc}), 2.07 (s, 3H, C(O)C*H*_{3NHAc}), 1.37-1.26 (m, 18H, 6×C*H*_{3Rha}). ¹³C NMR (D₂O, 100 MHz) δ (partial): 174.5, 174.2, 173.7 (3×*C*(O)CH₃), 102.6 (C-1_{A'}), 102.4 (C-1_{D}*), 101.4 (2C, C-1_{B}, C-1_{B'}), 101.3 (C-1_{A}), 101.1, 100.8 (C-1_{C}, C-1_{C'}), 100.6 (C-1_{D'}*), 97.6 (C-1_{E}, C-1_{E'}), 81.4, 81.0, 79.5 (2C, C-1_{B}, C-1_{B'}), 79.3, 76.9 (C-2_{A}), 76.0, 75.6, 72.7, 72.1, 71.9, 71.4, 71.3, 70.8, 70.1, 69.8, 69.8, 69.4, 69.3, 69.1, 68.4, 68.1, 65.7 (OCH₂), 60.7, 60.6 (4C, C-6_{E}, C-6_{E'}, C-6_{D}, C-6_{D'}), 55.6, 55.1 (C-2_{D}, C-2_{D'}), 39.4 (*C*H₂NH₂), 22.4 (C(O)*C*H_{3NHAc}), 22.3 (C(O)*C*H_{3NHAc}), 20.7 (C(O)*C*H_{3Ac}), 17.9 (2C), 16.9 (2C), 16.7, 16.6 (6×*C*H_{3Rha}). HR-ESI-TOF-MS m/z 1710.6890 [M + H]⁺ (calcd for C₆₈H₁₁₆N₃O₄₆, 1710.6830).

### 2-Aminoethyl α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-6-O-acetyl-2-deoxy-β-D-glucopyranosyl-(1→2)-3-O-acetyl-α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-2-deoxy-β-D-glucopyranoside (2).

A solution of **62** (25 mg, 7.6 µmol) in EtOH/HOAc (3.3 mL, 10:1 v/v) was passed through a 10% Pd/C cartridge (CatCart®30) using a H-Cube^{™} continuous flow hydrogenation system in the "Full-H₂" mode. The temperature was set to 60 °C and the flow rate was fixed to 0.5 mL/min. After 8 h of reaction, the cartridge was rinced with EtOH/H₂O (30 mL, 1:1 v/v) and the solvents were evaporated to dryness. The residue (17 mg) was purified by preparative RP-HPLC to furnish **2** (9.9 mg, 75%) as a white amorphous powder; HPLC (215 nm): *R*ₜ = 4.0 min (Kromasil 5 µm C₁₈ 100 Å 4.6 × 250 mm column; 0-25% linear gradient of CH₃CN in 0.01 N aqueous TFA over 20 min at a flow rate of 5.5 mL·min⁻¹). ¹H NMR (D₂O, 400 MHz) δ: 5.20-5.19 (m, 2H, H-1_{E}, H-1_{E'}), 5.19 (br s, 1H, H-1_{A}), 5.10 (dd, *J*_{1,2}= 2.8 Hz, H-3_{A}), 5.07 (br s, 2H, H-1_{B}, H-1_{B'}), 4.97 (br s, 1H, H-1_{A'}), 4.88 (br s, 1H, H-1_{C}*), 4.80 (br s, 1H, H-1_{C'}*), 4.58 (d, *J*_{1,2} = 8.5 Hz, 1H, H-1_{D}*), 4.54 (d, *J*_{1,2} = 8.5 Hz, 1H, H-1_{D'}*), 4.40 (d, *J*_{6a,6b} = 11.9 Hz, H-6a_{D'}), 4.39 (dd, *J*_{6a,6b} = 11.9 Hz, *J*_{5,6b} = 4.3 Hz, 1H, H-6b_{D'}), 4.24 (bs, 1H, H-2_{A}), 4.16-4.12 (m, 2H, H-5_{C}*, H-5_{C'}*), 4.04-4.02 (m, 1H, *OCH₂*), 3.98-3.46 (m, 43H, H-2_{A'}, H-3_{A'}, H-4_{A}, H-4_{A'}, H-5_{A}*, H-5_{A'}*, H-2_{B}, H-2_{B'}, H-3_{B}, H-3_{B'}, H-4_{B}, H-4_{B'}, H-5_{B}*, H-5_{B'}*, H-2_{C}, H-2_{C'}, H-3_{C}, H-3_{C'}, H-4_{C}, H-4_{C'}, H-2_{D}, H-2_{D'}, H-3_{D}, H-3_{D'}, H-4_{D}, H-4_{D'}, H-5_{D}, H-5_{D'}, H-6a_{D}, H-6b_{D}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₂), 3.24-3.16 (m, 2H, C*H*₂NH₂), 2.21 (s, 3H, C(O)C*H*_{3Ac}), 2.15 (s, 3H, C(O)C*H*_{3Ac}), 2.11 (s, 3H, C(O)C*H*_{3NHAc}), 2.06 (s, 3H, C(O)C*H*_{3NHAc}), 1.35-1.26 (m, 18H, 6×C*H*_{3Rha}). ¹³C NMR (D₂O, 100 MHz) δ (partial): 174.5, 174.2, 174.1, 173.7 (4×*C*(O)CH₃), 102.6 (C-1_{A'}), 102.3 (C-1_{D}*), 101.3 (3×C, C-1_{A}, C-1_{B}, C-1_{B'}), 101.0, 100.8 (C-1_{C}, C-1_{C'}), 100.5 (C-1_{D'}*), 97.7 (2×C, C-1_{E}, C-1_{E'}), 81.4, 80.8, 79.8 (C-2_{B'}*), 79.6 (C-2_{B}*), 79.0, 76.9 (C-2_{A}), 75.9, 73.2, 72.7, 72.7, 72.1, 71.9, 71.4, 71.3, 70.1, 69.9, 69.8, 69.7, 69.4, 69.3, 69.1, 69.0, 68.4, 68.3, 65.7 (O*C*H₂), 63.5 (C-6_{D'}), 60.8 (C-6_{D}*), 60.7 (2×C, C-6_{E}*, C-6_{E'}*), 55.5 (C-2_{D}*), 55.1 (C-2_{D'}*), 39.5 (*C*H₂NH₂), 22.4 (C(O)*C*H_{3NHAc}), 22.3 (C(O)*C*H_{3NHAc}), 20.7 (C(O)*C*H_{3Ac}), 20.4 (C(O)*C*H_{3Ac}), 17.9, 17.9, 16.9, 16.8, 16.7, 16.6 (6×*C*H_{3Rha}). HR-ESI-TOF-MS m/z 1752.6924 [M + H]⁺ (calcd for C₇₀H₁₁₈N₃O₄₇, 1752.6936).

### 2-Aminoethyl α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-6-O-acetyl-2-deoxy-β-D-glucopyranosyl-(1→2)-α-L-rhamnopyranosyl-(1→2)-α-L-rhamnopyranosyl-(1→3)-[α-D-glucopyranosyl-(1→4)]-α-L-rhamnopyranosyl-(1→3)-2-acetamido-2-deoxy-β-D-glucopyranoside (3).

A solution of **64** (20 mg, 6.1 µmol) in EtOH/HOAc (3.3 mL, 10:1 v/v) was passed through a 10% Pd/C cartridge (CatCart®30) using a H-Cube^{™} continuous flow hydrogenation system in the "Full-H₂" mode. The temperature was set to 60 °C and the flow rate was fixed to 0.5 mL/min. After 8 h of reaction, the cartridge was rinced with EtOH/H₂O (30 mL, 1:1 v/v) and the solvents were evaporated to dryness. The residue (14 mg) was purified by preparative RP-HPLC to furnish 3 (8.7 mg, 84%) as a white amorphous powder; HPLC (215 nm): *R*ₜ = 4.0 min (Kromasil 5 µm C₁₈ 100 Å 4.6 × 250 mm column; 0-25% linear gradient of CH₃CN in 0.01 N aqueous TFA over 20 min at a flow rate of 5.5 mL·min⁻¹). ¹H NMR (D₂O, 400 MHz) δ: 5.21-5.20 (m, 2H, H-1_{E}, H-1_{E'}), 5.07, 5.06 (2 br s, 2H, H-1_{B}*, H-1_{B'}*), 5.02 (br s, 1H, H-1_{A}*), 4.97 (br s, 1H, H-1_{A'}), 4.84, 4.82 (2 br s, 2H, H-1_{C}, H-1_{C'}), 4.74 (d, *J*_{1,2} = 8.6 Hz, 1H, H-1_{D}'), 4.58 (d, *J*_{1,2} = 8.6 Hz, 1H, H-1_{D}), 4.41 (br d, *J*_{6a,6b} = 11.9 Hz, 1H, H-6a_{D'}), 4.32 (br d, *J*_{6a,6b} =11.9 Hz, 1H, H-6b_{D'}), 4.18-4.14 (m, 3H, H-2_{A}, H-5_{C}, H-5_{C'}), 4.09-4.00 (m, 4H, H-2_{A'}, H-2_{B}, H-2_{B'}, OC*H*₂), 3.98-3.26 (m, 41H, H-3_{A}, H-3_{A'}, H-4_{A}, H-4_{A'}, H-5_{A}, H-5_{A'}, H-3_{B}, H-3_{B'}, H-4_{B}, H-4_{B'}, H-5_{B}, H-5_{B'}, H-2_{C}, H-2_{C'}, H-3_{C}, H-3_{C'}, H-4_{C}, H-4_{C'}, H-2_{D}, H-2_{D'}, H-3_{D}, H-3_{D'}, H-4_{D}, H-4_{D'}, H-5_{D}, H-5_{D'}, H-6a_{D}, H-6b_{D}, H-2_{E}, H-2_{E'}, H-3_{E}, H-3_{E'}, H-4_{E}, H-4_{E'}, H-5_{E}, H-5_{E'}, H-6a_{E}, H-6a_{E'}, H-6b_{E}, H-6b_{E'}, OC*H*₂), 3.24-3.17 (m, 2H, C*H*₂NH₂), 2.15 (s, 3H, C(O)C*H*_{3Ac}), 2.07 (s, 3H, C(O)C*H*_{3NHAc}), 2.06 (s, 3H, C(O)C*H*_{3NHAc}), 1.35-1.26 (m, 18H, 6×C*H*_{3Rha}). ¹³C NMR (D₂O, 100 MHz) δ (partial): 174.5, 174.4, 174.2 (3×*C*(O)CH₃), 102.6 (C-1_{A'}), 102.3 (C-1_{D'}), 101.4 (2C, C-1_{B}*, C-1_{B'}*), 101.3 (C-1_{A}), 100.9 (2C, C-1_{C}, C-1_{C'}), 100.5 (C-1_{D}), 97.8 (2C, C-1_{E}, C-1_{E'}), 81.4, 81.1, 79.5, 79.0, 78.6, 76.0, 73.3, 72.7, 72.7, 72.4, 72.2, 72.1, 71.9, 71.4, 71.3, 70.7, 70.3, 70.2, 69.9, 69.8, 69.8, 69.7, 69.4, 69.3, 69.1, 68.4, 68.3, 65.7 (O*C*H₂), 63.4 (C-6_{D'}), 60.8 (C-6_{D}*), 60.7 (2×C, C-6_{E}*, C-6_{E'}*), 55.6 (C-2_{D}), 55.1 (C-2_{D'}*), 39.2 (*C*H₂NH₂), 22.4 (C(O)*C*H_{3NHAc}), 22.3 (C(O)*C*H_{3NHAc}), 20.4 (C(O)*C*H_{3Ac}), 17.9, 17.9, 16.9, 16.7 (2C), 16.7, 16.6 (6×*C*H_{3Rha}). HR-ESI-TOF-MS *m*/*z* 1710.6809 [M + H]⁺ (calcd for C₆₈H₁₁₆N₃O₄₆, 1710.6830).

### REFERENCES

(1) von Seidlein, L.; Kim, D. R.; Ali, M.; Lee, H.; Wang, X.; Thiem, V. D.; Canh do, G.; Chaicumpa, W.; Agtini, M. D.; Hossain, A.; Bhutta, Z. A.; Mason, C.; Sethabutr, O.; Talukder, K.; Nair, G. B.; Deen, J. L.; Kotloff, K.; Clemens, J. PLoS Med 2006, 3, e353.
(2) Petri, W. A., Jr.; Miller, M.; Binder, H. J.; Levine, M. M.; Dillingham, R.; Guerrant, R. L. J Clin Invest 2008, 118, 1277-1290.
(3) Levine, M. M.; Kotloff, K. L.; Barry, E. M.; Pasetti, M. F.; Sztein, M. B. Nature Reviews Microbiology 2007, 5, 540-553.
(4) Kotloff, K. L.; Winickoff, J. P.; Ivanoff, B.; Clemens, J. D.; Swerdlow, D. L.; Sansonetti, P. J.; Adak, G. K.; Levine, M. M. Bull World Health Organ 1999, 77, 651-666.
(5) Allison, G. E.; Verma, N. K. Trends Microbiol 2000, 8, 17-23.
(6) Cohen, D.; Ashkenazi, S.; Green, M. S.; Gdalevich, M.; Robin, G.; Slepon, R.; Yavzori, M.; Orr, N.; Block, C.; Ashkenazi, I.; Shemer, J.; Taylor, D. N.; Hale, T. L.; Sadoff, J. C.; Pavliakova, D.; Schneerson, R.; Robbins, J. B. Lancet 1997, 349, 155-159.
(7) Passwell, J. H.; Ashkenzi, S.; Banet-Levi, Y.; Ramon-Saraf, R.; Farzam, N.; Lerner-Geva, L.; Even-Nir, H.; Yerushalmi, B.; Chu, C.; Shiloach, J.; Robbins, J. B.; Schneerson, R. Vaccine 2010, 28, 2231-2235.
(8) Robbins, J. B.; Schneerson, R.; Szu, S. C. J Infect Dis 1995, 171, 1387-1398.
(9) Pozsgay, V.; Chu, C.; Pannell, L.; Wolfe, J.; Robbins, J. B.; Schneerson, R. Proc Natl Acad Sci U S A 1999, 96, 5194-5197.
(10) Wright, K.; Guerreiro, C.; Laurent, I.; Baleux, F.; Mulard, L. A. Org Biomol Chem 2004, 2, 1518-1527.
(11) Belot, F.; Guerreiro, C.; Baleux, F.; Mulard, L. A. Chemistry-a European Journal 2005, 11, 1625-1635.
(12) Phalipon, A.; Tanguy, M.; Grandjean, C.; Guerreiro, C.; Belot, F.; Cohen, D.; Sansonetti, P. J.; Mulard, L. A. Journal of Immunology 2009, 182, 2241-2247.
(13) Said Hassane, F.; Phalipon, A.; Tanguy, M.; Guerreiro, C.; Belot, F.; Frisch, B.; Mulard, L. A.; Schuber, F. Vaccine 2009, 27, 5419-5426.
(14) Vulliez-Le Normand, B.; Saul, F. A.; Phalipon, A.; Belot, F.; Guerreiro, C.; Mulard, L. A.; Bentley, G. A. Proceedings of the National Academy of Sciences of the United States of America 2008, 105, 9976-9981.
(15) Kenne, L.; Lindberg, B.; Petersson, K.; Katzenellenbogen, E.; Romanowska, E. European Journal of Biochemistry 1978, 91, 279-284.
(16) Kubler-Kielb, J.; Vinogradov, E.; Chu, C. Y.; Schneerson, R. Carbohydrate Research 2007, 342, 643-647.
(17) Perepelov, A. V.; L'vov, V. L.; Liu, B.; Senchenkova, S. N.; Shekht, M. E.; Shashkov, A. S.; Feng, L.; Aparin, P. G.; Wang, L.; Knirel, Y. A. Carbohydrate Research 2009, 344, 687-692.
(18) Szu, S. C.; Li, X. R.; Stone, A. L.; Robbins, J. B. Infect Immun 1991, 59, 4555-4561.
(19) Berry, D. S.; Lynn, F.; Lee, C. H.; Frasch, C. E.; Bash, M. C. Infect Immun 2002, 70, 3707-3713.
(20) Lewis, A. L.; Nizet, V.; Varki, A. Proc Natl Acad Sci U S A 2004, 101, 11123-11128.
(21) McNeely, T. B.; Staub, J. M.; Rusk, C. M.; Blum, M. J.; Donnelly, J. J. Infect Immun 1998, 66, 3705-3710.
(22) Fusco, P. C.; Farley, E. K.; Huang, C. H.; Moore, S.; Michon, F. Clin Vaccine Immunol 2007, 14, 577-584.
(23) Mulard, L. A.; Ughetto-Monfrin, J. J Carbohydr Chem 1999, 18, 721-753.
(24) Costachel, C.; Sansonetti, P. J.; Mulard, L. A. Journal of Carbohydrate Chemistry 2000, 19, 1131-1150.
(25) Phalipon, A.; Costachel, C.; Grandjean, C.; Thuizat, A.; Guerreiro, C.; Tanguy, M.; Nato, F.; Vulliez-Le Normand, B.; Belot, F.; Wright, K.; Marcel-Peyre, V.; Sansonetti, P. J.; Mulard, L. A. Journal of Immunology 2006, 176, 1686-1694.
(26) Pozsgay, V.; Kubler-Kielb, J.; Schneerson, R.; Robbins, J. B. Proc Natl Acad Sci U S A 2007, 104, 14478-14482.
(27) Belot, F.; Wright, K.; Costachel, C.; Phalipon, A.; Mulard, L. A. Journal of Organic Chemistry 2004, 69, 1060-1074.
(28) Hassner, A.; Strand, G.; Rubinstein, M.; Patchornik, A. Journal of the American Chemical Society 1975, 97, 1614-1615.
(29) Wada, T.; Ohkubo, A.; Mochizuki, A.; Sekine, M. Tetrahedron Letters 2001, 42, 1069-1072.
(30) Blatter, G.; Beau, J. M.; Jacquinet, J. C. Carbohydrate Research 1994, 260, 189-202.
(31) Donohoe, T. J.; Logan, J. G.; Laffan, D. D. P. Organic Letters 2003, 5, 4995-4998.
(32) Bongat, A. F. G.; Demchenko, A. V. Carbohydrate Research 2007, 342, 374-406.
(33) Schmidt, R. R.; Kinzy, W. Adv. Carbohydr. Chem. Biochem. 1994, 50, 21-123.
(34) Yu, B.; Tao, H. C. Tetrahedron Letters 2001, 42, 2405-2407.
(35) Yu, B.; Tao, H. C. Journal of Organic Chemistry 2002, 67, 9099-9102.
(36) Boutet, J.; Guerreiro, C.; Mulard, L. A. Tetrahedron 2008, 64, 10558-10572.
(37) Rajput, V. K.; Mukhopadhyay, B. Journal of Organic Chemistry 2008, 73, 6924-6927.
(38) Bousquet, E.; Khitri, M.; Lay, L.; Nicotra, F.; Panza, L.; Russo, G. Carbohydrate Research 1998, 311, 171-181.
(39) Gurjar, M. K.; Mainkar, A. S. Tetrahedron 1992, 48, 6729-6738.
(40) Love, K. R.; Andrade, R. B.; Seeberger, P. H. Journal of Organic Chemistry 2001, 66, 8165-8176.
(41) Matsuda, H.; Hashimoto, N.; Okuno, T. Synthetic Communications 2002, 32, 3347-3355.
(42) Djerassi, C. Chemical Reviews 1948, 43, 271-317.
(43) Zhu, S. L.; Li, Y. X.; Yu, B. Journal of Organic Chemistry 2008, 73, 4978-4985.
(44) Oltvoort, J. J.; Vanboeckel, C. A. A.; Dekoning, J. H.; Vanboom, J. H. Synthesis-Stuttgart 1981, 305-308.
(45) Nashed, M. A.; Anderson, L. Journal of the Chemical Society-Chemical Communications 1982, 1274-1276.
(46) Boutet, J.; Kim, T. H.; Guerreiro, C.; Mulard, L. A. Tetrahedron Letters 2008, 49, 5339-5342.
(47) Inouye, Y.; Onodera, A.; Kitaoka, S.; Hirano, S. Journal of the American Chemical Society 1956, 78, 4722-4724.
(48) Vauzeilles, B.; Dausse, B.; Palmier, S.; Beau, J. M. Tetrahedron Letters 2001, 42, 7567-7570.
(49) Arosio, D.; Vrasidas, I.; Valentini, P.; Liskamp, R. M. J.; Pieters, R. J.; Bernardi, A. Organic & Biomolecular Chemistry 2004, 2, 2113-2124.
(50) Feng, F.; Okuyama, K.; Niikura, K.; Ohta, T.; Sadamoto, R.; Monde, K.; Noguchi, T.; Nishimura, S. I. Organic & Biomolecular Chemistry 2004, 2, 1617-1623.
(51) Wolfrom, M. L.; Diwadkar, A. B.; Gelas, J.; Horton, D. Carbohydrate Research 1974, 35, 87-96.
(52) Timmons, S. C.; Jakeman, D. L. Carbohydrate Research 2007, 342, 2695-2704.
(53) Gauthier, C.; Legault, J.; Lavoie, S.; Rondeau, S.; Tremblay, S.; Pichette, A. Tetrahedron 2008, 64, 7386-7399.
(54) Cai, Y.; Ling, C. C.; Bundle, D. R. Organic Letters 2005, 7, 4021-4024.
(55) Cai, Y.; Ling, C. C.; Bundle, D. R. Journal of Organic Chemistry 2009, 74, 580-589.
(56) Jacquine.Jc; Sinay, P. Carbohydrate Research 1974, 32, 101-114.
(57) Furneaux, R. H.; Gainsford, G. J.; Lynch, G. P.; Yorke, S. C. Tetrahedron 1993, 49, 9605-9612.
(58) Noguchi, M.; Tanaka, T.; Gyakushi, H.; Kobayashi, A.; Shoda, S. Journal of Organic Chemistry 2009, 74, 2210-2212.
(59) Christensen, H.; Christiansen, M. S.; Petersen, J.; Jensen, H. H. Organic & Biomolecular Chemistry 2008, 6, 3276-3283.
(60) Wittmann, V.; Lennartz, D. *European Journal of Organic Chemistry* **2002**, 1363-1367.
(61) Ni, J. H.; Singh, S.; Wang, L. X. Bioconjugate Chemistry 2003, 14, 232-238.
(62) Lubineau, A.; Bonnaffe, D. European Journal of Organic Chemistry 1999, 2523-2532.
(63) Mulard, L. A.; Costachel, C.; Sansonetti, P. J. Journal of Carbohydrate Chemistry 2000, 19, 849-877.
(64) Chauvin, A. L.; Nepogodiev, S. A.; Field, R. A. Journal of Organic Chemistry 2005, 70, 960-966.
(65) Boutet, J.; Mulard, L. A. European Journal of Organic Chemistry 2008, 5526-5542.
(66) Pozsgay, V.; Coxon, B. Carbohydrate Research 1994, 257, 189-215.
(67) Gonzalez, A. G.; Brouard, I.; Leon, F.; Padron, J. I.; Bermejo, J. Tetrahedron Letters 2001, 42, 3187-3188.
(68) Kunesch, N.; Miet, C.; Poisson, J. Tetrahedron Letters 1987, 28, 3569-3572.
(69) Liu, H. M.; Yan, X. B.; Li, W.; Huang, C. H. Carbohydrate Research 2002, 337, 1763-1767.
(70) Wang, S. M.; Ge, W. Z.; Liu, H. M.; Zou, D. P.; Yan, X. B. Steroids 2004, 69, 599-604.
(71) Wang, S. M.; Zhang, Y. B.; Liu, H. M.; Yu, G. B.; Wang, K. R. Steroids 2007, 72, 26-30.
(72) Du, Y. G.; Wei, G. H.; Linhardt, R. J. Journal of Organic Chemistry 2004, 69, 2206-2209.
(73) Yeom, C. E.; Lee, S. Y.; Kim, Y. J.; Kim, B. M. Synlett 2005, 1527-1530.
(74) Gauthier, C.; Legault, J.; Lavoie, S.; Rondeau, S.; Tremblay, S.; Pichette, A. Journal of Natural Products 2009, 72, 72-81.
(75) Meldgaard, M.; Hansen, F. G.; Wengel, J. Journal of Organic Chemistry 2004, 69, 6310-6322.
(76) Love, K. R.; Seeberger, P. H. Journal of Organic Chemistry 2005, 70, 3168-3177.
(77) Schmidt, R. R.; Toepfer, A. Tetrahedron Letters 1991, 32, 3353-3356.
(78) Chen, J. F.; Zhou, Y.; Chen, C.; Xu, W. C.; Yu, B. Carbohydrate Research 2008, 343, 2853-2862.
(79) Popelova, A.; Kefurt, K.; Hlavackova, M.; Moravcova, J. Carbohydrate Research 2005, 340, 161-166.
(80) Saksena, R.; Adamo, R.; Kovac, P. Bioorganic & Medicinal Chemistry 2007, 15, 4283-4310.
(81) Wang, Z.; Zhou, L. Y.; El-Boubbou, K.; Ye, X. S.; Huang, X. F. Journal of Organic Chemistry 2007, 72, 6409-6420.
(82) Alpe, M.; Oscarson, S. Carbohydrate Research 2002, 337, 1715-1722.
(83) Dhenin, S. G. Y.; Moreau, V.; Nevers, M. C.; Creminon, C.; Djedaini-Pilard, F. Organic & Biomolecular Chemistry 2009, 7, 5184-5199.
(84) Boutet, J.; Guerreiro, C.; Mulard, L. A. Journal of Organic Chemistry 2009, 74, 2651-2670.
(85) Valerio, S.; Pastore, A.; Adinolfi, M.; Iadonisi, A. Journal of Organic Chemistry 2008, 73, 4496-4503.
(86) Wong, C. H. Abstracts of Papers of the American Chemical Society 1998, 215, U113-U113.
(87) Boger, D. L.; Garbaccio, R. M.; Jin, Q. Journal of Organic Chemistry 1997, 62, 8875-8891.
(88) Zhang, Y. C.; Li, Y. X.; Zhu, S. L.; Guan, H. S.; Lin, F.; Yu, B. Carbohydrate Research 2004, 339, 1753-1759.
(89) Wright, J. A.; Yu, J. Q.; Spencer, J. B. Tetrahedron Letters 2001, 42, 4033-4036.
(90) Zhang, H. J.; Yan, S. Q.; Liang, X. M.; Wu, J. P.; Wang, D. Q.; Kong, F. Z. Carbohydrate Research 2007, 342, 2810-2817.
(91) Ishihara, K.; Kurihara, H.; Yamamoto, H. Journal of Organic Chemistry 1993, 58, 3791-3793.
(92) Garegg, P. J.; Olsson, L.; Oscarson, S. Journal of Carbohydrate Chemistry 1997, 16, 973-981.
(93) Gemma, E.; Lahmann, M.; Oscarson, S. Carbohydrate Research 2005, 340, 2558-2562.
(94) Pozsgay, V.; Ekborg, G.; Sampathkumar, S. G. Carbohydrate Research 2006, 341, 1408-1427.
(95) Knudsen, K. R.; Holden, J.; Ley, S. V.; Ladlow, M. Advanced Synthesis & Catalysis 2007, 349, 535-538.
(96) Gigg, R.; Payne, S.; Conant, R. Journal of Carbohydrate Chemistry 1983, 2, 207-223.
(97) Tamura, K.; Mizukami, H.; Maeda, K.; Watanabe, H.; Uneyama, K. Journal of Organic Chemistry 1993, 58, 32-35.

## Claims

1. A decasaccharide {A'B'(E')C'D'AB(E)CD}-OR of formula (I): 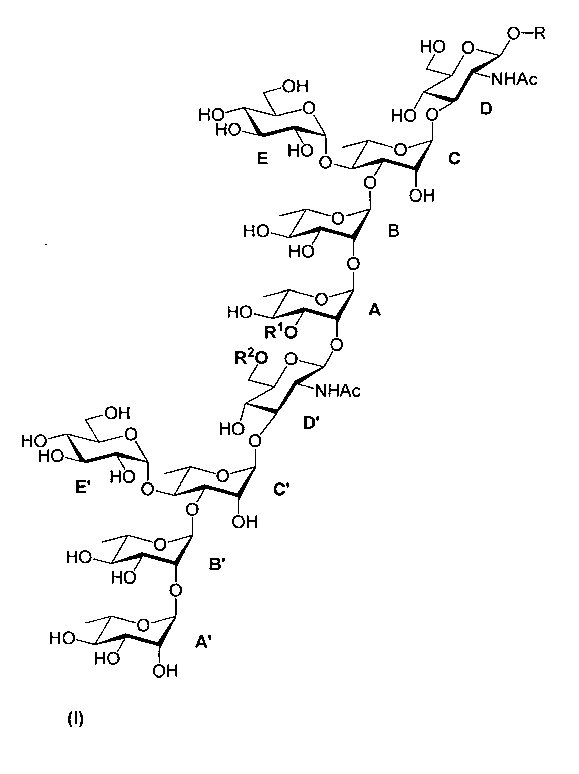 Wherein:
R is H or -Alk-Z;
Z is a group selected from NH₂, NHR₃, -C(=NH)-OR₃, -C≡N, -C(=O)OH, -C(=O)-R₃, -CH(OR₃)(OR₄), -CH(SR₃)(OR₄), -CH(SR₃)(SR₄), -CH=CH-R₃, -C≡C-R₃, -N=C=O, -N=C=S, -SCO-R₃, -SH, -S-S-R₃, -N₃, -CONHNH₂, - NH-NH₂, an epoxyde group or halogen,
Alk is a C₁-C₁₂ alkylene group;
R₃, R₄ are each independently H, C₁-C₆ alkyl or C₂-C₈ alkenyl, or R₃ and R₄ together form with the atoms to which they are attached, a 5 to 7 membered heterocycloalkyl group;
R₁ and R₂ are each independently selected from H or CH₃C(=O)- (noted Ac), with the proviso that at least one of R₁ or R₂ is Ac.

2. The decasaccharide {A'B'(E')C'D'AB(E)C D}-OR of formula (I) according to claim 1, wherein Z is NH₂.

3. The decasaccharide {A'B'(E')C'D'AB(E)C D}-OR of formula (I) according to any of claims 1 or 2, wherein R is (CH₂)₂NH₂.

4. A conjugate molecule comprising a decasaccharide residue {A'B'(E')C'D'AB(E)C D}-O- as defined in any of claims 1 to 3, said decasaccharide residue being covalently bound to a carrier.

5. A decasaccharide {A'B'(E')C'D'AB(E)CD}-OR' of formula (II): 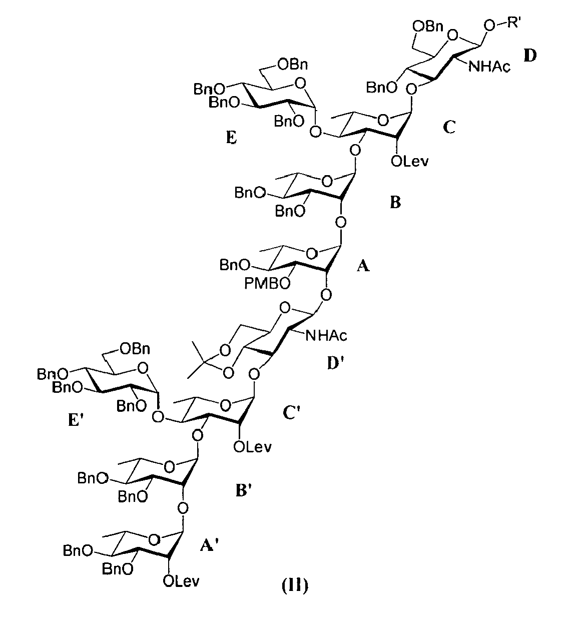 Wherein R' is R or a precursor group of R, R being as defined in claim 1.

6. The decasaccharide {A'B'(E')C'D'AB(E)C D}-OR' of formula (II) according to claim 5, wherein R' is -(CH₂)ₙ-N₃ with n being an integer from 1 to 10.

7. A method for preparing a decasaccharide of formula (I) as defined in any of claims 1 to 3, said method comprising the steps consisting of:
i) deprotection of *para*-methoxybenzyl (PMB) and/or isopropylidene groups (-iPr-) on the 3_{A} and/or 6_{D'} hydroxyl moieties of a compound of formula (II) according to any of claims 5 or 6 ;
ii) acetylation of the resulting deprotected 3_{A} and/or 6_{D'} hydroxyl moieties ;
iii) cleavage of all remaining protecting groups of the resulting compound ; and optionally
iv) conversion of R' into R, thereby obtaining the decasaccharide of formula (I).

8. A use of a decasaccharide of formula (II) as defined in any of claims 5 or 6, for the preparation of a decasaccharide of formula (I) as defined in any of claims 1 to 3.

9. A method of preparation of a decasaccharide of formula (II) as defined in claims 5 or 6, said method comprising the steps of:
i) coupling the hexasaccharide D'AB(E)CD of formula (III), wherein R' is as defined hereabove, and the tetrasaccharide A'B'(E')C' of formula (IV), according to a glycosylation reaction; and optionally
ii) recovering the obtained decasaccharide of formula (II). 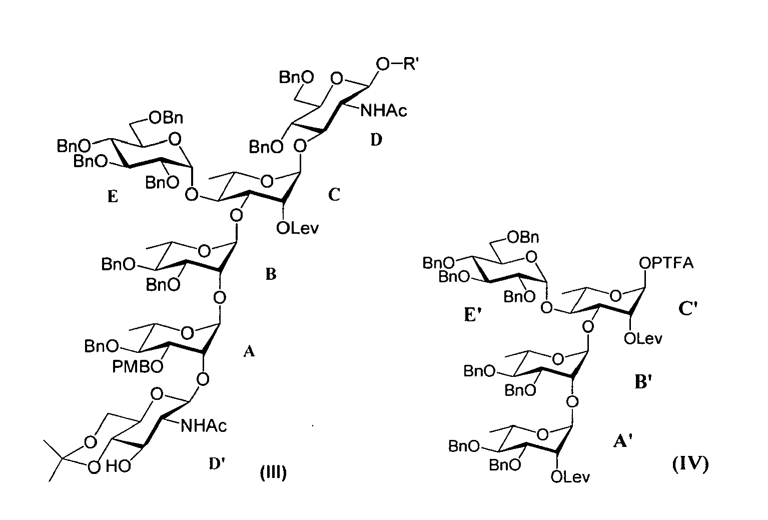 Wherein R' is as defined in claim 5.

10. The method of claim 9, wherein the hexasaccharide D'AB(E)CD of formula (III) is prepared by a method comprising :
i) reacting the pentasaccharide D'AB(E)C of formula (V) with the monosaccharide D of formula (VI) according to a glycosylation reaction: 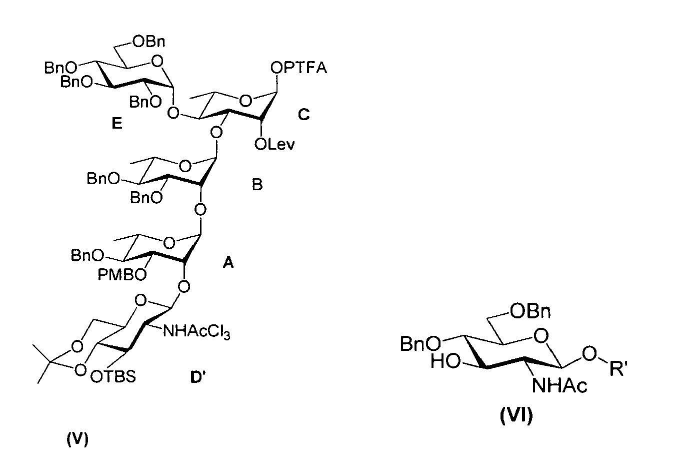 And
ii) deprotecting the 3_{D'} hydroxyl group of the obtained pentasaccharide.

11. The method of claim 10, wherein the pentasaccharide D'AB(E)C of formula (V) is prepared by a method comprising :
i) reacting a tetrasaccharide AB(E)C of formula (VII) with a monosaccharide D' of formula (VIII) according to a glycosylation reaction: 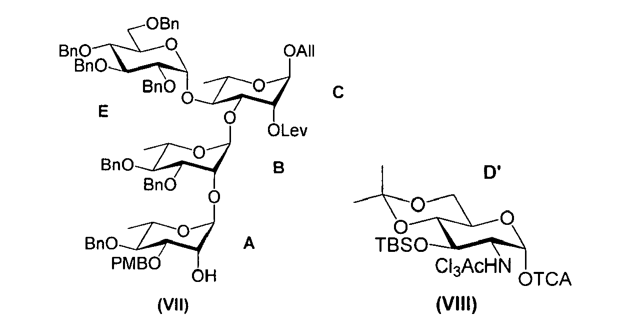
ii) deprotecting the 1_{C} hydroxyl group of the obtained pentasaccharide ; And
iii) converting the deprotected 1_{C} hydroxyl group into a -OPTFA group.

12. The method of claim 11, wherein the tetrasaccharide AB(E)C of formula (VII) is prepared according to a method comprising:
i) reacting a trisaccharide B(E)C of formula (IX) with a monosaccharide A of formula (X) according to a glycosylation reaction: 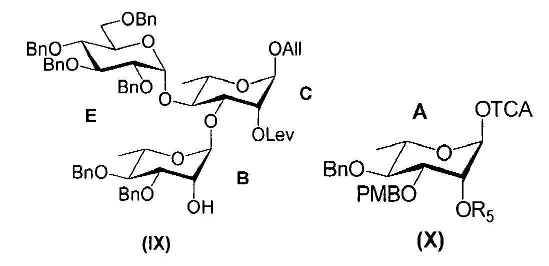 Wherein R₅ is AZMB or chloroacetyl, And
ii) deprotecting the 2_{A} hydroxyl group in the obtained tetrasaccharide.

13. A hexasaccharide D'AB(E)CD of formula (III) : 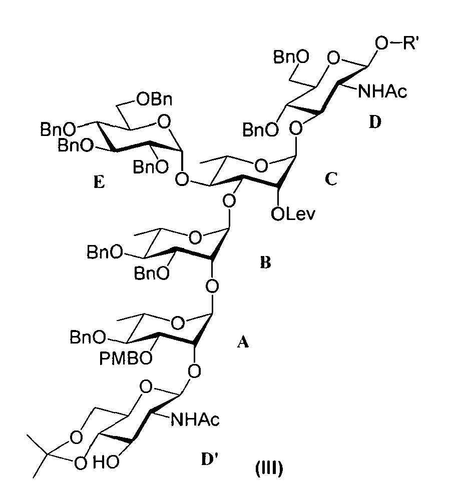 Wherein R' is as defined in claim 5.

14. A pentasaccharide D'AB(E)C of formula (V):

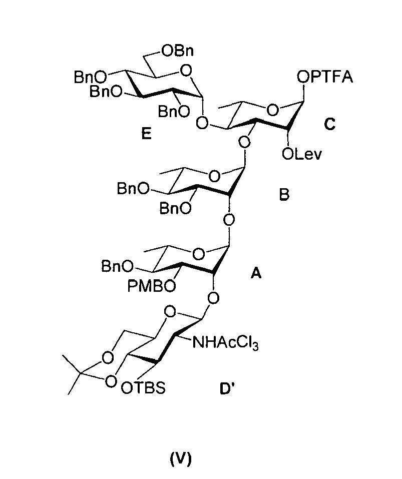

15. A tetrasaccharide A'B'(E')C' of formula (IV):

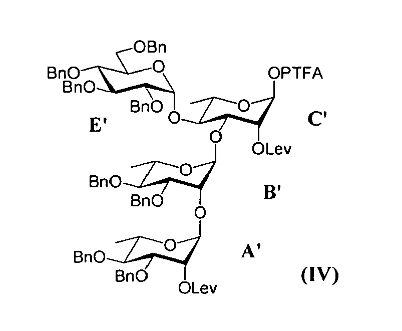

16. A use of a decasaccharide of formule (II) according to any of claims 5 or 6 for the preparation of a decasaccharide of formula (I) according to any of claims 1 to 3.

17. A kit for the diagnostic of *Shigella* infection, wherein said kit comprises a conjugate molecule according to claim 4.
